# EUROPEAN PATENT APPLICATION

(11) **EP 2 468 864 A1**
(43) Date of publication of application: **27.06.2012**
(21) Application number: 12153919.1
(22) Date of filing: 28.02.2008
(51) Int. Cl.: C12N 15/113, A61K 31/713

(54) **Nucleic acid compounds for inhibiting VEGF family gene expression and uses thereof**

(30) Priority: 02.03.2007 US 934940 P; 16.03.2007 US 934930 P; 20.04.2007 US 934931 P; 24.04.2007 US 934934 P; 24.04.2007 US 934928 P; 25.04.2007 US 934942 P; 25.04.2007 US 934943 P; 03.05.2007 US 932949 P
(62) Divisional of application: 08731034.8
(71) Applicant: Marina Biotech, Inc., Bothell WA 98021-7266 (US)
(72) Inventor: Quay, Steven C., Woodinville, WA 98072 (US); McSwiggen, James, Boulder, CO 80304 (US); Vaish, Narendra K., Kirkland, WA 98034 (US); Ahmadian, Mohammad, Carlsbad, CA 92009 (US)
(74) Representative: MacLean, Martin Robert

(57) **Abstract**

The present disclosure provides meroduplex ribonucleic acid molecules (mdRNA) capable of decreasing or silencing one or more VEGF family gene expression. An mdRNA of this disclosure comprises at least three strands that combine to form at least two non-overlapping double-stranded regions separated by a nick or gap wherein one strand is complementary to one or more VEGF family mRNA. In addition, the meroduplex may have at least one uridine substituted with a 5-methyluridine and optionally other modifications or combinations thereof. Also provided are methods of decreasing expression of one or more VEGF family gene in a cell or in a subject to treat one or more VEGF family-related disease.

## Description

### CROSS-REFERENCE TO RELATED APPLICATIONS

The present application claims priority to U.S. Provisional Patent Application Nos. 60/934,940, filed March 2, 2007; 60/934,930, filed March 16, 2007; 60/934,931, filed April 20, 2007; 60/934,928, filed April 24, 2007; 60/934,934, filed April 24, 2007; 60/934,942, filed April 25, 2007; 60/934,943, filed April 25, 2007; and 60/932,949, filed May 3, 2007, each of which is incorporated by reference in its entirety.

### TECHNICAL FIELD

The present disclosure relates generally to compounds for use in treating hyperproliferative or inflammatory disorders by gene silencing and, more specifically, to a nicked or gapped double-stranded RNA (dsRNA) comprising at least three strands that decreases expression of one or more VEGF family gene, and to uses of such dsRNA to treat or prevent hyperproliferative or inflammatory diseases associated with inappropriate expression of one or more VEGF family members. The dsRNA that decreases one or more VEGF family gene expression may optionally have at least one uridine substituted with a 5-methyluridine.

### BACKGROUND

RNA interference (RNAi) refers to the cellular process of sequence specific, post-transcriptional gene silencing in animals mediated by small inhibitory nucleic acid molecules, such as a double-stranded RNA (dsRNA) that is homologous to a portion of a targeted messenger RNA (Fire et al., Nature 391:806, 1998; Hamilton et al., Science 286:950, 1999). RNAi has been observed in a variety of organisms, including mammalians (Fire et al., Nature 391:806, 1998; Bahramian and Zarbl, Mol. Cell. Biol. 19:274, 1999; Wianny and Goetz, Nature Cell Biol. 2:70, 1999). RNAi can be induced by introducing an exogenous 21-nucleotide RNA duplex into cultured mammalian cells (Elbashir et al., Nature 411:494, 2001a).

The mechanism by which dsRNA mediates targeted gene-silencing can be described as involving two steps. The first step involves degradation of long dsRNAs by a ribonuclease III-like enzyme, referred to as Dicer, into short interfering RNAs (siRNAs) having from 21 to 23 nucleotides with double-stranded regions of about 19 base pairs and a two nucleotide, generally, overhang at each 3'-end (Berstein et al., Nature 409:363, 2001; Elbashir et al., Genes Dev. 15:188, 2001b; and Kim et al., Nature Biotech. 23:222, 2005). The second step of RNAi gene-silencing involves activation of a multi-component nuclease having one strand (guide or antisense strand) from the siRNA and an Argonaute protein to form an RNA-induced silencing complex ("RISC") (Elbashir et al., Genes Dev. 15:188, 2001). Argonaute initially associates with a double-stranded siRNA and then endonucleolytically cleaves the non-incorporated strand (passenger or sense strand) to facilitate its release due to resulting thermodynamic instability of the cleaved duplex (Leuschner et al., EMBO 7:314, 2006). The guide strand in the activated RISC binds to a complementary target mRNA and cleaves the mRNA to promote gene silencing. Cleavage of the target RNA occurs in the middle of the target region that is complementary to the guide strand (Elbashir *et al.*, 200 1 b).

The family of Vascular Endothelial Cell Growth Factors (VEGFs) is, at this time, known to comprise six closely related polypeptides, VEGFA, VEGFB, VEGFC, VEGFD, VEGFE, and placental growth factor (PGF) (see Ferrara, J. Mol. Bio. 77:527, 1999). The VEGFs are pro-angiogenic factors that impact vascular proliferation and/or vascular permeability. The biological activities of each VEGF family member is mediated through one or more of a corresponding family of at least four cell surface localized VEGF receptors (VEGFR), including, VEGFR1, VEGFR2, VEGFR3, NRP1 and NRP2 (Neuropilin receptors 1 and 2, respectively).

VEGFA driven angiogenesis has a role in the pathogenesis of diverse human disease, including cancer, arthritis, atherosclerosis, diabetic retinopathy, intraocular neovascular disorder, and other conditions (Woolard et al., Cancer Res. 64:7822, 2004). VEGFB has a role in angiogenesis and endothelial cell growth, and has been implicated in cancer such as neuroblastoma. Studies indicate that VEGFC plays an important role in lymphangiogenesis, which is a critical process in the progression of many malignant tumors, including non-small-lung cancer (NSCLC), and there are also data that indicate VEGFC may possess angiogenic properties relating to capillaries. In human tumors and a mouse tumor model, FIGF was capable of promoting tumor angiogenesis, tumor lymphangiogenesis, and metastatic spread (Stacker et al., Nature Med. 7:186, 2001; Achen et al., Growth Factors 20:99, 2002). PGF has been found to affect angiogenesis in disease but not in health, so inhibition of PGF may be useful in inhibiting tumor growth without affecting quiescent vessels. The recognized importance of VEGF in cancer has led to the recent approval of humanized monoclonal antibody, Avastin (bevacizumab), for treating colorectal cancer (Ferrara et al., Nat'l. Rev. Drug Discov. 3:391, 2004), but suffers from the need for high systemic doses. An inhibitor therapeutic directed to VEGFB, VEGFC, FIGF, or PGF has not been approved to date.

There continues to be a need for alternative effective therapeutic modalities useful for treating or preventing VEGF family-associated diseases or disorders in which reduced gene expression (gene silencing) of one or more VEGF family genes would be beneficial. The present disclosure meets such needs, and further provides other related advantages.

### BRIEF SUMMARY

Briefly, the present disclosure provides nicked or gapped double-stranded RNA (dsRNA) comprising at least three strands that is suitable as a substrate for Dicer or as a RISC activator to modify expression of one or more vascular endothelial growth factor (VEGF) family messenger RNA (mRNA).

In one aspect, the instant disclosure provides a meroduplex mdRNA molecule, comprising a first strand that is complementary to vascular endothelial growth factor (VEGF) mRNA as set forth in SEQ ID NO:1158, 1159, 1160, 1161, 1162, 1163, or 1164 *(i.e.,* VEGF variants 1 to 7) and is fully complementary, with up to three mismatches, to at least one other human VEGF family mRNA selected from SEQ ID NO:1165, 1166, 1167, or 1168 (*i.e.,* VEGFB, VEGFC, FIGF, PGF, respectively), and a second strand and a third strand that are each complementary to non-overlapping regions of the first strand, wherein the second strand and third strands can anneal with the first strand to form at least two double-stranded regions spaced apart by up to 10 nucleotides and thereby forming a gap between the second and third strands, and wherein (a) the mdRNA molecule optionally includes at least one double-stranded region of 5 base pairs to 13 base pairs, or (b) the double-stranded regions combined total about 15 base pairs to about 40 base pairs and the mdRNA molecule optionally has blunt ends. In certain embodiments, the first strand is about 15 to about 40 nucleotides in length, and the second and third strands are each, individually, about 5 to about 20 nucleotides, wherein the combined length of the second and third strands is about 15 nucleotides to about 40 nucleotides. In other embodiments, the first strand is about 15 to about 40 nucleotides in length and is complementary to at least about 15, 19, 20, 21, 22, 23, 24, 25, 26, 27, 28, 29, 30, 31, 32, 33, 34, 35, 36, 37, 38, 39, or 40 contiguous nucleotides of a human VEGF family mRNA as set forth in at least two of SEQ ID NOS:1158-1168. In still further embodiments, the first strand is about 15 to about 40 nucleotides in length and is at least about 70%, 75%, 80%, 85%, 90%, 95%, 96%, 97%, 98%, or 99% identical to a sequence that is complementary to at least about 15, 19, 20, 21, 22, 23, 24, 25, 26, 27, 28, 29, 30, 31, 32, 33, 34, 35, 36, 37, 38, 39, or 40 contiguous nucleotides of a human VEGF family mRNA as set forth in at least two of SEQ ID NOS:1158-1168.

In other embodiments, the mdRNA is a RISC activator (e.g., the first strand has about 15 nucleotides to about 25 nucleotides) or a Dicer substrate (e.g., the first strand has about 26 nucleotides to about 40 nucleotides). In some embodiments, the gap comprises at least one to ten unpaired nucleotides in the first strand positioned between the double-stranded regions formed by the second and third strands when annealed to the first strand, or the gap is a nick. In certain embodiments, the nick or gap is located 10 nucleotides from the 5'-end of the first (antisense) strand or at the Argonaute cleavage site. In another embodiment, the meroduplex nick or gap is positioned such that the thermal stability is maximized for the first and second strand duplex and for the first and third strand duplex as compared to the thermal stability of such meroduplexes having a nick or gap in a different position.

In another aspect, the instant disclosure provides an mdRNA molecule having a first strand that is complementary to human VEGF mRNA as set forth in SEQ ID NO:1158, 1159, 1160, 1161, 1162, 1163, or 1164 and is fully complementary, with up to three mismatches, to at least one other human VEGF family mRNA selected from SEQ ID NO:1165, 1166, 1167, or 1168, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein (a) the mdRNA molecule optionally includes at least one double-stranded region of 5 base pairs to 13 base pairs, or (b) the double-stranded regions combined total about 15 base pairs to about 40 base pairs and the mdRNA molecule optionally has blunt ends; and wherein at least one pyrimidine of the mdRNA comprises a pyrimidine nucleoside according to Formula I or II: 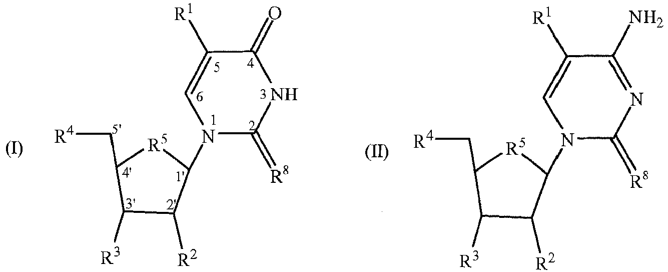 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C≡N, or heterocyclo group; R³ and R⁴ are each independently a hydroxyl, a protected hydroxyl, a phosphate, or an internucleoside linking group; and R⁵ and R⁸ are independently O or S. In certain embodiments, at least one nucleoside is according to Formula I in which R¹ is methyl and R² is -OH. In certain related embodiments, at least one uridine of the dsRNA molecule is replaced with a nucleoside according to Formula I in which R¹ is methyl and R² is - OH, or R¹ is methyl, R² is -OH, and R⁸ is S. In some embodiments, the at least one R¹ is a C₁-C₅ alkyl, such as methyl. In some embodiments, at least one R² is selected from 2ʹ-O-(C₁-C₅) alkyl, 2ʹ-O-methyl, 2ʹ-OCH₂OCH₂CH₃, 2ʹ-OCH₂CH₂OCH₃, 2ʹ-O-allyl, or fluoro. In some embodiments, at least one pyrimidine nucleoside of the mdRNA molecule is a locked nucleic acid (LNA) in the form of a bicyclic sugar, wherein R² is oxygen, and the 2ʹ-O and 4'-C form an oxymethylene bridge on the same ribose ring (*e.g*., a 5-methyluridine LNA) or is a G clamp. In other embodiments, one or more of the nucleosides are according to Formula I in which R¹ is methyl and R² is a 2ʹ-O-(C₁-C₅) alkyl, such as 2ʹ-O-methyl. In some embodiments, the gap comprises at least one unpaired nucleotide in the first strand positioned between the double-stranded regions formed by the second and third strands when annealed to the first strand, or the gap is a nick. In certain embodiments, the nick or gap is located 10 nucleotides from the 5'-end of the first strand or at the Argonaute cleavage site. In another embodiment, the meroduplex nick or gap is positioned such that the thermal stability is maximized for the first and second strand duplex and for the first and third strand duplex as compared to the thermal stability of such meroduplexes having a nick or gap in a different position.

In still another aspect, the instant disclosure provides a method for reducing the expression of one or more human VEGF family genes in a cell, comprising administering an mdRNA molecule to a cell expressing one or more VEGF family gene, wherein the mdRNA molecule is capable of specifically binding to one or more VEGF family mRNA and thereby reducing expression of one or more VEGF genes in the cell. In a related aspect, there is provided a method of treating or preventing a disease associated with VEGF family expression in a subject by administering an mdRNA molecule of this disclosure. In certain embodiments, the cell or subject is human. In certain embodiments, the disease is a hyperproliferative disease, such as cancer, or an inflammatory disorder, such as arthritis.

In any of the aspects of this disclosure, some embodiments provide mdRNA molecule having a 5-methyluridine (ribothymidine) , a 2-thioribothymidine, or 2'-O-methyl-5-methyluridine in place of at least one uridine on the first, second, or third strand, or in place of each and every uridine on the first, second, or third strand. In further embodiments, the mdRNA further comprises one or more non-standard nucleoside, such as a deoxyuridine, locked nucleic acid (LNA) molecule, such as a 5-methyluridine LNA, a universal-binding nucleotide, or any combination thereof. Exemplary universal-binding nucleotides include C-phenyl, C-naphthyl, inosine, azole carboxamide, 1-β-D-ribofuranosyl-4-nitroindole, 1-β-D-ribofuranosyl-5-nitroindole, 1-β-D-ribofuranosyl-6-nitroindole, or 1-β-D-ribofuranosyl-3-nitropyrrole. In some embodiments, the mdRNA molecule further comprises a 2'-sugar substitution, such as a 2'-O-methyl, 2'-O-methoxyethyl, 2'-O-2-methoxyethyl, 2'-O-allyl, or halogen (*e.g*., 2'-fluoro). In certain embodiments, the mdRNA molecule further comprises a terminal cap substituent on one or both ends of the first strand, second strand, or third strand, such as independently an alkyl, abasic, deoxy abasic, glyceryl, dinucleotide, acyclic nucleotide, or inverted deoxynucleotide moiety. In other embodiments, the mdRNA molecule further comprises at least one modified internucleoside linkage, such as independently a phosphorothioate, chiral phosphorothioate, phosphorodithioate, phosphotriester, aminoalkylphosphotriester, methyl phosphonate, alkyl phosphonate, 3'-alkylene phosphonate, 5'-alkylene phosphonate, chiral phosphonate, phosphonoacetate, thiophosphonoacetate, phosphinate, phosphoramidate, 3'-amino phosphoramidate, aminoalkylphosphoramidate, thionophosphoramidate, selenophosphate, thionoalkylphosphonate, thionoalkylphosphotriester, or boranophosphate linkage.

In any of the aspects of this disclosure, some embodiments provide an mdRNA comprising an overhang of one to four nucleotides on at least one 3'-end that is not part of the gap, such as at least one deoxyribonucleotide or two deoxyribonucleotides (*e.g*., thymidine). In some embodiments, at least one or two 5'-terminal ribonucleotide of the second strand within the double-stranded region comprises a 2'-sugar substitution. In related embodiments, at least one or two 5'-terminal ribonucleotide of the first strand within the double-stranded region comprises a 2'-sugar substitution. In other related embodiments, at least one or two 5'-terminal ribonucleotide of the second strand and at least one or two 5'-terminal ribonucleotide of the first strand within the double-stranded regions comprise independent 2'-sugar substitutions. In other embodiments, the mdRNA molecule comprises at least three 5-methyluridines within at least one double-stranded region. In some embodiments, the mdRNA molecule has a blunt end at one or both ends. In other embodiments, the 5'-terminal of the third strand is a hydroxyl or a phosphate.

### BRIEF DESCRIPTION OF THE DRAWINGS

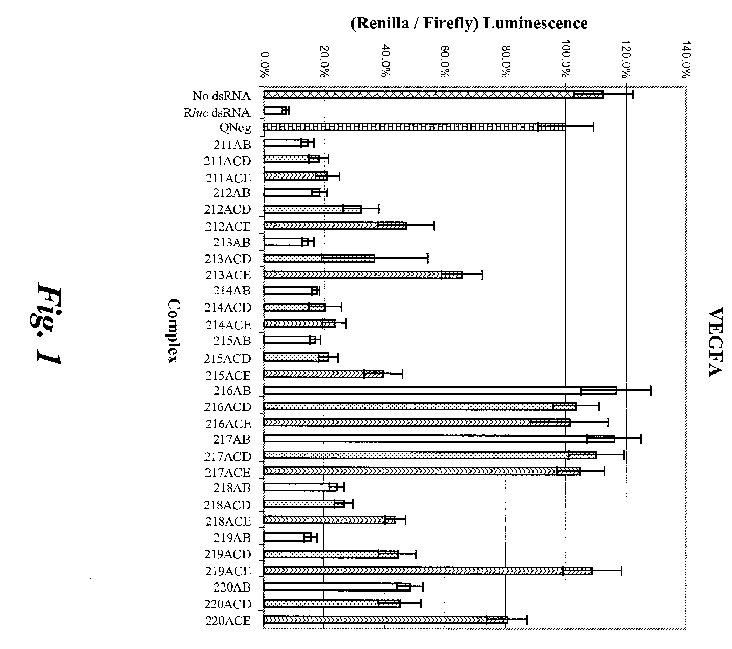

Figure 1 shows the gene silencing activity of ten different VEGF-specific nicked and gapped dsRNA Dicer substrate. This is the graphical representation of the data found in Table 1 (the Complex numbers on the x-axis correspond to the Set numbers for each of the ten different VEGF dsRNA shown in Table 1).

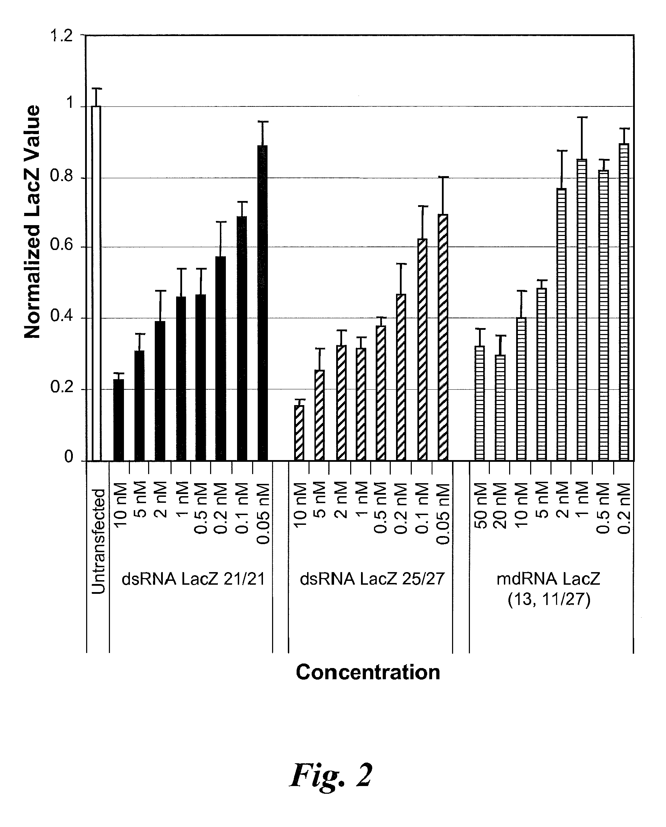

Figure 2 shows knockdown activity for RISC activator lacZ dsRNA (21 nucleotide sense strand/21 nucleotide antisense strand; 21/21), Dicer substrate lacZ dsRNA (25 nucleotide sense strand/27 nucleotide antisense strand; 25/27), and meroduplex lacZ mdRNA (13 nucleotide sense strand and 11 nucleotide sense strand/27 nucleotide antisense strand; 13, 11/27 - the sense strand is missing one nucleotide so that a single nucleotide gap is left between the 13 nucleotide and 11 nucleotide sense strands when annealed to the 27 nucleotide antisense strand. Knockdown activities were normalized to a Qneg control dsRNA and presented as a normalized value of Qneg (*i.e*., Qneg represents 100% or "normal" gene expression levels). A smaller value indicates a greater knockdown effect.

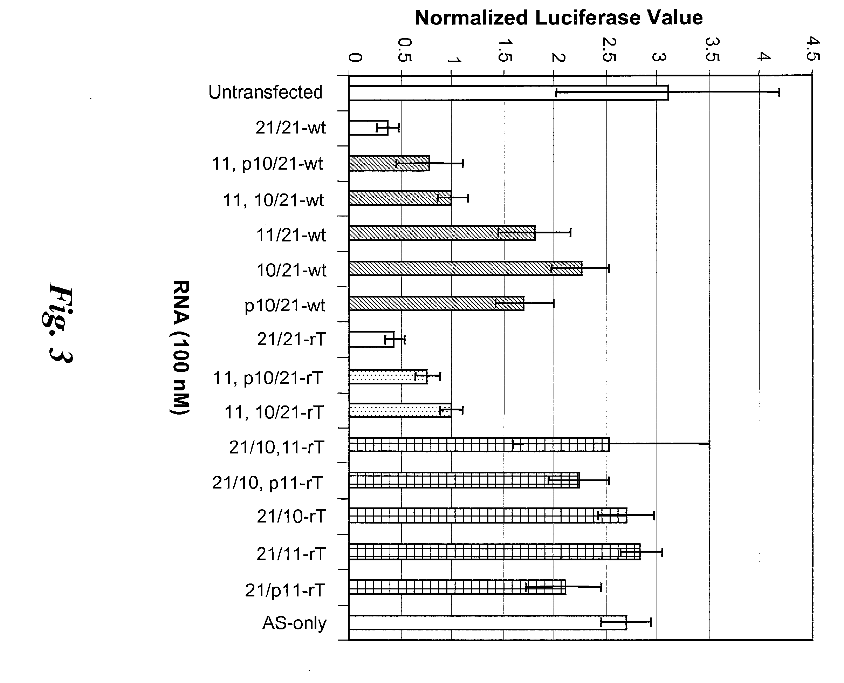

Figure 3 shows knockdown activity of a RISC activator influenza dsRNA G1498 (21/21) and nicked dsRNA (10, 11/21) at 100 nM. The "wt" designation indicates an unsubstituted RNA molecule; "rT" indicates RNA having each uridine substituted with a ribothymidine; and "p" indicates that the 5'-nucleotide of that strand was phosphorylated. The 21 nucleotide sense and antisense strands of G1498 were individually nicked between the nucleotides 10 and 11 as measured from the 5'-end, and is referred to as 11, 10/21 and 21/10, 11, respectively. The G1498 single stranded 21 nucleotide antisense strand alone (designated AS-only) was used as a control.
Figure 4 shows knockdown activity of a lacZ dicer substrate (25/27) having a nick in one of each of positions 8 to 14 and a one nucleotide gap at position 13 of the sense strand (counted from the 5'-end). A dideoxy guanosine (ddG) was incorporated at the 5'-end of the 3'-most strand of the nicked or gapped sense sequence at position 13.
Figure 5 shows knockdown activity of a dicer substrate influenza dsRNA G1498DS (25/27) and this sequence nicked at one of each of positions 8 to 14 of the sense strand, and shows the activity of these nicked molecules that are also phosphorylated or have a locked nucleic acid substitution.
Figure 6 shows a dose dependent knockdown activity a dicer substrate influenza dsRNA G1498DS (25/27) and this sequence nicked at position 13 of the sense strand.
Figure 7 shows knockdown activity of a dicer substrate influenza dsRNA G1498DS having a nick or a gap of one to six nucleotides that begins at any one of positions 8 to 12 of the sense strand.
Figure 8 shows knockdown activity of a LacZ RISC dsRNA having a nick or a gap of one to six nucleotides that begins at any one of positions 8 to 14 of the sense strand.
Figure 9 shows knockdown activity of an influenza RISC dsRNA having a nick at any one of positions 8 to 14 of the sense strand and further having one or two locked nucleic acids (LNA) per sense strand. The inserts on the right side of the graph provides a graphic depiction of the meroduplex structures (for clarity, a single antisense strand is shown at the bottom of the grouping with each of the different nicked sense strands above the antisense) having different nick positions with the relative positioning of the LNAs on the sense strands.
Figure 10 shows knockdown activity of a LacZ dicer substrate dsRNA having a nick at any one of positions 8 to 14 of the sense strand as compared to the same nicked dicer substrates but having a locked nucleic acid substitution.
Figure 11 shows the percent knockdown in influenza viral titers using influenza specific mdRNA against influenza strain WSN.
Figure 12 shows the *in vivo* reduction in PR8 influenza viral titers using influenza specific mdRNA as measured by TCID₅₀.

### DETAILED DESCRIPTION

The instant disclosure is predicated upon the unexpected discovery that a nicked or gapped double-stranded RNA (dsRNA) comprising at least three strands is a suitable substrate for Dicer or RISC and, therefore, may be advantageously employed for gene silencing via, for example, the RNA interference pathway. That is, partially duplexed dsRNA molecules described herein (also referred to as meroduplexes having a nick or gap in at least one strand) are capable of initiating an RNA interference cascade that modifies (*e.g*., reduces) expression of a target messenger RNA (mRNA) or a family of related mRNAs, such as a vascular endothelial growth factor (VEGF) mRNA or a family of VEGF mRNAs (including, for example, VEGF, VEGFB, VEGFC, FIGF, PGF). This is surprising because the thermodynamically less stable nicked or gapped dsRNA passenger strand (as compared to an intact dsRNA) would be expected to fall apart before any gene silencing effect would result (Leuschner et al., EMBO 7:314, 2006).

Exemplary meroduplex ribonucleic acid (mdRNA) molecules described herein include a first (antisense) strand that is complementary to a human VEGF mRNA as set forth in SEQ ID NO:1158, 1159, 1160, 1161, 1162, 1163, or 1164 *(i.e.,* VEGF variants 1 to 7) and is fully complementary, with up to three mismatches, to at least one other human VEGF family mRNA selected from SEQ ID NO:1165, 1166, 1167, or 1168 (*i*.*e.*, VEGFB, VEGFC, FIGF, PGF, respectively), along with second and third strands (together forming a gapped sense strand) that are each complementary to non-overlapping regions of the first strand, wherein the second and third strands can anneal with the first strand to form at least two double-stranded regions separated by a gap, and wherein at least one double-stranded region is optionally from about 5 base pairs to 13 base pairs, or the combined double-stranded regions total about 15 base pairs to about 40 base pairs and the mdRNA is optionally blunt-ended.

The gap can be from 0 nucleotides (*e.g*., a nick in which only a phosphodiester bond between two nucleotides is broken in a polynucleotide molecule) up to about 10 nucleotides (*e.g*., the first strand will have at least one unpaired nucleotide). In certain embodiments, the nick or gap is located between nucleotides 9 and 10 from the 5'-end of the second (a portion of the sense) strand or is at the Argonaute cleavage site. In another embodiment, the nick or gap is is positioned such that the thermal stability is maximized for the first and second strand duplex and for the first and third strand duplex as compared to the thermal stability of such meroduplexes having a nick or gap in a different position. Also provided herein are methods of using such dsRNA to reduce expression of a VEGF gene or VEGF gene family in a cell or to treat or prevent diseases or disorders associated with VEGF gene expression or expression of one or more VEGF gene family members, including hyperproliferative disorders (*e.g*., cancer) and inflammatory conditions (*e.g*., arthritis).

Prior to introducing more detail to this disclosure, it may be helpful to an appreciation thereof to provide definitions of certain terms to be used herein.

In the present description, any concentration range, percentage range, ratio range, or integer range is to be understood to include the value of any integer within the recited range and, when appropriate, fractions thereof (such as one tenth and one hundredth of an integer), unless otherwise indicated. Also, any number range recited herein relating to any physical feature, such as polymer subunits, size or thickness, are to be understood to include any integer within the recited range, unless otherwise indicated. As used herein, "about" or "consisting essentially of" mean ± 20% of the indicated range, value, or structure, unless otherwise indicated. As used herein, the terms "include" and "comprise" are open ended and are used synonymously. It should be understood that the terms "a" and "an" as used herein refer to "one or more" of the enumerated components. The use of the alternative (*e.g*., "or") should be understood to mean either one, both, or any combination thereof of the alternatives.

As used herein, "complementary" refers to a nucleic acid molecule that can form hydrogen bond(s) with another nucleic acid molecule or itself by either traditional Watson-Crick base pairing or other non-traditional types of pairing (*e.g*., Hoogsteen or reversed Hoogsteen hydrogen bonding) between complementary nucleosides or nucleotides. In reference to the nucleic molecules of the present disclosure, the binding free energy for a nucleic acid molecule with its complementary sequence is sufficient to allow the relevant function of the nucleic acid molecule to proceed, for example, RNAi activity, and there is a sufficient degree of complementarity to avoid non-specific binding of the nucleic acid molecule (*e.g*., dsRNA) to non-target sequences under conditions in which specific binding is desired, *e.g*., under physiological conditions in the case of *in vivo* assays or therapeutic treatment, or under conditions in which the assays are performed in the case of *in vitro* assays (*e.g.*, hybridization assays). Determination of binding free energies for nucleic acid molecules is well known in the art (*see, e.g.,* Turner et al., CSH Symp. Quant. Biol. LII:123, 1987; Frier et al., Proc. Nat'l. Acad. Sci. USA 83:9373, 1986; Turner et al., J. Am. Chem. Soc. 109:3783, 1987). Thus, "complementary" or "specifically hybridizable" or "specifically binds" are terms that indicate a sufficient degree of complementarity or precise pairing such that stable and specific binding occurs between a nucleic acid molecule (*e.g*., dsRNA) and a DNA or RNA target. It is understood in the art that a nucleic acid molecule need not be 100% complementary to a target nucleic acid sequence to be specifically hybridizable or to specifically bind. That is, two or more nucleic acid molecules may be less than fully complementary and is indicated by a percentage of contiguous residues in a nucleic acid molecule that can form hydrogen bonds with a second nucleic acid molecule.

For example, a first nucleic acid molecule may have 10 nucleotides and a second nucleic acid molecule may have 10 nucleotides, then base pairing of 5, 6, 7, 8, 9, or 10 nucleotides between the first and second nucleic acid molecules, which may or may not form a contiguous double-stranded region, represents 50%, 60%, 70%, 80%, 90%, and 100% complementarity, respectively. In certain embodiments, complementary nucleic acid molecules may have wrongly paired bases - that is, bases that cannot form a traditional Watson-Crick base pair or other non-traditional types of pair (*e.g.*, "mismatched" bases). For instance, complementary nucleic acid molecules may be identified as having a certain number of "mismatches," such as zero or about 1, about 2, about 3, about 4 or about 5.

"Perfectly" or "fully" complementary nucleic acid molecules means those in which a certain number of nucleotides of a first nucleic acid molecule hydrogen bond (anneal) with the same number of residues in a second nucleic acid molecule to form a contiguous double-stranded region. For example, two or more fully complementary nucleic acid molecule strands can have the same number of nucleotides (*i.e*., have the same length and form one double-stranded region, with or without an overhang) or have a different number of nucleotides (*e.g.*, one strand may be shorter than but fully contained within a second strand or one strand may overhang the second strand).

By "ribonucleic acid" or "RNA" is meant a nucleic acid molecule comprising at least one ribonucleotide molecule. As used herein, "ribonucleotide" refers to a nucleotide with a hydroxyl group at the 2'-position of a β-*D*-ribofuranose moiety. The term RNA includes double-stranded (ds) RNA, single-stranded (ss) RNA, isolated RNA (such as partially purified RNA, essentially pure RNA, synthetic RNA, recombinantly produced RNA), altered RNA (which differs from naturally occurring RNA by the addition, deletion, substitution or alteration of one or more nucleotides), or any combination thereof. For example, such altered RNA can include addition of non-nucleotide material, such as at one or both ends of an RNA molecule, internally at one or more nucleotides of the RNA, or any combination thereof. Nucleotides in RNA molecules of the instant disclosure can also comprise non-standard nucleotides, such as naturally occurring nucleotides, non-naturally occurring nucleotides, chemically-modified nucleotides, deoxynucleotides, or any combination thereof. These altered RNAs may be referred to as analogs or analogs of RNA containing standard nucleotides *(i.e.,* standard nucleotides, as used herein, are considered to be adenine, cytidine, guanidine, thymidine, and uridine).

The term "dsRNA" as used herein, which is interchangeable with "mdRNA," refers to any nucleic acid molecule comprising at least one ribonucleotide molecule and capable of inhibiting or down regulating gene expression, for example, by promoting RNA interference ("RNAi") or gene silencing in a sequence-specific manner. The dsRNAs (mdRNAs) of the instant disclosure may be suitable substrates for Dicer or for association with RISC to mediate gene silencing by RNAi. Examples of dsRNA molecules of this disclosure are shown in Table A herein. One or both strands of the dsRNA can further comprise a terminal phosphate group, such as a 5'-phosphate or 5', 3'-diphosphate. As used herein, dsRNA molecules, in addition to at least one ribonucleotide, can further include substitutions, chemically-modified nucleotides, and non-nucleotides. In certain embodiments, dsRNA molecules comprise ribonucleotides up to about 100% of the nucleotide positions.

In addition, as used herein, the term dsRNA is meant to be equivalent to other terms used to describe nucleic acid molecules that are capable of mediating sequence specific RNAi, for example, meroduplex RNA (mdRNA), nicked dsRNA (ndsRNA), gapped dsRNA (gdsRNA), short interfering nucleic acid (siNA), siRNA, micro-RNA (miRNA), short hairpin RNA (shRNA), short interfering oligonucleotide, short interfering substituted oligonucleotide, short interfering modified oligonucleotide, chemically-modified dsRNA, post-transcriptional gene silencing RNA (ptgsRNA), or the like. The term "large double-stranded (ds) RNA" refers to any double-stranded RNA longer than about 40 bp to about 100 bp or more, particularly up to about 300 bp to about 500 bp. The sequence of a large dsRNA may represent a segment of an mRNA or an entire mRNA. A double-stranded structure may be formed by self-complementary nucleic acid molecule or by annealing of two or more distinct complementary nucleic acid molecule strands.

In one aspect, a dsRNA comprises two separate oligonucleotides, comprising a first strand (antisense) and a second strand (sense), wherein the antisense and sense strands are self-complementary (*e.g*., each strand comprises a nucleotide sequence that is complementary to a nucleotide sequence in the other strand and the two separate strands form a duplex or double-stranded structure, for example, wherein the double-stranded region is about 15 to about 24 or 25 base pairs or about 25 or 26 to about 40 base pairs); the antisense strand comprises a nucleotide sequence that is complementary to a nucleotide sequence in a target nucleic acid molecule or a portion thereof (*e*.*g*., a human VEGF mRNA of SEQ ID NO:1158-1168, or any combination thereof); and the sense strand comprises a nucleotide sequence corresponding (*i*.*e*., homologous) to the target nucleic acid sequence or a portion thereof (*e.g*., a sense strand of about 15 to about 25 nucleotides or about 26 to about 40 nucleotides corresponds to the target nucleic acid or a portion thereof).

In another aspect, the dsRNA is assembled from a single oligonucleotide in which the self-complementary sense and antisense strands of the dsRNA are linked by together by a nucleic acid based-linker or a non-nucleic acid-based linker. In certain embodiments, the first (antisense) and second (sense) strands of the dsRNA molecule are covalently linked by a nucleotide or non-nucleotide linker as described herein and known in the art. In other embodiments, a first dsRNA molecule is covalently linked to at least one second dsRNA molecule by a nucleotide or non-nucleotide linker known in the art, wherein the first dsRNA molecule can be linked to a plurality of other dsRNA molecules that can be the same or different, or any combination thereof. In another embodiment, the linked dsRNA may include a third strand that forms a meroduplex with the linked dsRNA.

In still another aspect, dsRNA molecules described herein form a meroduplex RNA (mdRNA) having three or more strands such as, for example, an 'A' (first or antisense) strand, 'S1' (second) strand, and 'S2' (third) strand in which the 'S1' and 'S2' strands are complementary to and form base pairs (bp) with non-overlapping regions of the 'A' strand (*e.g*., an mdRNA can have the form of A:S1S2). The double-stranded region formed by the annealing of the 'S1' and 'A' strands is distinct from and non-overlapping with the double-stranded region formed by the annealing of the 'S2' and'A' strands. An mdRNA molecule is a "gapped" molecule, *e.g*., it contains a "gap" ranging from 0 nucleotides up to about 10 nucleotides (or a gap of 11, 12, 13, 14, 15, 16, 17, 18, 19, 20, 21, 22, 23, 24, 25, 26, 27, 28, 29, 30, 31, 32, 33, 34 or 35 nucleotides). In one embodiment, the A:S1 duplex is separated from the A:S2 duplex by a gap resulting from at least one unpaired nucleotide (up to about 10 unpaired nucleotides) in the 'A' strand that is positioned between the A:S1 duplex and the A:S2 duplex and that is distinct from any one or more unpaired nucleotide at the 3'-end of one or more of the 'A', 'S1', or 'S2' strands. In another embodiment, the A:S1 duplex is separated from the A:S2 duplex by a gap of zero nucleotides (*e*.*g*., a nick in which only a phosphodiester bond between two nucleotides is broken or missing in the polynucleotide molecule) between the A:S1 duplex and the A:S2 duplex - which can also be referred to as nicked dsRNA (ndsRNA). For example, A:S1S2 may be comprised of a dsRNA having at least two double-stranded regions that combined total about 14 base pairs to about 40 base pairs and the double-stranded regions are separated by a gap of 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 nucleotides, optionally having blunt ends, or A:S1S2 may comprise a dsRNA having at least two double-stranded regions spaced apart by up to 10 nucleotides and thereby forming a gap between the second and third strands wherein at least one of the double-stranded regions optionally has from 5 base pairs to 13 base pairs.

A dsRNA or large dsRNA may include a substitution or modification in which the substitution or modification may be in a phosphate backbone bond, a sugar, a base, or a nucleoside. Such nucleoside substitutions can include natural non-standard nucleosides (*e.g*., 5-methyluridine or 5-methylcytidine), and such backbone, sugar, or nucleoside modifications can include an alkyl or heteroatom substitution or addition, such as a methyl, alkoxyalkyl, halogen, nitrogen or sulfur, or any other modification known in the art.

In addition, as used herein, the term "RNAi" is meant to be equivalent to other terms used to describe sequence specific RNA interference, such as post transcriptional gene silencing, translational inhibition, or epigenetics. For example, dsRNA molecules of this disclosure can be used to epigenetically silence genes at the post-transcriptional level or the pre-transcriptional level or any combination thereof.

As used herein, "target nucleic acid" refers to any nucleic acid sequence whose expression or activity is to be altered (*e.g*., VEGF). The target nucleic acid can be DNA, RNA, or analogs thereof, and includes single, double, and multi-stranded forms. By "target site" or "target sequence" is meant a sequence within a target nucleic acid (*e.g*., mRNA) that is "targeted" for cleavage by RNAi and mediated by a dsRNA construct of this disclosure containing a sequence within the antisense strand that is complementary to the target site or sequence.

As used herein, "off-target effect" or "off-target profile" refers to the observed altered expression pattern of one or more genes in a cell or other biological sample not targeted, directly or indirectly, for gene silencing by an mdRNA or dsRNA. For example, an off-target effect can be quantified by using a DNA microarray to determine how many non-target genes have an expression level altered by about 2-fold or more in the presence of a candidate mdRNA or dsRNA, or analog thereof specific for a target sequence, such as one or more VEGF family mRNA. A "minimal off-target effect" means that an mdRNA or dsRNA affects expression by about 2-fold or more of about 25% to about 1% of the non-target genes examined or it means that the off-target effect of substituted or modified mdRNA or dsRNA (*e.g*., having at least one uridine substituted with a 5-methyluridine and optionally having at least one nucleotide modified at the 2'-position), is reduced by at least about 1% to about 80% or more as compared to the effect on non-target genes of an unsubstituted or unmodified mdRNA or dsRNA.

By "sense region" or "sense strand" is meant one ore more nucleotide sequences of a dsRNA molecule having complementarity to one ore more antisense regions of the dsRNA molecule. In addition, the sense region of a dsRNA molecule comprises a nucleic acid sequence having homology or identity to a target sequence, such as VEGF. By "antisense region" or "antisense strand" is meant a nucleotide sequence of a dsRNA molecule having complementarity to a target nucleic acid sequence, such as VEGF. In addition, the antisense region of a dsRNA molecule can comprise a nucleic acid sequence regions having complementarity to one or more sense strands of the dsRNA molecule.

"Analog" as used herein refers to a compound that is structurally similar to a parent compound (*e.g*., a nucleic acid molecule), but differs slightly in composition (e.g., one atom or functional group is different, added, or removed). The analog may or may not have different chemical or physical properties than the original compound and may or may not have improved biological or chemical activity. For example, the analog may be more hydrophilic or it may have altered activity as compared to a parent compound. The analog may mimic the chemical or biological activity of the parent compound (*e.g*., it may have similar or identical activity), or, in some cases, may have increased or decreased activity. The analog may be a naturally or non-naturally occurring (*e.g.*, chemically-modified or recombinant) variant of the original compound. An example of an RNA analog is an RNA molecule having a non-standard nucleotide, such as 5-methyuridine or 5-methylcytidine, which may impart certain desirable properties (*e.g*., improve stability, bioavailability, minimize off-target effects or interferon response).

As used herein, the term "universal base" refers to nucleotide base analogs that form base pairs with each of the standard DNA/RNA bases with little discrimination between them. A universal base is thus interchangeable with all of the standard bases when substituted into a nucleotide duplex (*see, e.g.,* Loakes et al., J. Mol. Bio. 270:426, 1997). Examplary universal bases include C-phenyl, C-naphthyl and other aromatic derivatives, inosine, azole carboxamides, or nitroazole derivatives such as 3-nitropyrrole, 4-nitroindole, 5-nitroindole, and 6-nitroindole (*see, e.g.,* Loakes, Nucleic Acids Res. 29:2437, 2001).

The term "gene" as used herein, especially in the context of "target gene" or "gene target" for RNAi, means a nucleic acid molecule that encodes an RNA or a transcription product of such gene, including a messenger RNA (mRNA, also referred to as structural genes that encode for a polypeptide), an mRNA splice variant of such gene, a functional RNA (fRNA), or non-coding RNA (ncRNA), such as small temporal RNA (stRNA), microRNA (miRNA), small nuclear RNA (snRNA), short interfering RNA (siRNA), small nucleolar RNA (snRNA), ribosomal RNA (rRNA), transfer RNA (tRNA) and precursor RNAs thereof. Such non-coding RNAs can serve as target nucleic acid molecules for dsRNA mediated RNAi to alter the activity of the target RNA involved in functional or regulatory cellular processes.

As used herein, "gene silencing" refers to a partial or complete loss-of-function through targeted inhibition of gene expression in a cell, which may also be referred to as RNAi "knockdown," "inhibition," "down-regulation," or "reduction" of expression of a target gene, such as a human VEGF gene. Depending on the circumstances and the biological problem to be addressed, it may be preferable to partially reduce gene expression. Alternatively, it might be desirable to reduce gene expression as much as possible. The extent of silencing may be determined by methods described herein and as known in the art, some of which are summarized in PCT Publication No. WO 99/32619. Depending on the assay, quantification of gene expression permits detection of various amounts of inhibition that may be desired in certain embodiments of this disclosure, including prophylactic and therapeutic methods, which will be capable of knocking down target gene expression, in terms of mRNA level or protein level or activity, for example, by equal to or greater than 10%, 30%, 50%, 75% 90%, 95% or 99% of baseline (*e.g*., normal) or other control levels, including elevated expression levels as may be associated with particular disease states or other conditions targeted for therapy.

As used herein, the term "therapeutically effective amount" means an amount of dsRNA that is sufficient to result in a decrease in severity of disease symptoms, an increase in frequency or duration of disease symptom-free periods, or a prevention of impairment or disability due to the disease, in the subject (*e.g*., human) to which it is administered. For example, a therapeutically effective amount of dsRNA directed against an mRNA of VEGF (*e.g*., SEQ ID NO:1158-1168, or any combination thereof) can inhibit cell growth or hyperproliferative (*e.g*., neoplastic) cell growth by at least about 20%, at least about 40%, at least about 60%, or at least about 80% relative to untreated subjects. A therapeutically effective amount of a therapeutic compound can decrease, for example, tumor size or otherwise ameliorate symptoms in a subject. One of ordinary skill in the art would be able to determine such therapeutically effective amounts based on such factors as the subject's size, the severity of symptoms, and the particular composition or route of administration selected. The nucleic acid molecules of the instant disclosure, individually, or in combination or in conjunction with other drugs, can be used to treat diseases or conditions discussed herein. For example, to treat a particular disease, disorder, or condition, the dsRNA molecules can be administered to a patient or can be administered to other appropriate cells evident to those skilled in the art, individually or in combination with one or more drugs, under conditions suitable for treatment.

Also, one or more dsRNA may be used to knockdown expression of a VEGF family mRNA as set forth in any one or more of SEQ ID NO:1158-1168, or a related mRNA splice variant. In this regard it is noted that a VEGF family gene may be transcribed into two or more mRNA splice variants; and thus, for example, in certain embodiments, knockdown of one mRNA splice variant without affecting the other mRNA splice variant may be desired, or vice versa; or knockdown of all transcription products may be targeted.

In addition, it should be understood that the individual compounds, or groups of compounds, derived from the various combinations of the structures and substituents described herein, are disclosed by the present application to the same extent as if each compound or group of compounds was set forth individually. Thus, selection of particular structures or particular substituents is within the scope of the present disclosure. As described herein, all value ranges are inclusive over the indicated range. Thus, a range of C₁-C₄ will be understood to include the values of 1, 2, 3, and 4, such that C₁, C₂, C₃ and C₄ are included.

The term "alkyl" as used herein refers to saturated straight- or branched-chain aliphatic groups containing from 1-20 carbon atoms, preferably 1-8 carbon atoms and most preferably 1-4 carbon atoms. This definition applies as well to the alkyl portion of alkoxy, alkanoyl and aralkyl groups. The alkyl group may be substituted or unsubstituted. In certain embodiments, the alkyl is a (C₁-C₄) alkyl or methyl.

The term "cycloalkyl" as used herein refers to a saturated cyclic hydrocarbon ring system containing from 3 to 12 carbon atoms that may be optionally substituted. Exemplary embodiments include, but are not limited to, cyclopropyl, cyclobutyl, cyclopentyl and cyclohexyl. In certain embodiments, the cycloalkyl group is cyclopropyl. In another embodiment, the (cycloalkyl)alkyl groups contain from 3 to 12 carbon atoms in the cyclic portion and 1 to 6 carbon atoms in the alkyl portion. In certain embodiments, the (cycloalkyl)alkyl group is cyclopropylmethyl. The alkyl groups are optionally substituted with from one to three substituents selected from the group consisting of halogen, hydroxy and amino.

The terms "alkanoyl" and "alkanoyloxy" as used herein refer, respectively, to -C(O)-alkyl groups and -O-C(=O)- alkyl groups, each optionally containing 2 to 10 carbon atoms. Specific embodiments of alkanoyl and alkanoyloxy groups are acetyl and acetoxy, respectively.

The term "alkenyl" refers to an unsaturated branched, straight-chain or cyclic alkyl group having 2 to 15 carbon atoms and having at least one carbon-carbon double bond derived by the removal of one hydrogen atom from a single carbon atom of a parent alkene. The group may be in either the *cis* or *trans* conformation about the double bond(s). Certain embodiments include ethenyl, 1-propenyl, 2-propenyl, 1-methylethenyl, 1-butenyl, 2-butenyl, 3-butenyl, 2-methyl-2-propenyl, 1-pentenyl, 2-pentenyl, 4-pentenyl, 3-methyl-2-butenyl, 1-hexenyl, 2-hexenyl, 1-heptenyl, 2-heptenyl, 1-octenyl, 2-octenyl, 1,3-octadienyl, 2-nonenyl, 1,3-nonadienyl, 2-decenyl, etc., or the like. The alkenyl group may be substituted or unsubstituted.

The term "alkynyl" as used herein refers to an unsaturated branched, straight-chain, or cyclic alkyl group having 2 to 10 carbon atoms and having at least one carbon-carbon triple bond derived by the removal of one hydrogen atom from a single carbon atom of a parent alkyne. Exemplary alkynyls include ethynyl, 1-propynyl, 2-propynyl, 1-butynyl, 2-butynyl, 3-butynyl, 1-pentynyl, 2-pentynyl, 4-pentynyl, 1-octynyl, 6-methyl-1-heptynyl, 2-decynyl, or the like. The alkynyl group may be substituted or unsubstituted.

The term "hydroxyalkyl" alone or in combination, refers to an alkyl group as previously defined, wherein one or several hydrogen atoms, preferably one hydrogen atom has been replaced by a hydroxyl group. Examples include hydroxymethyl, hydroxyethyl and 2-hydroxyethyl.

The term "aminoalkyl" as used herein refers to the group -NRR', where R and R' may independently be hydrogen or (C₁-C₄) alkyl.

The term "alkylaminoalkyl" refers to an alkylamino group linked via an alkyl group (e.g., a group having the general structure -alkyl-NH-alkyl or -alkyl-N(alkyl)(alkyl)). Such groups include, but are not limited to, mono- and di-(C₁-C₈ alkyl)aminoC₁-C₈ alkyl, in which each alkyl may be the same or different.

The term "dialkylaminoalkyl" refers to alkylamino groups attached to an alkyl group. Examples include, but are not limited to, N,N-dimethylaminomethyl, N,N-dimethylaminoethyl N,N-dimethylaminopropyl, and the like. The term dialkylaminoalkyl also includes groups where the bridging alkyl moiety is optionally substituted.

The term "haloalkyl" refers to an alkyl group substituted with one or more halo groups, for example chloromethyl, 2-bromoethyl, 3-iodopropyl, trifluoromethyl, perfluoropropyl, 8-chlorononyl, or the like.

The term "carboxyalkyl" as used herein refers to the substituent -R^{Z}-COOH, wherein R¹⁰ is alkylene; and carbalkoxyalkyl refers to -R¹⁰-C(=O)OR¹¹, wherein R¹⁰ and R¹¹ are alkylene and alkyl respectively. In certain embodiments, alkyl refers to a saturated straight- or branched-chain hydrocarbyl radical of 1 to 6 carbon atoms such as methyl, ethyl, n-propyl, isopropyl, n-butyl, t-butyl, n-pentyl, 2-methylpentyl, n-hexyl, and so forth. Alkylene is the same as alkyl except that the group is divalent.

The term "alkoxy" includes substituted and unsubstituted alkyl, alkenyl, and alkynyl groups covalently linked to an oxygen atom. In one embodiment, the alkoxy group contains 1 to about 10 carbon atoms. Embodiments of alkoxy groups include, but are not limited to, methoxy, ethoxy, isopropyloxy, propoxy, butoxy, and pentoxy groups. Embodiments of substituted alkoxy groups include halogenated alkoxy groups. In a further embodiment, the alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Exemplary halogen substituted alkoxy groups include, but are not limited to, fluoromethoxy, difluoromethoxy, trifluoromethoxy, chloromethoxy, dichloromethoxy, and trichloromethoxy.

The term "alkoxyalkyl" refers to an alkylene group substituted with an alkoxy group. For example, methoxyethyl (CH₃OCH₂CH₂-) and ethoxymethyl (CH₃CH₂OCH₂-) are both C₃ alkoxyalkyl groups.

The term "aryl" as used herein refers to monocyclic or bicyclic aromatic hydrocarbon groups having from 6 to 12 carbon atoms in the ring portion, for example, phenyl, naphthyl, biphenyl and diphenyl groups, each of which may be substituted with, for example, one to four substituents such as alkyl; substituted alkyl as defmed above, halogen, trifluoromethyl, trifluoromethoxy, hydroxy, alkoxy, cycloalkyloxy, alkanoyl, alkanoyloxy, amino, alkylamino, dialkylamino, nitro, cyano, carboxy, carboxyalkyl, carbamyl, carbamoyl and aryloxy. Specific embodiments of aryl groups in accordance with the present disclosure include phenyl, substituted phenyl, naphthyl, biphenyl, and diphenyl.

The term "aroyl," as used alone or in combination herein, refers to an aryl radical derived from an aromatic carboxylic acid, such as optionally substituted benzoic or naphthoic acids.

The term "aralkyl" as used herein refers to an aryl group bonded to the 2-pyridinyl ring or the 4-pyridinyl ring through an alkyl group, preferably one containing 1 to 10 carbon atoms. A preferred aralkyl group is benzyl.

The term "carboxy," as used herein, represents a group of the formula -C(=O)OH or -C(=O)O⁻.

The term "carbonyl" as used herein refers to a group in which an oxygen atom is double-bonded to a carbon atom -C=O.

The term "trifluoromethyl" as used herein refers to -CF₃.

The term "trifluoromethoxy" as used herein refers to -OCF₃.

The term "hydroxyl" as used herein refers to -OH or -O⁻.

The term "nitrile" or "cyano" as used herein refers to the group -CN.

The term "nitro," as used herein alone or in combination refers to a -NO₂ group.

The term "amino" as used herein refers to the group -NR⁹R⁹, wherein R⁹ may independently be hydrogen, alkyl, aryl, alkoxy, or heteroaryl. The term "aminoalkyl" as used herein represents a more detailed selection as compared to "amino" and refers to the group -NRʹRʹ, wherein Rʹ may independently be hydrogen or (C₁-C₄) alkyl. The term "dialkylamino" refers to an amino group having two attached alkyl groups that can be the same or different.

The term "alkanoylamino" refers to alkyl, alkenyl or alkynyl groups containing the group -C(=O)- followed by -N(H)-, for example acetylamino, propanoylamino and butanoylamino and the like.

The term "carbonylamino" refers to the group -NRʹ-CO-CH₂-Rʹ, wherein R' is independently selected from hydrogen or (C₁-C₄) alkyl.

The term "carbamoyl" as used herein refers to -O-C(O)NH₂.

The term "carbamyl" as used herein refers to a functional group in which a nitrogen atom is directly bonded to a carbonyl, *e.g*., as in -NR"C(=O)R" or -C(=O)NR"R", wherein R" can be independently hydrogen, alkyl, substituted alkyl, alkenyl, substituted alkenyl, alkoxy, cycloalkyl, aryl, heterocyclo, or heteroaryl.

The term "alkylsulfonylamino" refers to refers to the group -NHS(O)₂R¹², wherein R¹² is alkyl.

The term "halogen" as used herein refers to bromine, chlorine, fluorine or iodine. In one embodiment, the halogen is fluorine. In another embodiment, the halogen is chlorine.

The term "heterocyclo" refers to an optionally substituted, unsaturated, partially saturated, or fully saturated, aromatic or nonaromatic cyclic group that is a 4 to 7 membered monocyclic, or 7 to 11 membered bicyclic ring system that has at least one heteroatom in at least one carbon atom-containing ring. The substituents on the heterocyclo rings may be selected from those given above for the aryl groups. Each ring of the heterocyclo group containing a heteroatom may have 1, 2, or 3 heteroatoms selected from nitrogen, oxygen or sulfur. Plural heteroatoms in a given heterocyclo ring may be the same or different.

Exemplary monocyclic heterocyclo groups include pyrrolidinyl, pyrrolyl, indolyl, pyrazolyl, imidazolyl, oxazolyl, isoxazolyl, thiazolyl, furyl, tetrahydrofuryl, thienyl, piperidinyl, piperazinyl, azepinyl, pyrimidinyl, pyridazinyl, tetrahydropyranyl, morpholinyl, dioxanyl, triazinyl and triazolyl. Preferred bicyclic heterocyclo groups include benzothiazolyl, benzoxazolyl, benzothienyl, quinolinyl, tetrahydroisoquinolinyl, benzimidazolyl, benzofuryl, indazolyl, benzisothiazolyl, isoindolinyl and tetrahydroquinolinyl. In more detailed embodiments heterocyclo groups may include indolyl, imidazolyl, furyl, thienyl, thiazolyl, pyrrolidyl, pyridyl and pyrimidyl.

"Substituted" refers to a group in which one or more hydrogen atoms are each independently replaced with the same or different substituent(s). Representative substituents include -X, -R⁶, -O-, =O, -OR, -SR⁶, -S-, =S, -NR⁶R⁶, =NR⁶, -CX₃, -CF₃, -CN, -OCN, -SCN, -NO, -NO₂, =N₂, -N₃, -S(=O)₂2O-, -S(=O)₂OH, -S(=O)₂R⁶, -OS(=O)₂O-, -OS(=O)₂OH, -OS(=O)₂R⁶, -P(=O)(O⁻)₂, -P(=O)(OH)(O⁻), -OP(=O)₂(O⁻), -C(-O)R⁶, -C(=S)R⁶, -C(=O)OR⁶, -C(=O)O-, -C(=S)OR⁶, -NR⁶-C(=O)-N(R⁶)₂, -NR⁶-C(=S)-N(R⁶)₂, and -C(=NR⁶)NR⁶R⁶, wherein each X is independently a halogen; and each R⁶ is independently hydrogen, halogen, alkyl, aryl, arylalkyl, arylaryl, arylheteroalkyl, heteroaryl, heteroarylalkyl, NR⁷R⁷, -C(=O)R⁷, and -S(=O)₂R⁷; and each R⁷ is independently hydrogen, alkyl, alkanyl, alkynyl, aryl, arylalkyl, arylheteralkyl, arylaryl, heteroaryl or heteroarylalkyl. Aryl containing substituents, whether or not having one or more substitutions, may be attached in a para (*p*-), meta (*m*-) or ortho (*o*-) conformation, or any combination thereof.

### Vascular Endothelial Growth Factor (VEGF) Family and Exemplary dsRNA Molecules

VEGF, also known as vascular endothelial growth factor (VEGFA or VEGF-A), is a pro-angiogenic factor involved in tumor angiogenesis having a variety of functions, including: (a) increasing vascular permeability, which might facilitate tumor dissemination via the circulation causing a greater delivery of oxygen and nutrients; (b) recruiting circulating endothelial precursor cells, and (c) acting as a survival factor for immature tumor blood vessels. VEGF expression or overexpression has been shown to be a mediator of angiogenesis across multiple tumor types, including colorectal, lung, breast and other cancers. VEGFA is expressed as eight different isoforms (VEGF₂₀₆, isoform a; VEGF₁₈₉, isoform b; VEGF₁₈₃, isoform c; VEGF₁₆₅, isoform d; VEGF₁₄₈, isoform e; VEGF₁₂₁, isoform f; VEGF_{165b}, isoform g; and VEGF₁₄₅). The VEGF₁₆₅ isoform appears to be the predominant and most potent mitogenic isoform secreted by normal and malignant cells.

As set forth above, VEGF (also known as vascular permeability factor, VPF, vascular endothelial growth factor, VEGFA, MGC70609) is a potent secreted mitogen critical for physiologic and tumor angiogenesis. More detail regarding VEGF and related disorders are described at www.ncbi.nlm.nih.gov/entrez/query.fcgi?db=OMIM, which is part of the Online Mendelian Inheritance in Man database (OMIM Accession No. 192240). The various human VEGF mRNA sequences have Genbank accession numbers NM_001025366.1 (transcript variant 1; SEQ ID NO:1158); NM_003376.4 (transcript variant 2; SEQ ID NO:1159); NM_001025367.1 (transcript variant 3; SEQ ID NO:1160); NM_001025368.1 (transcript variant 4; SEQ ID NO:1161); NM_001025369.1 (transcript variant 5; SEQ ID NO:1162); NM_001025370.1 (transcript variant 6; SEQ ID NO:1163); and NM_001033756.1 (transcript variant 7; SEQ ID NO:1164). As used herein, reference to a VEGF mRNA or RNA sequence or sense strand means an RNA having a sequence of any VEGF isoform as set forth in SEQ ID NO:1158, 1159, 1160, 1161, 1162, 1163, or 1164, as well as variants and homologs having at least 80% or more identity with the human VEGF sequence as set forth in SEQ ID NO:1158, 1159, 1160, 1161, 1162, 1163, or 1164.

The other human VEGF family members are implicated in a variety of diseases and disorders. Expression of VEGFB, VEGFC, FIGF, or PGF has been implicated in a variety of diseases and disorders, including hyperproliferative diseases, angiogenic diseases, lymphangiogenic diseases, and inflammatory disorders. More detail regarding VEGFB (also known as VEGF-related factor, VRF, VEGFL), VEGFC (also known as VEGF-related protein, VRP, Flt4-L), FIGF (also known as vascular endothelial growth factor D, VEGFD), and PGF (also known as placental growth factor, vascular endothelial growth factor-related protein; PLGF, PlGF; PlGF-2), along with any related disorders are described in the Online Mendelian Inheritance in Man database at www.ncbi.nlm.nih.gov/entrez/query.fcgi?db=OMIM (OMIM Accession Nos. 601398, 601528, 300091, and 601121, respectively). The complete human VEGFB, VEGFC, FIGF, and PGF mRNA sequences have GenBank accession number NM_003377.3 (SEQ ID NO:1165), NM_005429.2 (SEQ ID NO:1166), NM_004469.2 (SEQ ID NO:1167), and NM_002632.4 (SEQ ID NO:1168), respectively. As used herein, reference to VEGFB, VEGFC, FIGF, and PGF mRNAs or RNA sequences or sense strands means an RNA encompassed by SEQ ID NOS:1165, 1166, 1167, and 1168, respectively, as well as variants, isoforms, and homologs having at least 80% or more identity with the human VEGFB, VEGFC, FIGF, and PGF sequence as set forth in SEQ ID NO:1165, 1166, 1167, or 1168, respectively.

The "percent identity" between two or more nucleic acid sequences is a function of the number of identical positions shared by the sequences (*i.e*., % identity = number of identical positions / total number of positions x 100), taking into account the number of gaps, and the length of each gap that needs to be introduced to optimize alignment of two or more sequences. The comparison of sequences and determination of percent identity between two or more sequences can be accomplished using a mathematical algorithm, such as BLAST and Gapped BLAST programs at their default parameters (e.g., Altschul et al., J. Mol. Biol. 215:403, 1990; see also BLASTN at www.ncbi.nlm.nih.gov/BLAST).

In one aspect, the instant disclosure provides a meroduplex ribonucleic acid (mdRNA) molecule, comprising a first strand that is complementary to VEGF mRNA as set forth in SEQ ID NO:1158, 1159, 1160, 1161, 1162, 1163, or 1164 *(i.e.,* VEGF variants 1 to 7) and is fully complementary, with up to three mismatches, to at least one other human VEGF family mRNA selected from SEQ ID NO:1165, 1166, 1167, or 1168 *(i.e.,* VEGFB, VEGFC, FIGF, PGF, respectively), and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein (a) the mdRNA molecule optionally has at least one double-stranded region of 5 base pairs to 13 base pairs, or (b) wherein the combined double-stranded regions total about 15 base pairs to about 40 base pairs and the mdRNA molecule optionally has blunt ends; wherein at least one pyrimidine of the mdRNA is substituted with a pyrimidine nucleoside according to Formula I or II: 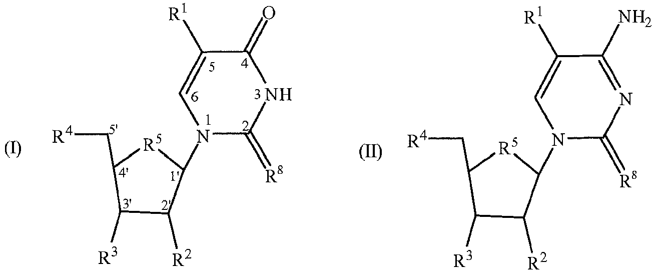 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C≡N, or heterocyclo group; R³ and R⁴ are each independently a hydroxyl, a protected hydroxyl, a phosphate, or an internucleoside linking group; and R⁵ and R⁸ are each independently O or S. In certain embodiments, at least one nucleoside is according to Formula I in which R¹ is methyl and R² is -OH, or R¹ is methyl, R² is -OH, and R⁸ is S. In other embodiments, the internucleoside linking group covalently links from about 5 to about 40 nucleosides. In some embodiments, the gap comprises at least one unpaired nucleotide in the first strand positioned between the double-stranded regions formed by the second and third strands when annealed to the first strand, or the gap is a nick. In certain embodiments, the nick or gap is located 10 nucleotides from the 5'-end of the first (antisense) strand or at the Argonaute cleavage site. In another embodiment, the meroduplex nick or gap is positioned such that the thermal stability is maximized for the first and second strand duplex and for the first and third strand duplex as compared to the thermal stability of such meroduplexes having a nick or gap in a different position.

In still another aspect, the instant disclosure provides an mdRNA molecule, comprising a first strand that is complementary to vascular endothelial growth factor (VEGF) mRNA as set forth in SEQ ID NO:1158, 1159, 1160, 1161, 1162, 1163, or 1164 and is fully complementary, with up to three mismatches, to at least one other human VEGF family mRNA selected from SEQ ID NO:1165, 1166, 1167, or 1168, and a second strand and a third strand that are each complementary to non-overlapping regions of the first strand, wherein the second strand and third strands can anneal with the first strand to form at least two double-stranded regions spaced apart by up to 10 nucleotides and thereby forming a gap between the second and third strands, and wherein the mdRNA molecule optionally includes at least one double-stranded region of about 5 base pairs to 13 base pairs. In a further aspect, the instant disclosure provides an mdRNA molecule having a first strand that is complementary to a VEGF mRNA as set forth in SEQ ID NO:1158, 1159, 1160, 1161, 1162, 1163, or 1164 and is fully complementary, with up to three mismatches, to at least one other human VEGF family mRNA selected from SEQ ID NO:1165, 1166, 1167, or 1168, and a second strand and a third strand that are each complementary to non-overlapping regions of the first strand, wherein the second strand and third strands can anneal with the first strand to form at least two double-stranded regions spaced apart by up to 10 nucleotides and thereby forming a gap between the second and third strands, and wherein the combined double-stranded regions total about 15 base pairs to about 40 base pairs and the mdRNA molecule optionally has blunt ends. In some embodiments, the gap comprises at least one unpaired nucleotide in the first strand positioned between the double-stranded regions formed by the second and third strands when annealed to the first strand, or the gap is a nick. In certain embodiments, the nick or gap is located between nucleotides 9 and 10 from the 5'-end of the second (a portion of the sense) strand or at the Argonaute cleavage site. In another embodiment, the nick or gap is located in a position wherein each of the two or more nicked or gapped strands has a maximal melting temperature (*i.e*., Tₘ or temperature at which 50% of one of the nicked or gapped strands is annealed to the first strand).

As provided herein, any of the aspects or embodiments disclosed herein would be useful in treating VEGF or VEGF family-associated diseases or disorders, such as hyperproliferative disease (e.g., cancer) or inflammatory disorders (*e.g*., arthritis). An advantage of the instant disclosure is the ability to use a single dsRNA to knockdown mRNA expression of one or more VEGF family member. For example, one or more dsRNA may be used to knockdown expression of VEGF mRNA as set forth in SEQ ID NO:1158-1168, or any combination thereof. In one embodiment, one or more dsRNA can be used to knockdown SEQ ID NO: 1158-1168 - that is, any of the VEGF variants, VEGFB, VEGFC, FIGF, and PGF. In another embodiment, one or more dsRNA can be used to knockdown SEQ ID NO:1158-1164 or SEQ ID NO:1158-1162 and 1164 - that is , any of the VEGF variants or VEGF variants 1-5 and 7, respectively.

In certain embodiments, one or more dsRNA can be used to knockdown SEQ ID NO:1158-1165 - that is, any of the VEGF variants and VEGFB. In further embodiments, one or more dsRNA can be used to knockdown SEQ ID NO:1158-1162, 1164 and 1165 - that is, any of VEGF variants 1-5 and 7, and VEGFB. In further embodiments, one or more dsRNA can be used to knockdown SEQ ID NO:1158-1164 and 1166 - that is, any of the VEGF variants and VEGFC. In further embodiments, one or more dsRNA can be used to knockdown SEQ ID NO:1158-1164 and 1167 - that is, any of the VEGF variants and FIGF. In further embodiments, one or more dsRNA can be used to knockdown SEQ ID NO:1158-1164 and 1168 - that is, any of the VEGF variants and PGF. In further embodiments, one or more dsRNA can be used to knockdown SEQ ID NO:1158-1165 and 1167 - that is, any of the VEGF variants, VEGFB, and FIGF. In further embodiments, one or more dsRNA can be used to knockdown SEQ ID NO:1158-1165 and 1168 - that is, any of the VEGF variants, VEGFB, and PGF. In further embodiments, one or more dsRNA can be used to knockdown SEQ ID NO:1158-1164, 1166, and 1167 - that is, any of the VEGF variants, VEGFC, and FIGF. In further embodiments, one or more dsRNA can be used to knockdown SEQ ID NO: 1158-1167 - that is, any of the VEGF variants, VEGFB, VEGFC, and FIGF. In further embodiments, one or more dsRNA can be used to knockdown SEQ ID NO:1165 and 1166 or 1165 and 1167 - that is, VEGFB and VEGFC or VEGFB and FIGF. In further embodiments, one or more dsRNA SEQ ID NO:1166 and 1167 - that is, VEGFC and FIGF or FIGF and VEGFC.

In some embodiments, the dsRNA comprises at least three strands in which the first strand comprises about 5 nucleotides to about 40 nucleotides, and the second and third strands include each, individually, about 5 nucleotides to about 20 nucleotides, wherein the combined length of the second and third strands is about 15 nucleotides to about 40 nucleotides. In other embodiments, the dsRNA comprises at least two or three strands in which the first strand comprises about 15 nucleotides to about 24 nucleotides or about 25 nucleotides to about 40 nucleotides. In further embodiments, the first strand will be complementary to at least about 15, 19, 20, 21, 22, 23, 24, 25, 26, 27, 28, 29, 30, 31, 32, 33, 34, 35, 36, 37, 38, 39, or 40 contiguous nucleotides of a second strand or a second and third strand or to a plurality of strands. In certain embodiments, the second and third strand or the plurality of strands complementary to the first strand have a nick or gap that is located between nucleotides 9 and 10 from the 5'-end of the second (a portion of the sense) strand or at the Argonaute cleavage site or within 5 to 10 nucleotides of the Argonaute cleavage site. In another embodiment, the nick or gap is located in a position wherein each of the two or more nicked or gapped strands has a maximal melting temperature (*i.e.*, Tₘ or temperature at which 50% of one of the nicked or gapped strands is annealed to the first strand).

In further examples, the first strand and its complement(s) will be able to form dsRNA and mdRNA molecules of this disclosure with about 19 to about 25 nucleotides of the first strand that is complementary to a VEGF or VEGF family mRNA. For example, a Dicer substrate dsRNA can have about 25 nucleotides to about 40 nucleotides, but only 19 nucleotides of the antisense (first) strand will be complementary to a VEGF or VEGF family mRNA. In further embodiments, the first strand can have complementarity to a VEGF or VEGF family mRNA in about 19 nucleotides to about 25 nucleotides and have zero, one, two, or three mismatches with the VEGF or VEGF family mRNA, such as a sequence set forth in SEQ ID NO:1158-1168, or any combination thereof, or the first strand of 19 nucleotides to about 25 nucleotides, that for example activates or is capable of loading into RISC, will have at least 80% identity with the corresponding nucleotides found in a VEGF or VEGF family mRNA, such as a sequence set forth in SEQ ID NO:1158-1168, or any combination thereof.

In certain embodments, one or more dsRNA comprise a first strand having full complementarity to SEQ ID NO:1158-1164, and having zero, one, two, or three mismatches with a sequence set forth in SEQ ID NO:1165 - that is, full complementarity with VEGF variants and up to three mismatches with VEGFB. In further embodiments, one or more dsRNA comprise a first strand having full complementarity to SEQ ID NO:1158-1164, and having two or three mismatches with a sequence set forth in SEQ ID NO:1166 - that is, full complementarity with VEGF variants and two to three mismatches with VEGFC. In further embodiments, one or more dsRNA comprise a first strand having full complementarity to SEQ ID NO:1158-1164, and having three mismatches with a sequence set forth in SEQ ID NOS:1167 or 1168 - that is, full complementarity with all VEGF variants and three mismatches with FIGF or PGF. In further embodiments, one or more dsRNA comprise a first strand having full complementarity to SEQ ID NO:1165, and having two or three mismatches with a sequence set forth in SEQ ID NOS: 1166 or 1167 - that is, full complementarity with VEGFB and two to three mismatches with VEGFC or FIGF. In further embodiments, one or more dsRNA comprise a first strand having full complementarity to SEQ ID NO:1166, and having two or three mismatches with a sequence set forth in SEQ ID NO:1167 - that is, full complementarity with VEGFC and two to three mismatches with FIGF. In further embodiments, one or more dsRNA comprise a first strand having full complementarity to SEQ ID NO:1167, and having two or three mismatches with a sequence set forth in SEQ ID NOS:1165 or 1166 - that is, full complementarity with FIGF and two to three mismatches with VEGFB or VEGFC.

Certain illustrative sense strand molecules that can be used to design mdRNA molecules as described herein, can be found in Table A of U.S. Provisional Patent Application No. 60/932,949 (filed May 3, 2007) and in the Sequence Listing submitted herewith (text file named "07-R011PCT_Sequence_Listing", created February 21, 2008 and having a size of 369 kilobytes), which are both herein incorporated by reference. In addition, the content of Table B as disclosed in U.S. Provisional Patent Application No. 60/934,930 (filed March 16, 2007), which was submitted with that application as a separate text file named "Table_B_Human_RefSeq_Accession_Numbers.txt" (created March 16, 2007 and having a size of 3,604 kilobytes), is incorporated herein by reference in its entirety.

### Substituting and Modifying VEGF dsRNA Molecules

The introduction of substituted and modified nucleotides into mdRNA and dsRNA molecules of this disclosure provides a powerful tool in overcoming potential limitations of *in vivo* stability and bioavailability inherent to native RNA molecules (*e.g.*, having standard nucleotides) that are exogenously delivered. For example, the use of dsRNA molecules of this disclosure can enable a lower dose of a particular nucleic acid molecule for a given therapeutic effect (*e.g*., reducing or silencing VEGF expression) since dsRNA molecules of this disclosure tend to have a longer half-life in serum. Furthermore, certain substitutions and modifications can improve the bioavailability of dsRNA by targeting particular cells or tissues or improving cellular uptake of the dsRNA molecules. Therefore, even if the activity of a dsRNA molecule of this disclosure is reduced as compared to a native RNA molecule, the overall activity of the substituted or modified dsRNA molecule can be greater than that of the native RNA molecule due to improved stability or delivery of the molecule. Unlike native unmodified dsRNA, substituted and modified dsRNA can also minimize the possibility of activating the interferon response in, for example, humans.

In certain embodiments, a dsRNA molecule of this disclosure has at least one uridine, at least three uridines, or each and every uridine (e.g., all uridines) of the first (antisense) strand of the dsRNA is a 5-methyluridine, 2-thioribothymidine, 2'-O-methyl-5-methyluridine, or any combination thereof. In a related embodiment, the dsRNA molecule or analog thereof of this disclosure has at least one uridine, at least three uridines, or each and every uridine of the second (sense) strand of the dsRNA substituted or replaced with 5-methyluridine, 2-thioribothymidine, 2'-O-methyl-5-methyluridine, or any combination thereof. In a related embodiment, the dsRNA molecule or analog thereof of this disclosure has at least one uridine, at least three uridines, or each and every uridine of the third (sense) strand of the dsRNA substituted or replaced with 5-methyluridine, 2-thioribothymidine, 2'-O-methyl-5-methyluridine, or any combination thereof. In still another embodiment, the dsRNA molecule or analog thereof of this disclosure has at least one uridine, at least three uridines, or each and every uridine of both the first (antisense) and second (sense) strands; of both the first (antisense) and third (sense) strands; of both the second (sense) and third (sense) strands; or of all of the first (antisense), second (sense) and third (sense) strands of the dsRNA substituted or replaced with 5-methyluridine, 2-thioribothymidine, 2'-O-methyl-5-methyluridine, or any combination thereof. In some embodiments, the double-stranded region of a dsRNA molecule has at least three 5-methyluridines, 2-thioribothymidine, 2'-O-methyl-5-methyluridine, or any combination thereof. In certain embodiments, dsRNA molecules comprise ribonucleotides at about 5% to about 95% of the nucleotide positions in one strand, both strands, or any combination thereof.

In further embodiments, a dsRNA molecule that decreases expression of one or more VEGF family gene by RNAi according to the instant disclosure further comprises one or more natural or synthetic non-standard nucleoside. In related embodiments, the non-standard nucleoside is one or more deoxyuridine, locked nucleic acid (LNA) molecule, a modified base (*e.g*., 5-methyluridine), a universal-binding nucleotide, a 2'-O-methyl nucleotide, a modified internucleoside linkage (*e.g*., phosphorothioate), a G clamp, or any combination thereof. In certain embodiments, the universal-binding nucleotide can be C-phenyl, C-naphthyl, inosine, azole carboxamide, 1-β-*D*-ribofuranosyl-4-nitroindole, 1-β-*D*-ribofuranosyl-5-nitroindole, 1-β-*D-*ribofuranosyl-6-nitroindole, or 1-β-*D*-ribofuranosyl-3-nitropyrrole.

Substituted or modified nucleotides present in dsRNA molecules, preferably in the sense or antisense strand, but also optionally in both the antisense and sense strands, comprise modified or substituted nucleotides according to this disclosure having properties or characteristics similar to natural or standard ribonucleotides. For example, this disclosure features dsRNA molecules including nucleotides having a Northern conformation (e.g., Northern pseudorotation cycle; *see, e.g.,* Saenger, Principles of Nucleic Acid Structure, Springer-Verlag ed., 1984). As such, chemically modified nucleotides present in dsRNA molecules of this disclosure, preferably in the antisense strand, but also optionally in the sense or both the antisense and sense strands, are resistant to nuclease degradation while at the same time maintaining the capacity to mediate RNAi. Exemplary nucleotides having a Northern configuration include locked nucleic acid (LNA) nucleotides (*e.g.*, 2ʹ-O, 4'-C-methylene-(*D-*ribofuranosyl) nucleotides), 2'-methoxyethyl (MOE) nucleotides, 2'-methyl-thio-ethyl, 2ʹ-deoxy-2ʹ-fluoro nucleotides, 2'-deoxy-2'-chloro nucleotides, 2ʹ-azido nucleotides, 5-methyluridines, or 2ʹ-O-methyl nucleotides. In certain embodiments, the LNA is a 5-methyluridine LNA or 2-thio-5-methyluridine LNA. In any of these embodiments, one or more substituted or modified nucleotides can be a G clamp (*e.g*., a cytosine analog that forms an additional hydrogen bond to guanine, such as 9-(aminoethoxy)phenoxazine; *see, e.g.,* Lin and Mateucci, J. Am. Chem. Soc. 120:8531, 1998).

As described herein, the first and one or more second strands of a dsRNA molecule or analog thereof provided by this disclosure can anneal or hybridize together (*e.g*., due to complementarity between the strands) to form at least one double-stranded region having a length of about 4 to about 10 base pairs, about 5 to about 13 base pairs, or about 15 to about 40 base pairs. In some embodiments, the dsRNA has at least one double-stranded region ranging in length from about 15 to about 24 base pairs or about 19 to about 23 base pairs. In other embodiments, the dsRNA has at least one double-stranded region ranging in length from about 26 to about 40 base pairs or about 27 to about 30 base pairs or about 30 to about 35 base pairs. In other embodiments, the two or more strands of a dsRNA molecule of this disclosure may optionally be covalently linked together by nucleotide or non-nucleotide linker molecules.

In certain embodiments, the dsRNA molecule or analog thereof comprises an overhang of one to four nucleotides on one or both 3'-ends of the dsRNA, such as an overhang comprising a deoxyribonucleotide or two deoxyribonucleotides (e.g., thymidine, adenine). In certain embodiments, the 3'-end comprising one or more deoxyribonucleotide is in an mdRNA molecule and is either in the gap, not in the gap, or any combination thereof. In some embodiments, dsRNA molecules or analogs thereof have a blunt end at one or both ends of the dsRNA. In certain embodiments, the 5'-end of the first or second strand is phosphorylated. In any of the embodiments of dsRNA molecules described herein, the 3'-terminal nucleotide overhangs can comprise ribonucleotides or deoxyribonucleotides that are chemically-modified at a nucleic acid sugar, base, or backbone. In any of the embodiments of dsRNA molecules described herein, the 3'-terminal nucleotide overhangs can comprise one or more universal base ribonucleotides. In any of the embodiments of dsRNA molecules described herein, the 3'-terminal nucleotide overhangs can comprise one or more acyclic nucleotides. In any of the embodiments of dsRNA molecules described herein, the dsRNA can further comprise a terminal phosphate group, such as a 5ʹ-phosphate (*see* Martinez et al., Cell. 110:563-574, 2002; and Schwarz et al., Molec. Cell 10:537-568, 2002) or a 5ʹ,3ʹ-diphosphate.

As set forth herein, the terminal structure of dsRNAs of this disclosure that decrease expression of one or more VEGF family genes by, for example, RNAi may either have blunt ends or one or more overhangs. In certain embodiments, the overhang may be at the 3'-end or the 5'-end. The total length of dsRNAs having overhangs is expressed as the sum of the length of the paired double-stranded portion together with the overhanging nucleotides. For example, if a 19 base pair dsRNA has a two nucleotide overhang at both ends, the total length is expressed as 21-mer. Furthermore, since the overhanging sequence may have low specificity to one or more VEGF family gene, it is not necessarily complementary (antisense) or identical (sense) to a VEGF family gene sequence. In further embodiments, a dsRNA of this disclosure that decreases expression of one or more VEGF family gene by RNAi may further comprise a low molecular weight structure (for example, a natural RNA molecule such as a tRNA, rRNA or viral RNA, or an artificial RNA molecule) at, for example, one or more overhanging portion of the dsRNA.

In further embodiments, a dsRNA molecule that decreases expression of one or more VEGF family genes by RNAi according to the instant disclosure may optionally comprise a 2'-sugar substitution, such as a 2'-deoxy, 2'-O-2-methoxyethyl, 2'-O-methoxyethyl, 2'-O-methyl, halogen, 2'-fluoro, 2'-O-allyl, or the like, or any combination thereof. In still further embodiments, a dsRNA molecule that decreases expression of one or more VEGF family gene by RNAi according to the instant disclosure further comprises a terminal cap substituent on one or both ends of the first strand or one or more second strands, such as an alkyl, abasic, deoxy abasic, glyceryl, dinucleotide, acyclic nucleotide, inverted deoxynucleotide moiety, or any combination thereof. In certain embodiments, at least one or two 5'-terminal ribonucleotides of the sense strand within the double-stranded region have a 2'-sugar substitution. In certain other embodiments, at least one or two 5'-terminal ribonucleotides of the antisense strand within the double-stranded region have a 2'-sugar substitution. In certain embodiments, at least one or two 5'-terminal ribonucleotides of the sense strand and the antisense strand within the double-stranded region have a 2'-sugar substitution.

In other embodiments, a dsRNA molecule that decreases expression of one or more target gene by RNAi according to the instant disclosure comprises one or more substitutions in the sugar backbone, including any combination of ribosyl, 2'-deoxyribosyl, a tetrofuranosyl (*e*.*g*., L-α-threofuranosyl), a hexopyranosyl (*e*.*g*., β-allopyranosyl, β-altropyranosyl, and β-glucopyranosyl), a pentopyranosyl (*e.g.*, β-ribopyranosyl, α-lyxopyranosyl, β-xylopyranosyl, and α-arabinopyranosyl), a carbocyclic (carbon only ring) analog, a pyranose, a furanose, a morpholino, or analogs or derivatives thereof.

In yet other embodiments, a dsRNA molecule that decreases expression of one or more VEGF family gene by RNAi according to the instant disclosure further comprises at least one modified internucleoside linkage, such as independently a phosphorothioate, chiral phosphorothioate, phosphorodithioate, phosphotriester, aminoalkylphosphotriester, methyl phosphonate, alkyl phosphonate, 3'-alkylene phosphonate, 5'-alkylene phosphonate, chiral phosphonate, phosphonoacetate, thiophosphonoacetate, phosphinate, phosphoramidate, 3'-amino phosphoramidate, aminoalkylphosphoramidate, thionophosphoramidate, selenophosphate, thionoalkylphosphonate, thionoalkylphosphotriester, boranophosphate linkage, or any combination thereof.

A modified internucleotide linkage, as described herein, can be present in one or more strands of a dsRNA molecule of this disclosure, for example, in the sense strand, the antisense strand, both strands, or a plurality of strands (*e.g*., in an mdRNA). The dsRNA molecules of this disclosure can comprise one or more modified internucleotide linkages at the 3'-end, the 5ʹ-end, or both of the 3ʹ- and 5'-ends of the sense strand or the antisense strand or both strands. In one embodiment, a dsRNA molecule capable of decreasing expression of one or more VEGF family gene by RNAi has one modified internucleotide linkage at the 3ʹ-end, such as a phosphorothioate linkage. For example, this disclosure provides a dsRNA molecule capable of decreasing expression of one or more VEGF family gene by RNAi having about 1 to about 8 or more phosphorothioate internucleotide linkages in one dsRNA strand. In yet another embodiment, this disclosure provides a dsRNA molecule capable of decreasing expression of one or more VEGF family gene by RNAi having about 1 to about 8 or more phosphorothioate internucleotide linkages in both dsRNA strands. In other embodiments, an exemplary dsRNA molecule of this disclosure can comprise from about 1 to about 5 or more consecutive phosphorothioate internucleotide linkages at the 5'-end of the sense strand, the antisense strand, both strands, or a plurality of strands. In another example, an exemplary dsRNA molecule of this disclosure can comprise one or more pyrimidine phosphorothioate internucleotide linkages in the sense strand, the antisense strand, either strand, or a plurality of strands. In yet another example, an exemplary dsRNA molecule of this disclosure can comprise one or more purine phosphorothioate internucleotide linkages in the sense strand, the antisense strand, either strand, or a plurality of strands.

Many exemplary modified nucleotide bases or analogs thereof useful in the dsRNA of the instant disclosure include 5-methylcytosine; 5-hydroxymethylcytosine; xanthine; hypoxanthine; 2-aminoadenine; 6-methyl, 2-propyl, or other alkyl derivatives of adenine and guanine; 8-substituted adenines and guanines (such as 8-aza, 8-halo, 8-amino, 8-thiol, 8-thioalkyl, 8-hydroxyl, or the like); 7-methyl, 7-deaza, and 3-deaza adenines and guanines; 2-thiouracil; 2-thiothymine; 2-thiocytosine; 5-methyl, 5-propynyl, 5-halo (such as 5-bromo or 5-fluoro), 5-trifluoromethyl, or other 5-substituted uracils and cytosines; and 6-azouracil. Further useful nucleotide bases can be found in Kurreck, Eur. J. Biochem. 270:1628, 2003; Herdewijn, Antisense Nucleic Acid Develop. 10:297, 2000*;* Concise Encyclopedia of Polymer Science and Engineering, pages 858-859, Kroschwitz, J. I., ed. John Wiley & Sons, 1990; U.S. Patent No. 3,687,808, and similar references.

Certain nucleotide base moieties are particularly useful for increasing the binding affinity of the dsRNA molecules of this disclosure to complementary targets. These include 5-substituted pyrimidines; 6-azapyrimidines; and N-2, N-6, or O-6 substituted purines (including 2-aminopropyladenine, 5-propynyluracil and 5-propynylcytosine). For example, 5-methyluridine and 5-methylcytosine substitutions are known to increase nucleic acid duplex stability, which can be combined with 2'-sugar modifications (such as 2'-methoxy or 2'-methoxyethyl) or internucleoside linkages (e.g., phosphorothioate) that provide nuclease resistance to the modified or substituted dsRNA.

In another aspect of the instant disclosure, there is provided a dsRNA that decreases expression of one or more VEGF family genes, comprising a first strand that is complementary to VEGF mRNA set forth in SEQ ID NO:1158, 1159, 1160, 1161, 1162, 1163, or 1164 (*i*.*e*., VEGF variants 1 to 7) and is fully complementary, with up to three mismatches, to at least one other human VEGF family mRNA selected from SEQ ID NO:1165, 1166, 1167, or 1168 (*i.e*., VEGFB, VEGFC, FIGF, PGF, respectively), and a second strand that is complementary to the first strand, wherein the first and second strands form a double-stranded region of about 15 to about 40 base pairs; wherein at least one pyrimidine of the dsRNA is substituted with a pyrimidine nucleoside according to Formula I or II: 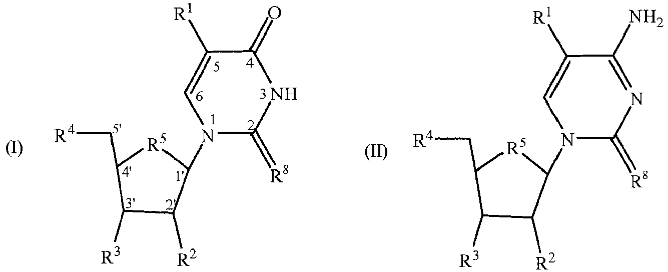 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C≡N, or heterocyclo group; R³ and R⁴ are each independently a hydroxyl, a protected hydroxyl, or an internucleoside linking group; and R⁵ and R⁸ are each independently O or S. In certain embodiments, at least one nucleoside is according to Formula I in which R¹ is methyl and R² is -OH, or R¹ is methyl, R² is -OH, and R⁸ is S. In other embodiments, the internucleoside linking group covalently links from about 2 to about 40 nucleosides.

In certain embodiments, the first and one or more second strands of a dsRNA, which decreases expression of one or more VEGF family gene by RNAi and has at least one pyrimidine substituted with a pyrimidine nucleoside according to Formula I or II, can anneal or hybridize together (*e.g*., due to complementarity between the strands) to form at least one double-stranded region having a length or a combined length of about 15 to about 40 base pairs. In some embodiments, the dsRNA has at least one double-stranded region ranging in length from about 4 base pairs to about 10 base pairs or about 5 to about 13 base pairs or about 15 to about 25 base pairs or about 19 to about 23 base pairs. In other embodiments, the dsRNA has at least one double-stranded region ranging in length from about 26 to about 40 base pairs or about 27 to about 30 base pairs or about 30 to about 35 base pairs. In certain embodiments, the dsRNA molecule or analog thereof has an overhang of one to four nucleotides on one or both 3'-ends, such as an overhang comprising a deoxyribonucleotide or two deoxyribonucleotides (*e.g*., thymidine). In some embodiments, dsRNA molecule or analog thereof has a blunt end at one or both ends of the dsRNA. In certain embodiments, the 5'-end of the first or second strand is phosphorylated.

In certain embodiments, at least one R¹ is a C₁-C₅ alkyl, such as methyl or ethyl. Within other exemplary embodiments of this disclosure, compounds of Formula I are a 5-alkyluridine (*e.g*., R¹ is alkyl, R² is -OH, and R³, R⁴, and R⁵ are as defined herein) or compounds of Formula II are a 5-alkylcytidine (*e.g*., R¹ is alkyl, R² is -OH, and R³, R⁴, and R⁵ are as defined herein). In related embodiments, the 5-alkyluridine is a 5-methyluridine (also referred to as ribothymidine or 't' or 'T^{r}' - *e.g*., R¹ is methyl and R² is -OH), and the 5-alkylcytidine is a 5-methylcytidine. In other embodiments, at least one, at least three, or all uridines of the first strand of the dsRNA are replaced with 5-methyluridine, or at least one, at least three, or all uridines of the second strand of the dsRNA are replaced with 5-methyluridine, or any combination thereof (*e.g*., such changes are made on both strands). In further embodiments, the 5-methyluridine may further have a 2'-O-methyl. In certain embodiments, at least one pyrimidine nucleoside of Formula I or Formula II has an R⁵ that is S.

In further embodiments, at least one pyrimidine nucleoside of the dsRNA is a locked nucleic acid (LNA) in the form of a bicyclic sugar, wherein R² is oxygen, and the 2'-O and 4'-C form an oxymethylene bridge on the same ribose ring. In a related embodiment, the LNA comprises a base substitution, such as a 5-methyluridine LNA or 2-thio-5-methyluridine LNA. In other embodiments, at least one, at least three, or all uridines of the first strand of the dsRNA are replaced with 5-methyluridine or 2-thioribothymidine or 5-methyluridine LNA or 2-thio-5-methyluridine LNA, or at least one, at least three, or all uridines of the second strand of the dsRNA are replaced with 5-methyluridine, 2-thioribothymidine, 5-methyluridine LNA, 2-thio-5-methyluridine LNA, or any combination thereof (*e.g*., such changes are made on both strands, or some substitutions include 5-methyluridine only, 2-thioribothymidine only, 5-methyluridine LNA only, 2-thio-5-methyluridine LNA only, or one or more 5-methyluridine or 2-thioribothymidine with one or more 5-methyluridine LNA or 2-thio-5-methyluridine LNA).

In further embodiments, a ribose of the pyrimidine nucleoside or the internucleoside linkage can be optionally modified. For example, compounds of Formula I or II are provided wherein R² is alkoxy, such as a 2'-O-methyl substitution (*e.g*., which may be in addition to a 5-alkyluridine or a 5-alkylcytidine, respectively). In certain embodiments, R² is selected from 2'-O-(C₁-C₅) alkyl, 2'-O-methyl, 2'-OCH₂OCH₂CH₃, 2'-OCH₂CH₂OCH₃, 2ʹ-O-allyl, or fluoro. In further embodiments, one or more of the pyrimidine nucleosides are according to Formula (I) in which R¹ is methyl and R² is a 2ʹ-O-(C₁-C₅) alkyl (*e.g*., 2ʹ-O-methyl). In other embodiments, one or more, or at least two, pyrimidine nucleosides according to Formula I or II have an R² that is not -H or -OH and is incorporated at a 3ʹ-end or 5'-end and not within the gap of one or more strands within the double-stranded region of the dsRNA molecule.

In further embodiments, a dsRNA molecule or analog thereof comprising a pyrimidine nucleoside according to Formula I or Formula II in which R² is not -H or -OH and an overhang, further comprises at least two of pyrimidine nucleosides that are incorporated either at a 3'-end or a 5'-end or both of one strand or two strands within the double-stranded region of the dsRNA molecule. In a related embodiment, at least one of the at least two pyrimidine nucleosides in which R² is not -H or -OH is located at a 3'-end or a 5'-end within the double-stranded region of at least one strand of the dsRNA molecule, and wherein at least one of the at least two pyrimidine nucleosides in which R² is not -H or -OH is located internally within a strand of the dsRNA molecule. In still further embodiments, a dsRNA molecule or analog thereof that has an overhang has a first of the two or more pyrimidine nucleosides in which R² is not -H or -OH that is incorporated at a 5'-end within the double-stranded region of the sense strand of the dsRNA molecule and a second of the two or more pyrimidine nucleosides is incorporated at a 5'-end within the double-stranded region of the antisense strand of the dsRNA molecule. In any of these embodiments, one or more substituted or modified nucleotides can be a G clamp (*e.g*., a cytosine analog that forms an additional hydrogen bond to guanine, such as 9-(aminoethoxy)phenoxazine; *see, e.g.,* Lin and Mateucci, 1998). In any of these embodiments, provided the one or more modified pyrimidine nucleosides are not within the gap.

In yet other embodiments, a dsRNA molecule or analog thereof of Formula I or II according to the instant disclosure that has an overhang comprises four or more independent pyrimidine nucleosides or four or more independent pyrimidine nucleosides in which R² is not -H or -OH, wherein (a) a first pyrimidine nucleoside is incorporated into a 3'-end within the double-stranded region of the sense (second) strand of the dsRNA, (b) a second pyrimidine nucleoside is incorporated into a 5'-end within the double-stranded region of the sense (second) strand, (c) a third pyrimidine nucleoside is incorporated into a 3'-end within the double-stranded region of the antisense (first) strand of the dsRNA, and (d) a fourth pyrimidine nucleoside is incorporated into a 5'-end within the double-stranded region of the antisense (first) strand. In any of these embodiments, provided the one or more pyrimidine nucleosides are not within the gap.

In further embodiments, a dsRNA molecule or analog thereof comprising a pyrimidine nucleoside according to Formula (I) or Formula (II) in which R² is not -H or -OH and is blunt-ended, further comprises at least two of pyrimidine nucleosides that are incorporated either at a 3'-end or a 5'-end or both of one strand or two strands of the dsRNA molecule. In a related embodiment, at least one of the at least two pyrimidine nucleosides in which R² is not -H or -OH is located at a 3'-end or a 5'-end of at least one strand of the dsRNA molecule, and wherein at least one of the at least two pyrimidine nucleosides in which R² is not -H or -OH is located internally within a strand of the dsRNA molecule. In still further embodiments, a dsRNA molecule or analog thereof that is blunt-ended has a first of the two or more pyrimidine nucleosides in which R² is not -H or -OH that is incorporated at a 5'-end of the sense strand of the dsRNA molecule and a second of the two or more pyrimidine nucleosides is incorporated at a 5'-end of the antisense strand of the dsRNA molecule. In any of these embodiments, provided the one or more pyrimidine nucleosides are not within the gap.

In yet other embodiments, a dsRNA molecule comprising a pyrimidine nucleoside according to Formula (I) or Formula (II) and that is blunt-ended comprises four or more independent pyrimidine nucleosides or four or more independent pyrimidine nucleosides in which R² is not -H or -OH, wherein (a) a first pyrimidine nucleoside is incorporated into a 3'-end within the double-stranded region of the sense (second) strand of the dsRNA, (b) a second pyrimidine nucleoside is incorporated into a 5'-end within the double-stranded region of the sense (second) strand, (c) a third pyrimidine nucleoside is incorporated into a 3'-end within the double-stranded region of the antisense (first) strand of the dsRNA, and (d) a fourth pyrimidine nucleoside is incorporated into a 5'-end within the double-stranded region of the antisense (first) strand. In any of these embodiments, provided the one or more pyrimidine nucleosides are not within the gap.

In still further embodiments, a dsRNA molecule or analog thereof of Formula I or II according to the instant disclosure further comprises a terminal cap substituent on one or both ends of the first strand or second strand, such as an alkyl, abasic, deoxy abasic, glyceryl, dinucleotide, acyclic nucleotide, inverted deoxynucleotide moiety, or any combination thereof. In further embodiments, one or more internucleoside linkage can be optionally modified. For example, a dsRNA molecule or analog thereof of Formula I or II according to the instant disclosure wherein at least one internucleoside linkage is modified to a phosphorothioate, chiral phosphorothioate, phosphorodithioate, phosphotriester, aminoalkylphosphotriester, methyl phosphonate, alkyl phosphonate, 3'-alkylene phosphonate, 5'-alkylene phosphonate, chiral phosphonate, phosphonoacetate, thiophosphonoacetate, phosphinate, phosphoramidate, 3'-amino phosphoramidate, aminoalkylphosphoramidate, thionophosphoramidate, thionoalkylphosphonate, thionoalkylphosphotriester, selenophosphate, boranophosphate linkage, or any combination thereof.

In still another embodiment, a nicked or gapped dsRNA molecule (ndsRNA or gdsRNA, respectively) that decreases expression of one or more VEGF family gene by RNAi, comprising a first strand that is complementary to a human VEGF mRNA set forth in SEQ ID NO:1158, 1159, 1160, 1161, 1162, 1163, or 1164 and is fully complementary, with up to three mismatches, to at least one other human VEGF family mRNA selected from SEQ ID NO:1165, 1166, 1167, or 1168, and two or more second strands that are complementary to the first strand, wherein the first and at least one of the second strands optionally form a non-overlapping double-stranded region of about 5 to about 13 base pairs. Any of the aforementioned substitutions or modifications are contemplated within this embodiment as well.

In another exemplary of this disclosure, the dsRNAs comprise at least two or more substituted pyrimidine nucleosides can each be independently selected wherein R¹ comprises any chemical modification or substitution as contemplated herein, for example an alkyl (e.g., methyl), halogen, hydroxy, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, carbonyl, alkanoylamino, carbamoyl, carbonylamino, alkylsulfonylamino, or heterocyclo group. When two or more modified ribonucleotides are present, each modified ribonucleotide can be independently modified to have the same, or different, modification or substitution at R¹ or R².

In other detailed embodiments, one or more substituted pyrimidine nucleosides according to Formula I or II can be located at any ribonucleotide position, or any combination of ribonucleotide positions, on either or both of the sense and antisense strands of a dsRNA molecule of this disclosure, including at one or more multiple terminal positions as noted above, or at any one or combination of multiple non-terminal ("internal") positions. In this regard, each of the sense and antisense strands can incorporate about 1 to about 6 or more of the substituted pyrimidine nucleosides.

In certain embodiments, when two or more substituted pyrimidine nucleosides are incorporated within a dsRNA of this disclosure, at least one of the substituted pyrimidine nucleosides will be at a 3'- or 5'-end of one or both strands, and in certain embodiments at least one of the substituted pyrimidine nucleosides will be at a 5'-end of one or both strands. In other embodiments, the substituted pyrimidine nucleosides are located at a position corresponding to a position of a pyrimidine in an unmodified dsRNA that is constructed as a homologous sequence for targeting a cognate mRNA, as described herein.

In addition, the terminal structure of the dsRNAs of this disclosure may have a stem-loop structure in which ends of one side of the dsRNA molecule are connected by a linker nucleic acid, *e.g.*, a linker RNA. The length of the double-stranded region (stem-loop portion) can be, for example, about 15 to about 49 bp, about 15 to about 35 bp, or about 21 to about 30 bp long. Alternatively, the length of the double-stranded region that is a final transcription product of dsRNAs to be expressed in a target cell may be, for example, approximately about 15 to about 49 bp, about 15 to about 35 bp, or about 21 to about 30 bp long. When linker segments are employed, there is no particular limitation in the length of the linker as long as it does not hinder pairing of the stem portion. For example, for stable pairing of the stem portion and suppression of recombination between DNAs coding for this portion, the linker portion may have a clover-leaf tRNA structure. Even if the linker has a length that would hinder pairing of the stem portion, it is possible, for example, to construct the linker portion to include introns so that the introns are excised during processing of a precursor RNA into mature RNA, thereby allowing pairing of the stem portion. In the case of a stem-loop dsRNA, either end (head or tail) of RNA with no loop structure may have a low molecular weight RNA. As described above, these low molecular weight RNAs may include a natural RNA molecule, such as tRNA, rRNA or viral RNA, or an artificial RNA molecule.

A dsRNA molecule may be comprised of a circular nucleic acid molecule, wherein the dsRNA is about 38 to about 70 nucleotides in length having from about 18 to about 23 (e.g., about 19 to about 21) base pairs wherein the circular oligonucleotide forms a dumbbell shaped structure having about 19 base pairs and 2 loops. In certain embodiments, a circular dsRNA molecule contains two loop motifs, wherein one or both loop portions of the dsRNA molecule is biodegradable. For example, a circular dsRNA molecule of this disclosure is designed such that degradation of the loop portions of the dsRNA molecule *in vivo* can generate a double-stranded dsRNA molecule with 3'-terminal overhangs, such as 3'-terminal nucleotide overhangs comprising from about 1 to about 4 (unpaired) nucleotides.

Substituting or modifying nucleosides of a dsRNA according to this disclosure can result in increased resistance to enzymatic degradation, such as exonucleolytic degradation, including 5'-exonucleolytic or 3'-exonucleolytic degradation. As such, in some embodiments, the dsRNAs described herein will exhibit significant resistance to enzymatic degradation compared to a corresponding dsRNA having standard nucleotides, and will thereby possess greater stability, increased half-life, and greater bioavailability in physiological environments (*e.g*., when introduced into a eukaryotic target cell). In addition to increasing resistance of the substituted or modified dsRNAs to exonucleolytic degradation, the incorporation of one or more pyrimidine nucleosides according to Formula I or II will render dsRNAs more resistant to other enzymatic or chemical degradation processes and thus more stable and bioavailable than otherwise identical dsRNAs that do not include the substitutions or modifications. In related aspects of this disclosure, dsRNA substitutions or modifications described herein will often improve stability of a modified dsRNA for use within research, diagnostic and treatment methods wherein the modified dsRNA is contacted with a biological sample, for example, a mammalian cell, intracellular compartment, serum or other extracellular fluid, tissue, or other *in vitro* or *in vivo* physiological compartment or environment. In one embodiment, diagnosis is performed on an isolated biological sample. In another embodiment, the diagnostic method is performed *in vitro.* In a further embodiment, the diagnostic method is not performed (directly) on a human or animal body.

In addition to increasing stability of substituted or modified dsRNAs, incorporation of one or more pyrimidine nucleosides according to Formula I or II in a dsRNA designed for gene silencing can provide additional desired functional results, including increasing a melting point of a substituted or modified dsRNA compared to a corresponding unmodified dsRNA. In another aspect of this disclosure, certain substitutions or modifications of dsRNAs described herein can reduce "off-target effects" of the substituted or modified dsRNA molecules when they are contacted with a biological sample (*e.g*., when introduced into a target eukaryotic cell having specific, and non-specific mRNA species present as potential specific and non-specific targets).

In further embodiments, dsRNAs of this disclosure can comprise one or more sense (second) strand that is homologous or corresponds to a sequence of a target gene (*e.g*., VEGF, VEGFB, VEGFC, FIGF, PGF) and an antisense (first) strand that is complementary to the sense strand and a sequence of the target gene. In exemplary embodiments, at least one strand of the dsRNA incorporates one or more pyrimidines substituted according to Formula I or II *(e.g.,* wherein the pyrimidine is replaced by more than one 5-methyluridine or the ribose is modified to incorporate a 2'-O-methyl substitution or any combination thereof). These and other multiple substitutions or modifications according to Formula I or II can be introduced into one or more pyrimidines, or into any combination and up to all pyrimidines present in one or all strands of a dsRNA of the instant disclosure, so long as the dsRNA has or retains RNAi activity similar to or better than the activity of an unmodified dsRNA.

In any of the embodiments described herein, the dsRNA may include multiple modifications. For example, a dsRNA having at least one ribothymidine or 2'-O-methyl-5-methyluridine may further comprise at least one LNA, 2'-methoxy, 2'-fluoro, 2'-deoxy, phosphorothioate linkage, an inverted base terminal cap, or any combination thereof. In certain embodiments, a dsRNA will have from one to all ribothymidines and have up to 75% LNA. In other embodiments, a dsRNA will have from one to all ribothymidines and have up to 75% 2'-methoxy (*e.g*., not at the Argonaute cleavage site). In still other embodiments, a dsRNA will have from one to all ribothymidines and have up to 100% 2'-fluoro. In further embodiments, a dsRNA will have from one to all ribothymidines and have up to 75% 2'-deoxy. In further embodiments, a dsRNA will have up to 75% LNA and have up to 75% 2'-methoxy. In still other embodiments, a dsRNA will have up to 75% LNA and have up to 100% 2'-fluoro. In further embodiments, a dsRNA will have up to 75% LNA and have up to 75% 2'-deoxy. In other embodiments, a dsRNA will have up to 75% 2'-methoxy and have up to 100% 2'-fluoro. In more embodiments, a dsRNA will have up to 75% 2'-methoxy and have up to 75% 2'-deoxy. In further embodiments, a dsRNA will have up to 100% 2'-fluoro and have up to 75% 2'-deoxy.

In further multiple modification embodiments, a dsRNA will have from one to all ribothymidines, up to 75% LNA, and up to 75% 2'-methoxy. In still further embodiments, a dsRNA will have from one to all ribothymidines, up to 75% LNA, and up to 100% 2'-fluoro. In further embodiments, a dsRNA will have from one to all ribothymidines, up to 75% LNA, and up to about 75% 2'-deoxy. In further embodiments, a dsRNA will have from one to all ribothymidines, up to 75% 2'-methoxy, and up to 75% 2'-fluoro. In further embodiments, a dsRNA will have from one to all ribothymidines, up to 75% 2'-methoxy, and up to 75% 2'-deoxy. In further embodiments, a dsRNA will have from one to all ribothymidines, up to 100% 2'-fluoro, and up to 75% 2'-deoxy. In yet further embodiments, a dsRNA will have from one to all ribothymidines, up to 75% LNA substitutions, up to 75% 2'-methoxy, up to 100% 2'-fluoro, and up to 75% 2'-deoxy. In other embodiments, a dsRNA will have up to 75% LNA, up to 75% 2'-methoxy, and up to 100% 2'-fluoro. In further embodiments, a dsRNA will have up to 75% LNA, up to 75% 2'-methoxy, and up to about 75% 2'-deoxy. In further embodiments, a dsRNA will have up to 75% LNA, up to 100% 2'-fluoro, and up to 75% 2'-deoxy. In still further embodiments, a dsRNA will have up to 75% 2'-methoxy, up to 100% 2'-fluoro, and up to 75% 2'-deoxy.

In any of these exemplary methods for using multiply modified dsRNA, the dsRNA may further comprise up to 100% phosphorothioate internucleoside linkages, from one to ten or more inverted base terminal caps, or any combination thereof. Additionally, any of these dsRNA may have these multiple modifications on one strand, two strands, three strands, a plurality of strands, or all strands, or on the same or different nucleoside within a dsRNA molecule. Finally, in any of these multiple modification dsRNA, the dsRNA must have gene silencing activity.

Within certain aspects, the present disclosure provides dsRNA that decreases expression of one or more VEGF family gene by RNAi (*e.g*., a VEGF of SEQ ID NO:1158-1168), and compositions comprising one or more dsRNA, wherein at least one dsRNA comprises one or more universal-binding nucleotide(s) in the first, second or third position in the anti-codon of the antisense strand of the dsRNA duplex and wherein the dsRNA is capable of specifically binding to one or more VEGF family sequence, such as an RNA expressed by a target cell. In cases wherein the sequence of the target VEGF RNA includes one or more single nucleotide substitutions, dsRNA comprising a universal-binding nucleotide retains its capacity to specifically bind a target VEGF RNA, thereby mediating gene silencing and, as a consequence, overcoming escape of the target VEGF from dsRNA-mediated gene silencing. Non-limiting examples of universal-binding nucleotides that may be suitably employed in the compositions and methods disclosed herein include inosine, 1-β-*D*-ribofuranosyl-5-nitroindole, and 1-β-*D-*ribofuranosyl-3-nitropyrrole.

In certain aspects, dsRNA disclosed herein can include between about 1 universal-binding nucleotide and about 10 universal-binding nucleotides. Within other aspects, the presently disclosed dsRNA may comprise a sense strand that is homologous to a sequence of one or more VEGF family gene and an antisense strand that is complementary to the sense strand, with the proviso that at least one nucleotide of the antisense strand of the otherwise complementary dsRNA duplex is replaced by one or more universal-binding nucleotide.

### Synthesis of Nucleic Acid Molecules

Exemplary molecules of the instant disclosure are recombinantly produced, chemically synthesized, or a combination thereof. Oligonucleotides (*e.g*., certain modified oligonucleotides or portions of oligonucleotides lacking ribonucleotides) are synthesized using protocols known in the art, for example as described in Caruthers et al., Methods in Enzymol. 211:3, 1992; PCT Publication No. WO 99/54459, Wincott et al., Nucleic Acids Res. 23:2677, 1995; Wincott et al., Methods Mol. Bio. 74:59, 1997; Brennan et al., Biotechnol Bioeng. 61:33, 1998; and U.S. Patent No. 6,001,311. Synthesis of RNA, including certain dsRNA molecules and analogs thereof of this disclosure can be made using procedures described in, *e.g.,* Usman et al., J. Am. Chem. Soc. 109:7845, 1987; Scaringe et al., Nucleic Acids Res. 18:5433, 1990; and Wincott *et al.,* 1995; Wincott *et al.,* 1997. In certain embodiments, the nucleic acid molecules of this disclosure can be synthesized separately and joined together post-synthetically, *e.g*., by ligation (Moore et al., Science 256:9923, 1992; PCT Publication No. WO 93/23569; Shabarova et al., Nucleic Acids Res. 19:4247, 1991; Bellon et al., Nucleosides & Nucleotides 16:951, 1997; Bellon et al., Bioconjugate Chem. 8:204, 1997), or by hybridization following synthesis or deprotection.

In further embodiments, dsRNAs of this disclosure that decrease expression of one or more VEGF family gene by RNAi can be made as single or multiple transcription products expressed by a polynucleotide vector encoding the single or multiple dsRNAs and directing their expression within host cells. In these embodiments the double-stranded portion of a final transcription product of the dsRNAs to be expressed within the target cell can be, for example, 5 to 40 bp, 15 to 24 bp, or about 25 to 40 bp long. Within exemplary embodiments, double-stranded portions of dsRNAs, in which two or more strands pair up, are not limited to completely paired nucleotide segments, and may contain non-pairing portions due to a mismatch (the corresponding nucleotides are not complementary), bulge (lacking in the corresponding complementary nucleotide on one strand), overhang, and the like. Non-pairing portions can be contained to the extent that they do not interfere with dsRNA formation. In more detailed embodiments, a "bulge" may comprise 1 to 2 non-pairing nucleotides, and the double-stranded region of dsRNAs in which two strands pair up may contain from about 1 to 7, or about 1 to 5 bulges. In addition, "mismatch" portions contained in the double-stranded region of siNAs may be present in numbers from about 1 to 7, or about 1 to 5. In other embodiments, the double-stranded region of dsRNAs of this disclosure may contain both bulge and mismatched portions in the approximate numerical ranges specified herein.

A dsRNA or analog thereof of this disclosure may be further comprised of a nucleotide, non-nucleotide, or mixed nucleotide/non-nucleotide linker that joins the sense region of the dsRNA to the antisense region of the dsRNA. In one embodiment, a nucleotide linker can be a linker of more than about 2 nucleotides length up to about 10 nucleotides in length. In another embodiment, the nucleotide linker can be a nucleic acid aptamer. By "aptamer" or "nucleic acid aptamer" as used herein is meant a nucleic acid molecule that binds specifically to a target molecule wherein the nucleic acid molecule has sequence that comprises a sequence recognized by the target molecule in its natural setting. Alternately, an aptamer can be a nucleic acid molecule that binds to a target molecule wherein the target molecule does not naturally bind to a nucleic acid. The target molecule can be any molecule of interest. For example, the aptamer can be used to bind to a ligand-binding domain of a protein, thereby preventing interaction of the naturally occurring ligand with the protein. This is a non-limiting example and those in the art will recognize that other embodiments can be readily generated using techniques generally known in the art *(see, e.g.,* Gold et al., Annu. Rev. Biochem. 64:763, 1995; Brody and Gold, J. Biotechnol. 74:5, 2000; Sun, Curr. Opin. Mol. Ther. 2:100, 2000; Kusser, J. Biotechnol. 74:27, 2000; Hermann and Patel, Science 287:820, 2000; and Jayasena, Clinical Chem. 45:1628, 1999).

A non-nucleotide linker may be comprised of an abasic nucleotide, polyether, polyamine, polyamide, peptide, carbohydrate, lipid, polyhydrocarbon, or other polymeric compounds (*e.g*., polyethylene glycols such as those having between 2 and 100 ethylene glycol units). Specific examples include those described by Seela and Kaiser, Nucleic Acids Res. 18:6353, 1990, and Nucleic Acids Res. 15:3113, 1987; Cload and Schepartz, J. Am. Chem. Soc. 113:6324, 1991; Richardson and Schepartz, J. Am. Chem. Soc. 113:5109, 1991; Ma et al., Nucleic Acids Res. 21:2585, 1993, and Biochemistry 32:1751, 1993; Durand et al., Nucleic Acids Res. 18:6353, 1990; McCurdy et al., Nucleosides & Nucleotides 10:287, 1991; Jaschke et al., Tetrahedron Lett. 34:301, 1993; Ono et al., Biochemistry 30:9914, 1991; Arnold et al., PCT Publication No. WO 89/02439; Usman et al., PCT Publication No. WO 95/06731; Dudycz et al., PCT Publication No. WO 95/11910 and Ferentz and Verdine, J. Am. Chem. Soc. 113:4000, 1991. The synthesis of a dsRNA molecule of this disclosure, which can be further modified, comprises: (a) synthesis of two complementary strands of the dsRNA molecule; and (b) annealing the two complementary strands together under conditions suitable to obtain a dsRNA molecule. In another embodiment, synthesis of the two complementary strands of a dsRNA molecule is by solid phase oligonucleotide synthesis. In yet another embodiment, synthesis of the two complementary strands of a dsRNA molecule is by solid phase tandem oligonucleotide synthesis.

Chemically synthesizing nucleic acid molecules with substitutions or modifications (base, sugar, phosphate, or any combination thereof) can prevent their degradation by serum ribonucleases, which may lead to increased potency. *See, e.g.,* Eckstein et al., PCT Publication No. WO 92/07065; Perrault et al., Nature 344:565, 1990; Pieken et al., Science 253:314, 1991; Usman and Cedergren, Trends in Biochem. Sci. 17:334, 1992; Usman et al., Nucleic Acids Symp. Ser. 31:163, 1994; Beigelman et al., J. Biol. Chem. 270:25702, 1995; Burgin et al., Biochemistry 35:14090, 1996; Burlina et al., Bioorg. Med. Chem. 5:1999, 1997; Thompson et al., Karpeisky et al., Tetrahedron Lett. 39:1131, 1998; Earnshaw and Gait, Biopolymers (Nucleic Acid Sciences) 48:39-55, 1998; Verma and Eckstein, Annu. Rev. Biochem. 67:99-134, 1998; Herdewijn, Antisense Nucleic Acid Drug Dev. 10:297, 2000*;* Kurreck, Eur. J. Biochem. 270:1628, 2003; Dorsett and Tuschl, Nature Rev. Drug Discov. 3:318, 2004; Rossi et al., PCT Publication No. WO 91/03162; Usman et al., PCT Publication No. WO 93/15187; Beigelman et al., PCT Publication No. WO 97/26270; Woolf et al., PCT Publication No. WO 98/13526; Sproat, U.S. Patent No. 5,334,711; Usman et al., U.S. Patent No. 5,627,053; Beigelman et al., U.S. Patent No. 5,716,824; Ötvös et al., U.S. Patent No. 5,767, 264; Gold et al., U.S. Patent No. 6,300,074. Each of the above references discloses various substitutions and chemical modifications to the base, phosphate, or sugar moieties of nucleic acid molecules, which can be used in the dsRNAs described herein. For example, oligonucleotides can be modified at the sugar moiety to enhance stability or prolong biological activity by increasing nuclease resistance. Representative sugar modifications include 2'-amino, 2ʹ-C-allyl, 2'-fluoro, 2'-O-methyl, 2'-O-allyl, or 2'-H. Other modifications to enhance stability or prolong biological activity can be internucleoside linkages, such as phosphorothioate, or base-modifications, such as locked nucleic acids (*see, e.g.,* U.S. Patent Nos. 6,670,461; 6,794,499; 6,268,490), or 5-methyluridine or 2'-O-methyl-5-methyluridine in place ofuridine *(see, e.g.,* U.S. Patent Application Publication No. 2006/0142230). Hence, dsRNA molecules of the instant disclosure can be modified to increase nuclease resistance or duplex stability while substantially retaining or having enhanced RNAi activity as compared to unmodified dsRNA.

In one embodiment, this disclosure features substituted or modified dsRNA molecules, such as phosphate backbone modifications comprising one or more phosphorothioate, phosphorodithioate, methylphosphonate, phosphotriester, morpholino, amidate carbamate, carboxymethyl, acetamidate, polyamide, sulfonate, sulfonamide, sulfamate, formacetal, thioformacetal, or alkylsilyl, substitutions. For a review of oligonucleotide backbone modifications, *see* Hunziker and Leumann, Nucleic Acid Analogues: Synthesis and Properties, in Modern Synthetic Methods, VCH, 331-417, 1995; and Mesmaeker et al., ACS, 24-39, 1994.

In another embodiment, a conjugate molecule can be optionally attached to a dsRNA or analog thereof that decreases expression of one or more VEGF family genes by RNAi. For example, such conjugate molecules may be polyethylene glycol, human serum albumin, polyarginine, Gln-Asn polymer, or a ligand for a cellular receptor that can, for example, mediate cellular uptake (*e.g.,* HIV TAT, *see* Vocero-Akbani et al., Nature Med. 5:23, 1999; *see also* U.S. Patent Application Publication No. 2004/0132161).. Examples of specific conjugate molecules contemplated by the instant disclosure that can be attached to a dsRNA or analog thereof of this disclosure are described in Vargeese et al., U.S. Patent Application Publication No. 2003/0130186, and U.S. Patent Application Publication No. 2004/0110296. In another embodiment, a conjugate molecule is covalently attached to a dsRNA or analog thereof that decreases expression of an one or more VEGF family genes by RNAi via a biodegradable linker. In certain embodiments, a conjugate molecule can be attached at the 3'-end of either the sense strand, the antisense strand, or both strands of a dsRNA molecule provided herein. In another embodiment, a conjugate molecule can be attached at the 5'-end of either the sense strand, the antisense strand, or both strands of the dsRNA or analog thereof. In yet another embodiment, a conjugate molecule is attached at both the 3'-end and 5'-end of either the sense strand, the antisense strand, or both strands of a dsRNA molecule, or any combination thereof. In further embodiments, a conjugate molecule of this disclosure comprises a molecule that facilitates delivery of a dsRNA or analog thereof into a biological system, such as a cell. A person of skill in the art can screen dsRNA of this disclosure having various conjugates to determine whether the dsRNA-conjugate possesses improved properties (*e.g*., pharmacokinetic profiles, bioavailability, stability) while maintaining the ability to mediate RNAi in, for example, an animal model as described herein or generally known in the art.

### Methods for Selecting dsRNA Molecules Specific for VEGF

As indicated above, the present disclosure also provides methods for selecting dsRNA and analogs thereof that are capable of specifically binding to one or more VEGF family gene (including a mRNA splice variant thereof) while being incapable of specifically binding or minimally binding to non-VEGF genes. The selection process disclosed herein is useful, for example, in eliminating dsRNAs analogs that are cytotoxic due to non-specific binding to, and subsequent degradation of, one or more non-VEGF genes.

Methods of the present disclosure do not require *a priori* knowledge of the nucleotide sequence of every possible gene variant (including mRNA splice variants) targeted by the dsRNA or analog thereof. In one embodiment, the nucleotide sequence of the dsRNA is selected from a conserved region or consensus sequence of one or more VEGF family genes. In another embodiment, the dsRNA may be selectively or preferentially targeted to a certain sequence contained in an mRNA splice variant of one or more VEGF family genes.

In certain embodiments, methods are provided for selecting one or more dsRNA molecule that decreases expression of one or more VEGF family gene by RNAi, comprising a first strand that is complementary to a human VEGF mRNA set forth in SEQ ID NO:1158, 1159, 1160, 1161, 1162, 1163, or 1164 and is fully complementary, with up to three mismatches, to at least one other human VEGF family mRNA selected from SEQ ID NO: 1165, 1166, 1167, or 1168, and a second strand that is complementary to the first strand, wherein the first and second strands form a double-stranded region of about 15 to about 40 base pairs (*e.g.,* VEGF sequences found in Table A from U.S. Application No. 60/932,949), and wherein at least one uridine of the dsRNA molecule is a 5-methyluridine or 2-thioribothymidine or 2'-O-methyl-5-methyluridine, which methods employ "off-target" profiling whereby one or more dsRNA provided herein is contacted with a cell, either *in vivo* or *in vitro,* and total VEGF mRNA is collected for use in probing a microarray comprising oligonucleotides having one or more nucleotide sequence from a panel of known genes, including non-VEGF genes (*e.g*., interferon). The "off-target" profile of the dsRNA provided herein is quantified by determining the number of non-VEGF genes having reduced expression levels in the presence of the candidate dsRNAs. The existence of "off target" binding indicates a dsRNA provided herein that is capable of specifically binding to one or more non-VEGF gene messages. In certain embodiments, a dsRNA as provided herein (*e.g*., sequences of Table A from U.S. Application No. 60/932,949) applicable to therapeutic use will exhibit a greater stability, minimal interferon response, and little or no "off-target" binding.

Still further embodiments provide methods for selecting more efficacious dsRNA by using one or more reporter gene constructs comprising a constitutive promoter, such as a cytomegalovirus (CMV) or phosphoglycerate kinase (PGK) promoter, operably fused to, and capable of altering the expression of one or more reporter genes, such as a luciferase, chloramphenicol (CAT), or β-galactosidase, which, in turn, is operably fused in-frame with a dsRNA (such as one having a length between about 15 base-pairs and about 40 base-pairs or from about 5 nucleotides to about 24 nucleotides, or about 25 nucleotides to about 40 nucleotides) that contains one or more VEGF family sequence, as provided herein.

Individual reporter gene expression constructs may be co-transfected with one or more dsRNA or analog thereof. The capacity of a given dsRNA to reduce the expression level of VEGF may be determined by comparing the measured reporter gene activity in cells transfected with or without a dsRNA molecule of interest.

Certain embodiments disclosed herein provide methods for selecting one or more modified dsRNA molecule(s) that employ the step of predicting the stability of a dsRNA duplex. In some embodiments, such a prediction is achieved by employing a theoretical melting curve wherein a higher theoretical melting curve indicates an increase in dsRNA duplex stability and a concomitant decrease in cytotoxic effects. Alternatively, stability of a dsRNA duplex may be determined empirically by measuring the hybridization of a single RNA analog strand as described herein to a complementary target gene within, for example, a polynucleotide array. The melting temperature (*i.e.,* the T*ₘ* value) for each modified RNA and complementary RNA immobilized on the array can be determined and, from this T*ₘ* value, the relative stability of the modified RNA pairing with a complementary RNA molecule determined.

For example, Kawase et al. (Nucleic Acids Res. 14:7727, 1986) have described an analysis of the nucleotide-pairing properties of Di (inosine) to A, C, G , and T, which was achieved by measuring the hybridization of oligonucleotides (ODNs) with Di in various positions to complementary sets of ODNs made as an array. The relative strength of nucleotide-pairing is I-C > I-A > I-G ≈ I-T. Generally, Di containing duplexes showed lower T*ₘ* values when compared to the corresponding wild type (WT) nucleotide pair. The stabilization of Di by pairing was in order of Dc > Da > Dg > Dt > Du. As a person of skill in the art would understand, although universal-binding nucleotides are used herein as an example of determining duplex stability (*i.e*., the T*ₘ* value), other nucleotide substitutions (e.g., 5-methyluridine for uridine) or further modifications (*e.g*., a ribose modification at the 2'-position) can also be evaluated by these or similar methods.

In still further embodiments of the presently disclosed methods, one or more anti-codon within an antisense strand of a dsRNA molecule or analog thereof is substituted with a universal-binding nucleotide in a second or third position in the anti-codon of the antisense strand. By substituting a universal-binding nucleotide for a first or second position, the one or more first or second position nucleotide-pair substitution allows the substituted dsRNA molecule to specifically bind to mRNA wherein a first or a second position nucleotide-pair substitution has occurred, wherein the one or more nucleotide-pair substitution results in an amino acid change in the corresponding gene product.

Any of these methods of identifying dsRNA of interest can also be used to examine a dsRNA that decreases expression of one or more VEGF family gene by RNA interference, comprising a first strand that is complementary to a human VEGF mRNA set forth in SEQ ID NO:1158, 1159, 1160, 1161, 1162, 1163, or 1164 and is fully complementary, with up to three mismatches, to at least one other human VEGF family mRNA selected from SEQ ID NO:1165, 1166, 1167, or 1168, and a second and third strand that have non-overlapping complementarity to the first strand, wherein the first and at least one of the second or third strand optionally form a double-stranded region of about 5 to about 13 base pairs; wherein at least one pyrimidine of the dsRNA comprises a pyrimidine nucleoside according to Formula I or II: 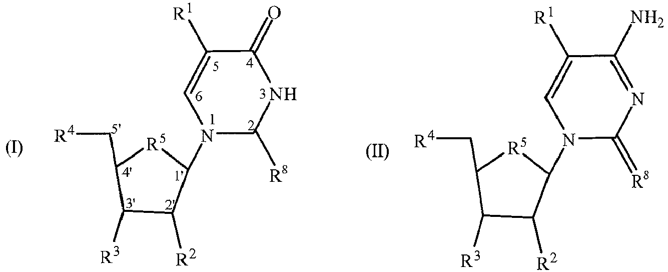 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C≡N, or heterocyclo group; R³ and R⁴ are each independently a hydroxyl, a protected hydroxyl, or an internucleoside linking group; and R⁵ and R⁸ are independently O or S. In certain embodiments, at least one nucleoside is according to Formula I in which R¹ is methyl and R² is -OH, or R¹ is methyl, R² is -OH, and R⁸ is S. In certain embodiments, at least one nucleoside is according to Formula I in which R¹ is methyl and R² is -O-methyl, or R¹ is methyl, R² is -O-methyl, and R⁸ is O. In other embodiments, the internucleoside linking group covalently links from about 5 to about 40 nucleosides.

### Compositions and Methods of Use

As set forth herein, dsRNA of the instant disclosure are designed to target one or more VEGF family gene that is expressed at an elevated level or continues to be expressed when it should not, and is a causal or contributing factor associated with, for example, a hyperproliferative, angiogenic, or inflammatory disease, state, or adverse condition. In this context, a dsRNA or analog thereof of this disclosure will effectively downregulate expression of one or more VEGF family gene to levels that prevent, alleviate, or reduce the severity or recurrence of one or more associated disease symptoms. Alternatively, for various distinct disease models in which expression of one or more VEGF family gene is not necessarily elevated as a consequence or sequel of disease or other adverse condition, down regulation of one or more VEGF family gene will nonetheless result in a therapeutic result by lowering gene expression (*e.g*., to reduce levels of a selected mRNA or protein product of one or more VEGF family gene). Furthermore, dsRNAs of this disclosure may be targeted to reduce expression of one or more VEGF family gene, which can result in upregulation of a "downstream" gene whose expression is negatively regulated, directly or indirectly, by one or more VEGF family protein. The dsRNA molecules of the instant disclosure comprise useful reagents and can be used in methods for a variety of therapeutic, diagnostic, target validation, genomic discovery, genetic engineering, and pharmacogenomic applications.

In certain embodiments, aqueous suspensions contain dsRNA of this disclosure in admixture with suitable excipients, such as suspending agents or dispersing or wetting agents. Exemplary suspending agents include sodium carboxymethylcellulose, methylcellulose, hydropropyl-methylcellulose, sodium alginate, polyvinylpyrrolidone, gum tragacanth and gum acacia. Representative dispersing or wetting agents include naturally-occurring phosphatides (*e.g*., lecithin), condensation products of an alkylene oxide with fatty acids (*e.g.,* polyoxyethylene stearate), condensation products of ethylene oxide with long chain aliphatic alcohols (*e.g*., heptadecaethyleneoxycetanol), condensation products of ethylene oxide with partial esters derived from fatty acids and hexitol (*e.g*., polyoxyethylene sorbitol monooleate), or condensation products of ethylene oxide with partial esters derived from fatty acids and hexitol anhydrides (*e.g*., polyethylene sorbitan monooleate). In certain embodiments, the aqueous suspensions can optionally contain one or more preservatives (*e.g*., ethyl or *n*-propyl-*p-*hydroxybenzoate), one or more coloring agents, one or more flavoring agents, or one or more sweetening agents (*e.g*., sucrose, saccharin). In additional embodiments, dispersible powders and granules suitable for preparation of an aqueous suspension by the addition of water provide dsRNA of this disclosure in admixture with a dispersing or wetting agent, suspending agent and optionally one or more preservative, coloring agent, flavoring agent, or sweetening agent.

The present disclosure includes dsRNA compositions prepared for storage or administration that include a pharmaceutically effective amount of a desired compound in a pharmaceutically acceptable carrier or diluent. Acceptable carriers or diluents for therapeutic use are well known in the pharmaceutical art, and are described, for example, in Remington's Pharmaceutical Sciences, Mack Publishing Co., A.R. Gennaro edit., 1985, hereby incorporated by reference herein. In certain embodiments, pharmaceutical compositions of this disclosure can optionally include preservatives, antioxidants, stabilizers, dyes, flavoring agents, or any combination thereof. Exemplary preservatives include sodium benzoate, sorbic acid and esters of p-hydroxybenzoic acid.

The dsRNA compositions of the instant disclosure can be effectively employed as pharmaceutically-acceptable formulations. Pharmaceutically-acceptable formulations prevent, alter the occurrence or severity of, or treat (alleviate one or more symptom(s) to a detectable or measurable extent) of a disease state or other adverse condition in a subject. A pharmaceutically acceptable formulation includes salts of the above compounds, *e.g*., acid addition salts, such as salts of hydrochloric acid, hydrobromic acid, acetic acid, or benzene sulfonic acid. A pharmaceutical composition or formulation refers to a composition or formulation in a form suitable for administration into a cell, or a subject such as a human (*e.g*., systemic administration). The formulations of the present disclosure, having an amount of dsRNA sufficient to treat or prevent a disorder associated with VEGF gene expression are, for example, suitable for topical (*e.g*., creams, ointments, skin patches, eye drops, ear drops) application or administration. Other routes of administration include oral, parenteral, sublingual, bladder wash-out, vaginal, rectal, enteric, suppository, nasal, and inhalation. The term parenteral, as used herein, includes subcutaneous, intravenous, intramuscular, intraarterial, intraabdominal, intraperitoneal, intraarticular, intraocular or retrobulbar, intraaural, intrathecal, intracavitary, intracelial, intraspinal, intrapulmonary or transpulmonary, intrasynovial, and intraurethral injection or infusion techniques. The pharmaceutical compositions of the present disclosure are formulated to allow the dsRNA contained therein to be bioavailable upon administration to a subject.

In further embodiments, dsRNA of this disclosure can be formulated as oily suspensions or emulsions (*e.g*., oil-in-water) by suspending dsRNA in, for example, a vegetable oil *(e.g.,* arachis oil, olive oil, sesame oil or coconut oil) or a mineral oil (*e.g*., liquid paraffin). Suitable emulsifying agents can be naturally-occurring gums (*e.g*., gum acacia or gum tragacanth), naturally-occurring phosphatides (*e.g*., soy bean, lecithin, esters or partial esters derived from fatty acids and hexitol), anhydrides (*e.g*., sorbitan monooleate), or condensation products of partial esters with ethylene oxide (*e.g*., polyoxyethylene sorbitan monooleate). In certain embodiments, the oily suspensions or emulsions can optionally contain a thickening agent, such as beeswax, hard paraffin or cetyl alcohol. In related embodiments, sweetening agents and flavoring agents can optionally be added to provide palatable oral preparations. In yet other embodiments, these compositions can be preserved by the optionally adding an anti-oxidant, such as ascorbic acid.

In further embodiments, dsRNA of this disclosure can be formulated as syrups and elixirs with sweetening agents (*e.g*., glycerol, propylene glycol, sorbitol, glucose or sucrose). Such formulations can also contain a demulcent, preservative, flavoring, coloring agent, or any combination thereof. In other embodiments, pharmaceutical compositions comprising dsRNA of this disclosure can be in the form of a sterile, injectable aqueous or oleaginous suspension. The sterile injectable preparation can also be a sterile, injectable solution or suspension in a nontoxic parenterally acceptable diluent or solvent (*e.g*., as a solution in 1,3-butanediol). Among the exemplary acceptable vehicles and solvents useful in the compositions of this disclosure is water, Ringer's solution, or isotonic sodium chloride solution. In addition, sterile, fixed oils may be employed as a solvent or suspending medium for the dsRNA of this disclosure. For this purpose, any bland fixed oil can be employed including synthetic mono- or diglycerides. In addition, fatty acids such as oleic acid find use in the preparation of parenteral formulations.

Pharmaceutical compositions and methods are provided herein that feature the presence or administration of one or more dsRNA of this disclosure combined, complexed, or conjugated with a polypeptide, optionally formulated with a pharmaceutically-acceptable carrier, such as a diluent, stabilizer, buffer, or the like. The negatively charged dsRNA molecules may be administered to a patient in need thereof, with or without stabilizers, buffers, or the like. When desired, use of a liposome delivery mechanism and standard protocols for formation of liposomes can be followed. The compositions of the present disclosure may also be formulated and used as a tablet, capsule, or elixir for oral administration, as a suppository for rectal administration, as a sterile or pyrogen-free solution, or as a suspension for injection, either with or without other compounds known in the art. Thus, dsRNAs of the present disclosure may be administered in any form, such as nasally, transdermally, parenterally, or by local injection.

In accordance with this disclosure herein, dsRNA molecules (optionally substituted or modified or conjugated), compositions thereof, and methods for inhibiting expression of one or more VEGF family gene in a cell or organism are provided. In certain embodiments, this disclosure provides methods and dsRNA compositions for treating a subject, including a human cell, tissue or individual, having a disease or at risk of developing a disease caused by or associated with the expression of one or more VEGF family gene. In one embodiment, the method includes administering a dsRNA of this disclosure or a pharmaceutical composition containing the dsRNA to a cell or an organism, such as a mammal, such that expression of the target gene is silenced. Subjects (*e.g*., mammalian, human) amendable for treatment using the dsRNA molecules (optionally substituted or modified or conjugated), compositions thereof, and methods of the present disclosure include those suffering from one or more condition associated, at least in part, with overexpression or inappropriate expression of one or more VEGF family gene, or which are amenable to treatment by reducing expression of one or more VEGF family protein, including a hyperproliferative (*e.g*., cancer), angiogenic (*e.g*., age-related macular degeneration), metabolic (*e.g*., diabetes), or inflammatory (*e.g*., arthritis) disorder or condition.

Within exemplary embodiments, the compositions and methods of this disclosure are useful as therapeutic tools to regulate expression of one or more VEGF family member to treat or prevent symptoms of, for example, hyperproliferative disorders. Exemplary hyperproliferative disorders include neoplasms, carcinomas, sarcomas, tumors, or cancer. More exemplary hyperproliferative disorders include oral cancer, throat cancer, laryngeal cancer, esophageal cancer, pharyngeal cancer, nasopharyngeal cancer, oropharyngeal cancer, gastrointestinal tract cancer, small intestine cancer, colon cancer, rectal cancer, colorectal cancer, anal cancer, pancreatic cancer, breast cancer, cervical cancer, uterine cancer, vulvar cancer, vaginal cancer, urinary tract cancer, bladder cancer, kidney cancer, adrenocortical cancer, islet cell carcinoma, gallbladder cancer, stomach cancer, prostate cancer, ovarian cancer, endometrial cancer, trophoblastic tumor, testicular cancer, penial cancer, bone cancer, osteosarcoma, liver cancer, extrahepatic bile duct cancer, skin cancer, basal cell carcinoma, lung cancer, small cell lung cancer, non-small cell lung cancer (NSCLC), brain cancer, melanoma, Kaposi's sarcoma, eye cancer, head and neck cancer, squamous cell carcinoma of head and neck, tymoma, thymic carcinoma, thyroid cancer, parathyroid cancer, Hippel-Lindau syndrome, leukemia, acute myeloid leukemia, chronic myelogenous leukemia, acute lymphoblastic leukemia, hairy cell leukemia, lymphoma, non-Hodgkin's lymphoma, Burkitt's lymphoma, T-cell lymphoma, multiple myeloma, malignant pleural mesothelioma, Barrett's adenocarcinoma, Wilm's tumor, or the like.

Exemplary inflammatory disorders include diabetes mellitus, rheumatoid arthritis, pannus growth in inflamed synovial lining, collagen-induced arthritis, spondylarthritis, ankylosing spondylitis, multiple sclerosis, encephalomyelitis, inflammatory bowel disease, Chron's disease, psoriasis or psoriatic arthritis, myasthenia gravis, systemic lupus erythematosis, graft-versus-host disease, and allergies. Other exemplary disorders include ocular neovascularization (*e.g*., retinal ischaemia, macular degeneration, diabetic retinopathy), glomerulonephritis, asthma, chronic bronchitis, lymphangiogenesis, and atherosclerosis.

In any of the methods disclosed herein there may be used with one or more dsRNA, or substituted or modified dsRNA as described herein, that comprises a first strand that is complementary to a human vascular endothelial growth factor (VEGF) family mRNA as set forth in SEQ ID NO:1158, 1159, 1160, 1161, 1162, 1163, or 1164 and is fully complementary, with up to three mismatches, to at least one other human VEGF family mRNA selected from SEQ ID NO:1165, 1166, 1167, or 1168, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein the mdRNA molecule optionally includes at least one double-stranded region of 5 base pairs to 13 base pairs. In other embodiments, subjects can be effectively treated, prophylactically or therapeutically, by administering an effective amount of one or more dsRNA having a first strand that is complementary to a human VEGF family mRNA as set forth in SEQ ID NO:1158, 1159, 1160, 1161, 1162, 1163, or 1164 and is fully complementary, with up to three mismatches, to at least one other human VEGF family mRNA selected from SEQ ID NO:1165, 1166, 1167, or 1168, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein the mdRNA molecule optionally includes at least one double-stranded region of 5 base pairs to 13 base pairs and at least one pyrimidine of the mdRNA is substituted with a pyrimidine nucleoside according to Formula I or II: 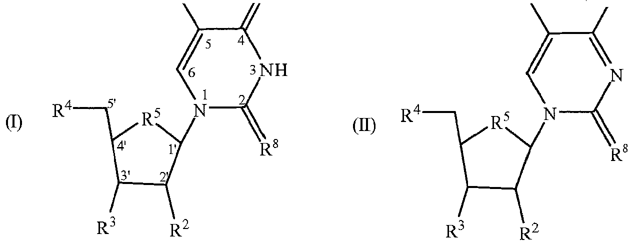 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C≡N, or heterocyclo group; R³ and R⁴ are each independently a hydroxyl, a protected hydroxyl, or an internucleoside linking group; and R⁵ and R⁸ are independently O or S. In certain embodiments, at least one nucleoside is according to Formula I in which R¹ is methyl and R² is -OH, or R¹ is methyl, R² is -OH, and R⁸ is S. In certain embodiments, at least one nucleoside is according to Formula I in which R¹ is methyl and R² is -O-methyl, or R¹ is methyl, R² is -O-methyl, and R⁸ is O. In other embodiments, the internucleoside linking group covalently links from about 5 to about 40 nucleosides.

In any of the methods described herein, the dsRNA used may include multiple modifications. For example, a dsRNA can have at least one 5-methyluridine, 2'-O-methyl-5-methyluridine, LNA, 2'-methoxy, 2'-fluoro, 2'-deoxy, phosphorothioate linkage, inverted base terminal cap, or any combination thereof. In certain exemplary methods, a dsRNA will have from one to all 5-methyluridines and have up to about 75% LNA. In other exemplary methods, a dsRNA will have from one to all 5-methyluridines and have up to about 75% 2'-methoxy provided the 2'-methoxy are not at the Argonaute cleavage site. In still other exemplary methods, a dsRNA will have from one to all 5-methyluridines and have up to about 100% 2'-fluoro substitutions. In further exemplary methods, a dsRNA will have from one to all 5-methyluridines and have up to about 75% 2'-deoxy. In further exemplary methods, a dsRNA will have up to about 75% LNA and have up to about 75% 2'-methoxy. In still other embodiments, a dsRNA will have up to about 75% LNA and have up to about 100% 2'-fluoro. In further exemplary methods, a dsRNA will have up to about 75% LNA and have up to about 75% 2'-deoxy. In further exemplary methods, a dsRNA will have up to about 75% 2'-methoxy and have up to about 100% 2'-fluoro. In further exemplary methods, a dsRNA will have up to about 75% 2'-methoxy and have up to about 75% 2'-deoxy. In further embodiments, a dsRNA will have up to about 100% 2'-fluoro and have up to about 75% 2'-deoxy.

In other exemplary methods for using multiply modified dsRNA, a dsRNA will have from one to all uridines substituted with 5-methyluridine, up to about 75% LNA, and up to about 75% 2'-methoxy. In still further exemplary methods, a dsRNA will have from one to all 5-methyluridines, up to about 75% LNA, and up to about 100% 2'-fluoro. In further exemplary methods, a dsRNA will have from one to all 5-methyluridines, up to about 75% LNA, and up to about 75% 2'-deoxy. In further exemplary methods, a dsRNA will have from one to all 5-methyluridines, up to about 75% 2'-methoxy, and up to about 75% 2'-fluoro. In further exemplary methods, a dsRNA will have from one to all 5-methyluridines, up to about 75% 2'-methoxy, and up to about 75% 2'-deoxy. In more exemplary methods, a dsRNA will have from one to all 5-methyluridines, up to about 100% 2'-fluoro, and up to about 75% 2'-deoxy. In yet other exemplary methods, a dsRNA will have from one to all 5-methyluridines, up to about 75% LNA, up to about 75% 2'-methoxy, up to about 100% 2'-fluoro, and up to about 75% 2'-deoxy. In other exemplary methods, a dsRNA will have up to about 75% LNA, up to about 75% 2'-methoxy, and up to about 100% 2'-fluoro. In further exemplary methods, a dsRNA will have up to about 75% LNA, up to about 75% 2'-methoxy, and up to about 75% 2'-deoxy. In more exemplary methods, a dsRNA will have up to about 75% LNA, up to about 100% 2'-fluoro, and up to about 75% 2'-deoxy. In still further exemplary methods, a dsRNA will have up to about 75% 2'-methoxy, up to about 100% 2'-fluoro, and up to about 75% 2'-deoxy.

In any of these exemplary methods for using multiply modified dsRNA, the dsRNA may further comprise up to 100% phosphorothioate internucleoside linkages, from one to ten or more inverted base terminal caps, or any combination thereof. Additionally, any of these dsRNA may have these multiple modifications on one strand, two strands, three strands, a plurality of strands, or all strands, or on the same or different nucleoside within a dsRNA molecule. Finally, in any of these multiple modification dsRNA, the dsRNA must have gene silencing activity.

In further embodiments, subjects can be effectively treated prophylactically or therapeutically by administering an effective amount of one or more dsRNA, or substituted or modified dsRNA as described herein, having a first strand that is complementary to a human VEGF family mRNA as set forth in SEQ ID NO:1158, 1159, 1160, 1161, 1162, 1163, or 1164 and is fully complementary, with up to three mismatches, to at least one other human VEGF family mRNA selected from SEQ ID NO:1165, 1166, 1167, or 1168, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein the combined double-stranded regions total about 15 base pairs to about 40 base pairs and the mdRNA molecule optionally has blunt ends. In still further embodiments, methods disclosed herein there may be used with one or more dsRNA that comprises a first strand that is complementary to a human VEGF family mRNA as set forth in SEQ ID NO:1158, 1159, 1160, 1161, 1162, 1163, or 1164 and is fully complementary, with up to three mismatches, to at least one other human VEGF family mRNA selected from SEQ ID NO:1165, 1166, 1167, or 1168, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein the combined double-stranded regions total about 15 base pairs to about 40 base pairs or the mdRNA molecule optionally includes at least one double-stranded region of 5 base pairs to 13 base pairs or optionally has blunt ends, or any combination thereof, and at least one pyrimidine of the mdRNA is has a pyrimidine nucleoside according to Formula I or II: 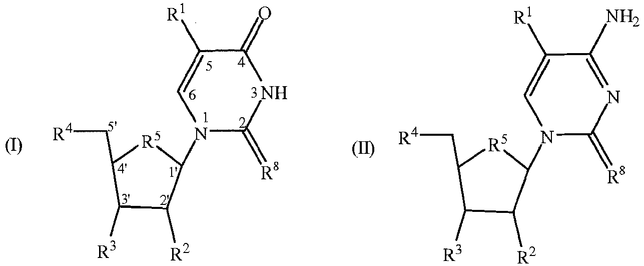 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C≡, or heterocyclo group; R³ and R⁴ are each independently a hydroxyl, a protected hydroxyl, or an internucleoside linking group; and R⁵ and R⁸ are independently O or S. In certain embodiments, at least one nucleoside is according to Formula I in which R¹ is methyl and R² is -OH, or R¹ is methyl, R² is -OH, and R⁸ is S. In certain embodiments, at least one nucleoside is according to Formula I in which R¹ is methyl and R² is -O-methyl, or R¹ is methyl, R² is -O-methyl, and R⁸ is O. In other embodiments, the internucleoside linking group covalently links from about 5 to about 40 nucleosides.

Within additional aspects of this disclosure, combination formulations and methods are provided comprising an effective amount of one or more dsRNA of the present disclosure in combination with one or more secondary or adjunctive active agents that are formulated together or administered coordinately with the dsRNA of this disclosure to control one or more VEGF family member-associated disease or condition as described herein. Useful adjunctive therapeutic agents in these combinatorial formulations and coordinate treatment methods include, for example, enzymatic nucleic acid molecules, allosteric nucleic acid molecules, antisense, decoy, or aptamer nucleic acid molecules, antibodies such as monoclonal antibodies, small molecules and other organic or inorganic compounds including metals, salts and ions, and other drugs and active agents indicated for treating one or more VEGF family member-associated disease or condition, including chemotherapeutic agents used to treat cancer, steroids, non-steroidal anti-inflammatory drugs (NSAIDs), or the like.

Exemplary chemotherapeutic agents include alkylating agents (*e.g.*, cisplatin, oxaliplatin, carboplatin, busulfan, nitrosoureas, nitrogen mustards, uramustine, temozolomide), antimetabolites (*e.g*., aminopterin, methotrexate, mercaptopurine, fluorouracil, cytarabine), taxanes (*e.g*., paclitaxel, docetaxel), anthracyclines (*e.g*., doxorubicin, daunorubicin, epirubicin, idaruicin, mitoxantrone, valrubicin), bleomycin, mytomycin, actinomycin, hydroxyurea, topoisomerase inhibitors (*e.g*., camptothecin, topotecan, irinotecan, etoposide, teniposide), monoclonoal antibodies (*e.g*., alemtuzumab, bevacizumab, cetuximab, gemtuzumab, panitumumab, rituximab, tositumomab, trastuzumab,), vinca alkaloids (*e.g*., vincristine, vinblastine, vindesine, vinorelbine), cyclophosphamide, prednisone, leucovorin, oxaliplatin.

To practice the coordinate administration methods of this disclosure, a dsRNA is administered, simultaneously or sequentially, in a coordinate treatment protocol with one or more of the secondary or adjunctive therapeutic agents contemplated herein. The coordinate administration may be done in either order, and there may be a time period while only one or both (or all) active therapeutic agents, individually or collectively, exert their biological activities. A distinguishing aspect of all such coordinate treatment methods is that the dsRNA present in the composition elicits some favorable clinical response, which may or may not be in conjunction with a secondary clinical response provided by the secondary therapeutic agent. Often, the coordinate administration of the dsRNA with a secondary therapeutic agent as contemplated herein will yield an enhanced therapeutic response beyond the therapeutic response elicited by either or both the purified dsRNA or secondary therapeutic agent alone.

In another embodiment, a dsRNA of this disclosure can include a conjugate member on one or more of the terminal nucleotides of a dsRNA. The conjugate member can be, for example, a lipophile, a terpene, a protein binding agent, a vitamin, a carbohydrate, or a peptide. For example, the conjugate member can be naproxen, nitroindole (or another conjugate that contributes to stacking interactions), folate, ibuprofen, or a C5 pyrimidine linker. In other embodiments, the conjugate member is a glyceride lipid conjugate (*e.g*., a dialkyl glyceride derivatives), vitamin E conjugates, or thio-cholesterols. Additional conjugate members include peptides that function, when conjugated to a modified dsRNA of this disclosure, to facilitate delivery of the dsRNA into a target cell, or otherwise enhance delivery, stability, or activity of the dsRNA when contacted with a biological sample (*e.g.*, a target cell expressing VEGFR). Exemplary peptide conjugate members for use within these aspects of this disclosure, include peptides PN27, PN28, PN29, PN58, PN61, PN73, PN158, PN159, PN173, PN182, PN183, PN202, PN204, PN250, PN361, PN365, PN404, PN453, PN509, and PN963, described, for example, in U.S. Patent Application Publication Nos. 2006/0040882 and 2006/0014289, and U.S. Provisional Patent Application Nos. 60/822,896 and 60/939,578; and PCT Application PCT/US2007/075744, which are all incorporated herein by reference. In certain embodiments, when peptide conjugate partners are used to enhance delivery of dsRNA of this disclosure, the resulting dsRNA formulations and methods will often exhibit further reduction of an interferon response in target cells as compared to dsRNAs delivered in combination with alternate delivery vehicles, such as lipid delivery vehicles (*e.g*., Lipofectamine™).

In still another embodiment, a dsRNA or analog thereof of this disclosure may be conjugated to the polypeptide and admixed with one or more non-cationic lipids or a combination of a non-cationic lipid and a cationic lipid to form a composition that enhances intracellular delivery of the dsRNA as compared to delivery resulting from contacting the target cells with a naked dsRNA. In more detailed aspects of this disclosure, the mixture, complex or conjugate comprising a dsRNA and a polypeptide can be optionally combined with *(e.g.,* admixed or complexed with) a cationic lipid, such as Lipofectine™. To produce these compositions comprised of a polypeptide, dsRNA and a cationic lipid, the dsRNA and peptide may be mixed together first in a suitable medium such as a cell culture medium, after which the cationic lipid is added to the mixture to form a dsRNA/delivery peptide/cationic lipid composition. Optionally, the peptide and cationic lipid can be mixed together first in a suitable medium such as a cell culture medium, followed by the addition of the dsRNA to form the dsRNA/delivery peptide/cationic lipid composition.

This disclosure also features the use of dsRNA compositions comprising surface-modified liposomes containing, for example, poly(ethylene glycol) lipids (PEG-modified, or long-circulating liposomes or stealth liposomes) (Lasic et al., Chem. Rev. 95:2601, 1995; Ishiwata et al., Chem. Pharm. Bull. 43:1005, 1995; Lasic et al., Science 267:1275, 1995; Oku et al., Biochim. Biophys. Acta 1238:86, 1995; Liu et al., J. Biol. Chem. 42:24864, 1995; PCT Publication Nos. WO 96/10391; WO 96/10390; WO 96/10392).

In another embodiment, compositions are provided for targeting dsRNA molecules of this disclosure to specific cell types, such as hepatocytes. For example, dsRNA can be complexed or conjugated glycoproteins or synthetic glycoconjugates glycoproteins or synthetic glycoconjugates having branched galactose (*e.g*., asialoorosomucoid), *N*-acetyl-*D-*galactosamine, or mannose *(see, e.g.,* Wu and Wu, J. Biol. Chem. 262:4429, 1987; Baenziger and Fiete, Cell 22: 611, 1980; Connolly et al., J. Biol. Chem. 257:939, 1982; Lee and Lee, Glycoconjugate J. 4:317, 1987; Ponpipom et al., J. Med. Chem. 24:1388, 1981) for a targeted delivery to, for example, the liver.

A pharmaceutically effective dose is that dose required to prevent, inhibit the occurrence of, or treat (alleviate a symptom to some extent, preferably all of the symptoms) a disease state. The pharmaceutically effective dose depends on the type of disease, the composition used, the route of administration, the type of subject being treated, the physical characteristics of the specific subject under consideration for treatment, concurrent medication, and other factors that those skilled in the medical arts will recognize. For example, an amount between 0.1 mg/kg and 100 mg/kg body weight/day of active ingredients may be administered depending on the potency of a dsRNA of this disclosure.

A specific dose level for any particular patient depends upon a variety of factors including the activity of the specific compound employed, age, body weight, general health, sex, diet, time of administration, route of administration, rate of excretion, drug combination, and the severity of the particular disease undergoing therapy. Following administration of dsRNA compositions as disclosed herein, test subjects will exhibit about a 10% up to about a 99% reduction in one or more symptoms associated with the disease or disorder being treated, as compared to placebo-treated or other suitable control subjects.

Dosage levels in the order of about 0.1 mg to about 140 mg per kilogram of body weight per day can be useful in the treatment of the above-indicated conditions (about 0.5 mg to about 7 g per patient per day). The amount of active ingredient that can be combined with the carrier materials to produce a single dosage form varies depending upon the host treated and the particular mode of administration. Dosage unit forms generally contain between from about 1 mg to about 500 mg of an active ingredient.

A dosage form of a dsRNA or composition thereof of this disclosure can be liquid, an emulsion, or a micelle, or in the form of an aerosol or droplets. A dosage form of a dsRNA or composition thereof of this disclosure can be solid, which can be reconstituted in a liquid prior to administration. The solid can be administered as a powder. The solid can be in the form of a capsule, tablet, or gel. In addition to *in vivo* gene inhibition, a skilled artisan will appreciate that the dsRNA and analogs thereof of the present disclosure are useful in a wide variety of *in vitro* applications, such as scientific and commercial research (*e.g*., elucidation of physiological pathways, drug discovery and development), and medical and veterinary diagnostics.

Nucleic acid molecules and polypeptides can be administered to cells by a variety of methods known to those of skill in the art, including administration within formulations that comprise a dsRNA alone, a dsRNA and a polypeptide complex / conjugate alone, or that further comprise one or more additional components, such as a pharmaceutically acceptable carrier, diluent, excipient, adjuvant, emulsifier, stabilizer, preservative, or the like. Other exemplary substances used to approximate physiological conditions include pH adjusting and buffering agents, tonicity adjusting agents, and wetting agents, for example, sodium acetate, sodium lactate, sodium chloride, potassium chloride, calcium chloride, sorbitan monolaurate, triethanolamine oleate, and mixtures thereof. For solid compositions, conventional nontoxic pharmaceutically acceptable carriers can be used which include, for example, pharmaceutical grades of mannitol, lactose, starch, magnesium stearate, sodium saccharin, talcum, cellulose, glucose, sucrose, magnesium carbonate, and the like.

In certain embodiments, the dsRNA and compositions thereof can be encapsulated in liposomes, administered by iontophoresis, or incorporated into other vehicles, such as hydrogels, cyclodextrins, biodegradable nanocapsules, bioadhesive microspheres, or proteinaceous vectors *(see, e.g.,* PCT Publication No. WO 00/53722). In certain embodiments of this disclosure, the dsRNA may be administered in a time release formulation, for example, in a composition that includes a slow release polymer. The dsRNA can be prepared with carriers that will protect against rapid release, for example, a controlled release vehicle such as a polymer, microencapsulated delivery system, or bioadhesive gel. Prolonged delivery of the dsRNA, in various compositions of this disclosure can be brought about by including in the composition agents that delay absorption, for example, aluminum monosterate hydrogels and gelatin.

Alternatively, a nucleic acid/peptide/vehicle combination can be locally delivered by direct injection or by use of, for example, an infusion pump. Direct injection of the nucleic acid molecules of this disclosure, whether subcutaneous, intramuscular, or intradermal, can take place using standard needle and syringe methodologies or by needle-free technologies, such as those described in Conry et al., Clin. Cancer Res. 5:2330, 1999 and PCT Publication No. WO 99/31262.

The dsRNA of this disclosure and compositions thereof may be administered to subjects by a variety of mucosal administration modes, including oral, rectal, vaginal, intranasal, intrapulmonary, or transdermal delivery, or by topical delivery to the eyes, ears, skin, or other mucosal surfaces. In one embodiment, the mucosal tissue layer includes an epithelial cell layer, which can be pulmonary, tracheal, bronchial, alveolar, nasal, buccal, epidermal, or gastrointestinal. Compositions of this disclosure can be administered using conventional actuators, such as mechanical spray devices, as well as pressurized, electrically activated, or other types of actuators. The dsRNAs can also be administered in the form of suppositories, *e.g*., for rectal administration. For example, these compositions can be mixed with an excipient that is solid at room temperature but liquid at the rectal temperature so that the dsRNA is released. Such materials include, for example, cocoa butter and polyethylene glycols.

Further methods for delivery of nucleic acid molecules, such as the dsRNAs of this disclosure, are described, for example, in Boado et al., J. Pharm. Sci. 87:1308, 1998; Tyler et al., FEBS Lett. 421:280, 1999; Pardridge et al., Proc. Nat'l Acad. Sci. USA 92:5592, 1995; Boado, Adv. Drug Delivery Rev. 15:73, 1995; Aldrian-Herrada et al., Nucleic Acids Res. 26:4910, 1998; Tyler et al., Proc. Nat'l Acad. Sci. USA 96:7053-7058, 1999; Akhtar et al., Trends Cell Bio. 2:139, 1992; "Delivery Strategies for Antisense Oligonucleotide Therapeutics," ed. Akhtar, 1995, Maurer et al., Mol. Membr. Biol. 16:129, 1999; Hofland and Huang, Handb. Exp. Pharmacol 137:165, 1999; and Lee et al., ACS Symp. Ser. 752:184, 2000; PCT Publication No. WO 94/02595.

All U.S. patents, U.S. patent application publications, U.S. patent applications, foreign patents, foreign patent applications, non-patent publications, figures, and websites referred to in this specification are expressly incorporated herein by reference, in their entirety.

### EXAMPLES

### EXAMPLE 1

### KNOCKDOWN OF GENE EXPRESSION BY MDRNA

The gene silencing activity of dsRNA as compared to nicked or gapped versions of the same dsRNA was examined using a dual fluorescence assay. A total of 22 different genes were targeted at ten different sites each (*see* Table 1).

A Dicer substrate dsRNA molecule was used, which has a 25 nucleotide sense strand, a 27 nucleotide antisense strand, and a two deoxynucleotide overhang at the 3'-end of the antisense strand (referred to as a 25/27 dsRNA). The nicked version of each dsRNA Dicer substrate has a nick at one of positions 9 to 16 on the sense strand as measured from the 5'-end of the sense strand. For example, an ndsRNA having a nick at position 11 has three strands - a 5'-sense strand of 11 nucleotides, a 3'-sense strand of 14 nucleotides, and an antisense strand of 27 nucleotides (which is also referred to as an N11-14/27 mdRNA). In addition, each of the sense strands of the ndsRNA have three locked nucleic acids (LNAs) evenly distributed along each sense fragment. If the nick is at position 9, then the LNAs can be found at positions 2, 6, and 9 of the 5' sense strand fragment and at positions 11, 18, and 23 of the 3' sense strand fragment. If the nick is at position 10, then the LNAs can be found at positions 2, 6, and 10 of the 5' sense strand fragment and at positions 12, 18, and 23 of the 3' sense strand fragment. If the nick is at position 11, then the LNAs can be found at positions 2, 6, and 11 of the 5' sense strand fragment and at positions 13, 18, and 23 of the 3' sense strand fragment. If the nick is at position 12, then the LNAs can be found at positions 2, 6, and 12 of the 5' sense strand fragment and at positions 14, 18, and 23 of the 3' sense strand fragment. If the nick is at position 13, then the LNAs can be found at positions 2, 7, and 13 of the 5' sense strand fragment and at positions 15, 18, and 23 of the 3' sense strand fragment. If the nick is at position 14, then the LNAs can be found at positions 2, 7, and 14 of the 5' sense strand fragment and at positions 16, 18, and 23 of the 3' sense strand fragment. If the nick is at position 15, then the LNAs can be found at positions 2, 8, and 15 of the 5' sense strand fragment and at positions 17, 19, and 23 of the 3' sense strand fragment. If the nick is at position 16, then the LNAs can be found at positions 2, 8, and 16 of the 5' sense strand fragment and at positions 18, 19, and 23 of the 3' sense strand fragment. Similarly, a gapped version of each dsRNA Dicer substrate has a single nucleotide missing at one of positions 10 to 17 on the sense strand as measured from the 5'-end of the sense strand. For example, a gdsRNA having a gap at position 11 has three strands - a 5'-sense strand of 11 nucleotides, a 3'-sense strand of 13 nucleotides, and an antisense strand of 27 nucleotides (which is also referred to as G 11-(1)-13/27 mdRNA). In addition, each of the sense strands of the gdsRNA contain three locked nucleic acids (LNAs) evenly distributed along each sense fragment (as described for the nicked counterparts).

In sum, three dsRNA were tested at each of the ten different sites per gene - an unmodified dsRNA, a nicked mdRNA with three LNAs per sense strand fragment, and a single nucleotide gapped mdRNA with three LNAs per sense strand fragment. In other words, 660 different dsRNA were examined.

Briefly, multiwell plates were seeded with about 7-8 x 10⁵ HeLa cells/well in DMEM having 10% fetal bovine serum, and incubated overnight at 37°C / 5% CO₂. The HeLa cell medium was changed to serum-free DMEM just prior to transfection. The psiCHECK™-2 vector, containing about a 1,000 basepair insert of a target gene, diluted in serum-free DMEM was mixed with diluted GenJet™ transfection reagent (SignalDT Biosystems, Hayward, California) according to the manufacturer's instructions and then incubated at room temperature for 10 minutes. The GenJet/ psiCHECK™-2-[target gene insert] solution was added to the HeLa cells and then incubated at 37°C, 5% CO₂ for 4.5 hours. After the vector transfection, cells were trypsinized and suspended in antibiotic-free DMEM containing 10% FBS at a concentration of 10⁵ cells per mL.

To transfect the dsRNA, the dsRNA was formulated in OPTI-MEM I reduced serum medium (Gibco® Invitrogen, Carlsbad, California) and placed in multiwell plates. Then Lipofectamine™ RNAiMAX (Invitrogen) was mixed with OPTI-MEM per manufacture's specifications, added to each well containing dsRNA, mixed manually, and incubated at room temperature for 10-20 minutes. Then 30 µL of vector-transfected HeLa cells at 10⁵ cells per mL were added to each well (final dsRNA concentration of 25 nM), the plates were spun for 30 seconds at 1,000 rpm, and then incubated at 37°C / 5% CO₂ for 2 days. The Cell Titer Blue (CTB) reagent (Promega, Madison, Wisconson) was used to assay for cell viability and proliferation - none of the dsRNA showed any substantial toxicity.

After transfecting, the media and CTB reagent were removed and the wells washed once with 100 PBS. Cells were assayed for firefly and *Renilla* luciferase reporter activity by first adding Dual-Glo™ Luciferase Reagent (Promega, Madison, WI) for 10 minutes with shaking, and then quantitating the luminescent signal on a VICTOR³™ 1420 Multilabel Counter (PerkinElmer). After measuring the firefly luminescence, Stop & Glo^{®} Reagent (Promega, Madison, WI) was added for 10 minutes with shaking to simultaneously quench the firefly reaction and initiate the *Renilla* luciferase reaction, which was then quantitated on a VICTOR³™ 1420 Multilabel Counter (PerkinElmer). The results are presented in Table 1.

**Table 1. Gene Silencing Activity* of dsRNA Dicer Substrate and mdRNA (nicked or gapped) Dicer Substrate**

| **Set** | **Target** | **Pos†** | **Dicer SEQ ID NOS‡** | **Dicer Mean (%)** | **Dicer 95% CI** | **Nicked SEQ ID NOS** | **Nicked Mean (%)** | **Nicked 95% CI** | **Gapped SEQ ID NOS** | **Gapped Mean (%)** | **Gapped 95% CI** | **Length 5'-S^** |
|---|---|---|---|---|---|---|---|---|---|---|---|---|
| 1 | AKT1 | 1862 | 63,283 | 20.6 | 4.0% | 503, 723, 283 | 23.5 | 5.7% | 503,940,283 | 54.3 | 12.0% | 14 |
| 2 | AKT1 | 1883 | 64,284 | 29.7 | 7.3% | 504,724,284 | 51.4 | 6.7% | 504,941,284 | 76.9 | 19.5% | 12 |
| 3 | AKT1 | 2178 | 65,285 | 15.4 | 2.4% | 505,725,285 | 22.3 | 6.4% | 505,942,285 | 24.4 | 5.1% | 14 |
| 4 | AKT1 | 2199 | 66,286 | 26.4 | 3.6% | 506,726,286 | 62.7 | 6.6% | 506,943,286 | 66.8 | 10.8% | 15 |
| 5 | AKT1 | 2264 | 67,287 | 35.2 | 7.3% | 507,727,287 | 34.1 | 7.3% | 507,944,287 | 31.3 | 5.2% | 12 |
| 6 | AKT1 | 2580 | 68,288 | 27.6 | 5.7% | 508,728,288 | 40.1 | 8.3% | 508,945,288 | 91.5 | 17.0% | 12 |
| 7 | AKT1 | 2606 | 69,289 | 14.0 | 2.6% | 509,729,289 | 14.9 | 3.2% | 509,946,289 | 33.4 | 6.9% | 11 |
| 8 | AKT1 | 2629 | 70,290 | 21.0 | 10.1% | 510,730,290 | 13.5 | 2.4% | 510,947,290 | 13.6 | 2.1% | 12 |
| 9 | AKT1 | 2661 | 71,291 | 37.4 | 6.6% | 511,731,291 | 41.0 | 12.1% | 511, 948, 291 | 71.6 | 11.9% | 15 |
| 10 | AKT1 | 2663 | 72,292 | 18.1 | 4.3% | 512,732,292 | 23.0 | 5.9% | 512,949,292 | 51.4 | 9.2% | 14 |
| 11 | BCR-ABL (b2a2) | 66 | 73,293 | 16.9 | 5.9% | 513, 733, 293 | 30.4 | 10.5% | 513,950,293 | 38.2 | 11.7% | 13 |
| 12 | BCR-ABL (b2a2) | 190 | 74,294 | 40.0 | 11.6% | 514,734,294 | 22.0 | 6.4% | 514,951,294 | 34.6 | 12.0% | 14 |
| 13 | BCR-ABL (b2a2) | 282 | 75,295 | 24.2 | 5.2% | 515,735,295 | 37.6 | 8.2% | 515,952,295 | 34.6 | 8.6% | 13 |
| 14 | BCR-ABL (b2a2) | 284 | 76,296 | 50.9 | 6.9% | 516,736,296 | 38.3 | 7.8% | 516,953,296 | 68.3 | 18.0% | 13 |
| 15 | BCR-ABL (b2a2) | 287 | 77,297 | 45.5 | 13.2% | 517,737,297 | 39.6 | 11.5% | 517,954,297 | 75.2 | 17.2% | 14 |
| 16 | BCR-ABL (b2a2) | 289 | 78,298 | 36.9 | 7.7% | 518, 738, 298 | 40.0 | 8.9% | 518,955,298 | 60.9 | 12.3% | 14 |
| 17 | BCR-ABL (b2a2) | 293 | 79,299 | 55.9 | 9.8% | 519,739,299 | 58.6 | 14.7% | 519,956,299 | 87.0 | 14.3% | 13 |
| 18 | BCR-ABL (b2a2) | 461 | 80,300 | 38.4 | 9.4% | 520,740,300 | 35.9 | 12.1% | 520,957,300 | 28.6 | 10.2% | 13 |
| 19 | BCR-ABL (b2a2) | 462 | 81,301 | 31.1 | 13.7% | 521, 741, 301 | 26.5 | 5.5% | 521,958,301 | 35.8 | 10.7% | 14 |
| 20 | BCR-ABL (b2a2) | 561 | 82,302 | 17.7 | 3.4% | 522,742,302 | 20.7 | 3.4% | 522,959,302 | 35.5 | 10.6% | 12 |
| 21 | BCR-ABL (b3a2) | 352 | 83,303 | 45.4 | 7.0% | 523,743,303 | 39.8 | 8.3% | 523,960,303 | 45.5 | 11.0% | 12 |
| 22 | BCR-ABL (b3a2) | 353 | 84,304 | 22.6 | 1.8% | 524,744,304 | 20.5 | 5.1% | 524,961,304 | 66.1 | 17.8% | 12 |
| 23 | BCR-ABL (b3a2) | 356 | 85,305 | 11.9 | 2.5% | 525,745,305 | 28.4 | 5.8% | 525,962,305 | 56.0 | 10.6% | 13 |
| 24 | BCR-ABL (b3a2) | 357 | 86,306 | 24.5 | 6.0% | 526,746,306 | 25.6 | 7.5% | 526,963,306 | 39.2 | 10.0% | 13 |
| 25 | BCR-ABL (b3a2) | 359 | 87,307 | 56.8 | 9.3% | 527,747,307 | 42.4 | 7.3% | 527,964,307 | 46.4 | 9.5% | 13 |
| 26 | BCR-ABL (b3a2) | 360 | 88,308 | 32.3 | 5.0% | 528,748,308 | 37.2 | 7.3% | 528,965,308 | 55.3 | 13.8% | 13 |
| 27 | BCR-ABL (b3a2) | 362 | 89,309 | 12.4 | 3.2% | 529,737,309 | 26.3 | 9.8% | 529,954,309 | 46.2 | 8.3% | 14 |
| 28 | BCR-ABL (b3a2) | 410 | 90,310 | 66.2 | 12.2% | 530,749,310 | 55.9 | 11.2% | 530,966,310 | 58.4 | 16.4% | 12 |
| 29 | BCR-ABL (b3a2) | 629 | 91,311 | 35.0 | 11.7% | 531,750,311 | 46.5 | 10.1% | 531,967,311 | 41.0 | 9.0% | 13 |
| 30 | BCR-ABL (b3a2) | 727 | 92,312 | 83.4 | 13.6% | 532,751,312 | 76.7 | 22.5% | 532,968,312 | 62.9 | 10.9% | 12 |
| 31 | EGFR | 4715 | 93,313 | 15.3 | 2.2% | 533, 752, 313 | 9.4 | 0.9% | 533, 969, 313 | 11.3 | 1.7% | 11 |
| 32 | EGFR | 4759 | 94,314 | 3.8 | 0.4% | 534,753,314 | 6.3 | 0.8% | 534,970,314 | 8.4 | 1.1% | 12 |
| 33 | EGFR | 4810 | 95,315 | 5.2 | 0.6% | 535,754,315 | 5.8 | 0.7% | 535,971,315 | 7.2 | 1.0% | 13 |
| 34 | EGFR | 5249 | 96,316 | 2.6 | 0.4% | 536,755,316 | 16.6 | 1.8% | 536,972,316 | 42.9 | 3.5% | 14 |
| 35 | EGFR | 5279 | 97,317 | 7.6 | 1.0% | 537,756,317 | 10.6 | 1.1% | 537,973,317 | 11.8 | 1.7% | 13 |
| 36 | EGFR | 5374 | 98, 318 | 9.6 | 1.0% | 538,757,318 | 8.7 | 0.9% | 538,974,318 | 34.7 | 4.3% | 12 |
| 37 | EGFR | 5442 | 99,319 | 4.1 | 0.8% | 539,758,319 | 15.1 | 1.8% | 539,975,319 | 19.7 | 2.4% | 12 |
| 38 | EGFR | 5451 | 100,320 | 5.1 | 0.3% | 540,759,320 | 11.5 | 1.3% | 540,976,320 | 16.5 | 3.0% | 13 |
| 39 | EGFR | 5469 | 101,321 | 5.6 | 0.8% | 541,760,321 | 5.1 | 0.5% | 541,977,321 | 12.2 | 2.5% | 13 |
| 40 | EGFR | 5483 | 102,322 | 2.2 | 0.4% | 542,761,322 | 2.4 | 0.5% | 542,978,322 | 6.1 | 0.7% | 9 |
| 41 | FLT1 | 863 | 103,323 | 7.6 | 1.1% | 543,762,323 | 10.2 | 3.3% | 543,979,323 | 29.2 | 8.1% | 12 |
| 42 | FLT1 | 906 | 104,324 | 10.0 | 2.4% | 544,763,324 | 10.8 | 0.8% | 544,980,324 | 12.4 | 2.1% | 12 |
| 43 | FLT1 | 993 | 105,325 | 12.2 | 2.5% | 545,764,325 | 13.7 | 2.8% | 545,981,325 | 20.0 | 11.3% | 13 |
| 44 | FLT1 | 1283 | 106,326 | 19.6 | 4.5% | 546,765,326 | 25.8 | 7.3% | 546,982,326 | 18.7 | 6.5% | 12 |
| 45 | FLT1 | 1289 | 107,327 | 15.5 | 2.0% | 547,766,327 | 13.5 | 1.6% | 547,983,327 | 22.5 | 5.0% | 12 |
| 46 | FLT1 | 1349 | 108,328 | 36.8 | 4.2% | 548,767,328 | 22.9 | 4.0% | 548,984,328 | 52.7 | 5.4% | 14 |
| 47 | FLT1 | 1354 | 109,329 | 36.6 | 4.0% | 549,768,329 | 49.7 | 5.9% | 549,985,329 | 45.8 | 9.3% | 14 |
| 48 | FLT1 | 1448 | 110,330 | 9.3 | 2.5% | 550,769,330 | 16.1 | 2.9% | 550,986,330 | 24.2 | 3.6% | 13 |
| 49 | FLT1 | 1459 | 111,331 | 13.7 | 3.6% | 551,770,331 | 20.0 | 8.7% | 551,987,331 | 22.4 | 4.4% | 12 |
| 50 | FLT1 | 1700 | 112,332 | 7.9 | 2.2% | 552,771,332 | 11.2 | 3.7% | 552,988,332 | 36.4 | 8.0% | 13 |
| 51 | FRAP1 | 7631 | 113, 333 | 9.5 | 2.7% | 553,772,333 | 23.3 | 4.9% | 553,989,333 | 61.8 | 18.3% | 13 |
| 52 | FRAP1 | 7784 | 114,334 | 15.1 | 1.7% | 554,773,334 | 19.9 | 2.8% | 554,990,334 | 29.3 | 3.4% | 12 |
| 53 | FRAP1 | 7812 | 115,335 | 11.9 | 2.9% | 555,774,335 | 14.4 | 3.2% | 555,991,335 | 28.3 | 12.7% | 11 |
| 54 | FRAP1 | 7853 | 116,336 | 16.8 | 3.3% | 556,775,336 | 24.1 | 3.7% | 556,992,336 | 67.5 | 9.2% | 11 |
| 55 | FRAP1 | 8018 | 117,337 | 41.1 | 9.1% | 557,776,337 | 19.8 | 3.3% | 557,993,337 | 41.8 | 9.6% | 12 |
| 56 | FRAP1 | 8102 | 118,338 | 35.7 | 5.1% | 558, 777, 338 | 30.2 | 6.3% | 558,994,338 | 39.5 | 9.9% | 12 |
| 57 | FRAP1 | 8177 | 119,339 | 21.2 | 3.9% | 559,778,339 | 33.2 | 9.3% | 559,995,339 | 47.3 | 12.3% | 14 |
| 58 | FRAP1 | 8348 | 120,340 | 25.8 | 3.6% | 560,779,340 | 26.8 | 4.4% | 560,996,340 | 37.4 | 4.7% | 11 |
| 59 | FRAP1 | 8435 | 121,341 | 41.1 | 6.7% | 561,780,341 | 54.1 | 9.5% | 561,997,341 | 74.9 | 8.5% | 12 |
| 60 | FRAP1 | 8542 | 122,342 | 23.1 | 4.8% | 562,781,342 | 16.5 | 5.5% | 562,998,342 | 33.6 | 6.4% | 10 |
| 61 | HIF1A | 1780 | 123,343 | 76.6 | 14.9% | 563,782,343 | 89.2 | 11.9% | 563,999,343 | 86.3 | 9.3% | 12 |
| 62 | HIF1A | 1831 | 124,344 | 9.0 | 0.6% | 564,783,344 | 14.0 | 2.3% | 564,1000,344 | 38.2 | 8.5% | 12 |
| 63 | HIF1A | 1870 | 125,345 | 21.4 | 4.5% | 565,784,345 | 21.2 | 3.3% | 565,1001,345 | 19.6 | 2.2% | 13 |
| 64 | HIF1A | 1941 | 126,346 | 8.9 | 2.1% | 566,785,346 | 11.4 | 2.2% | 566,1002,346 | 11.7 | 2.5% | 12 |
| 65 | HIF1A | 2068 | 127,347 | 7.8 | 1.5% | 567,786,347 | 7.0 | 1.4% | 567,1003,347 | 16.9 | 3.9% | 12 |
| 66 | HIF1A | 2133 | 128,348 | 13.0 | 2.0% | 568,787,348 | 16.7 | 3.1% | 568,1004,348 | 16.3 | 3.1% | 10 |
| 67 | HIF1A | 2232 | 129,349 | 8.6 | 2.0% | 569,788,349 | 17.4 | 3.6% | 569,1005,349 | 37.8 | 9.6% | 13 |
| 68 | HIF1A | 2273 | 130, 350 | 19.1 | 5.3% | 570,789,350 | 23.4 | 4.4% | 570,1006,350 | 20.3 | 3.4% | 12 |
| 69 | HIF1A | 2437 | 131,351 | 8.2 | 1.4% | 571,790,351 | 47.7 | 11.5% | 571,1007,351 | 72.4 | 14.3% | 13 |
| 70 | HIF1A | 2607 | 132, 352 | 8.0 | 2.1% | 572,791,352 | 11.0 | 1.2% | 572,1008,352 | 33.6 | 6.0% | 13 |
| 71 | IL17A | 923 | 133, 353 | 5.0 | 0.6% | 573,792,353 | 7.3 | 0.7% | 573,1009,353 | 26.3 | 2.5% | 12 |
| 72 | IL17A | 962 | 134, 354 | 6.7 | 0.8% | 574,793,354 | 7.7 | 0.9% | 574,1010,354 | 8.9 | 2.0% | 13 |
| 73 | IL17A | 969 | 135, 355 | 8.9 | 1.7% | 575,794,355 | 17.1 | 1.6% | 575,1011,355 | 49.5 | 4.3% | 14 |
| 74 | IL17A | 1098 | 136, 356 | 7.2 | 1.3% | 576,795,356 | 10.0 | 2.4% | 576,1012,356 | 15.4 | 2.8% | 12 |
| 75 | IL17A | 1201 | 137,357 | 14.1 | 2.2% | 577,796,357 | 13.4 | 1.1% | 577,1013,357 | 17.2 | 2.8% | 12 |
| 76 | IL17A | 1433 | 138,358 | 107.1 | 9.7% | 578,797,358 | 111.5 | 10.4% | 578,1014,358 | 108.1 | 8.8% | 13 |
| 77 | IL17A | 1455 | 139, 359 | 115.4 | 11.1% | 579,798,359 | 120.8 | 8.7% | 579,1015,359 | 120.3 | 9.9% | 12 |
| 78 | IL17A | 1478 | 140,360 | 82.7 | 6.3% | 580,799,360 | 87.6 | 5.0% | 580,1016,360 | 95.9 | 5.6% | 14 |
| 79 | IL17A | 1663 | 141,361 | 140.2 | 7.8% | 581,800,361 | 125.9 | 9.8% | 581,1017,361 | 114.7 | 10.1% | 14 |
| 80 | IL17A | 1764 | 142,362 | 114.3 | 9.2% | 582,801,362 | 109.4 | 2.9% | 582,1018,362 | 105.7 | 8.1% | 15 |
| 81 | IL18 | 210 | 143,363 | 13.8 | 2.8% | 583,802,363 | 23.9 | 5.8% | 583,1019,363 | 21.4 | 5.7% | 14 |
| 82 | IL18 | 368 | 144,364 | 22.5 | 1.8% | 584,803,364 | 21.0 | 2.0% | 584,1020,364 | 29.7 | 3.7% | 13 |
| 83 | IL18 | 479 | 145,365 | 88.1 | 12.9% | 585,804,365 | 66.3 | 9.8% | 585,1021,365 | 80.0 | 16.8% | 14 |
| 84 | IL18 | 508 | 146,366 | 8.0 | 1.9% | 586,805,366 | 15.7 | 3.5% | 586,1022,366 | 17.0 | 5.7% | 12 |
| 85 | IL18 | 521 | 147,367 | 9.9 | 2.1% | 587,806,367 | 10.8 | 2.1% | 587,1023,367 | 18.4 | 3.3% | 11 |
| 86 | IL18 | 573 | 148,368 | 18.6 | 4.7% | 588,807,368 | 24.8 | 7.6% | 588,1024,368 | 48.8 | 7.7% | 14 |
| 87 | IL18 | 605 | 149,369 | 27.5 | 6.1% | 589,808,369 | 21.3 | 3.9% | 589,1025,369 | 14.9 | 2.7% | 13 |
| 88 | IL18 | 663 | 150,370 | 5.3 | 1.0% | 590,809,370 | 8.2 | 1.5% | 590,1026,370 | 11.7 | 3.4% | 12 |
| 89 | IL18 | 785 | 151,371 | 8.6 | 1.0% | 591,810,371 | 11.7 | 2.8% | 591,1027,371 | 21.1 | 9.1% | 12 |
| 90 | IL18 | 918 | 152,372 | 13.9 | 1.6% | 592,811,372 | 15.0 | 3.0% | 592,1028,372 | 30.4 | 3.6% | 11 |
| 91 | IL6 | 24 | 153,373 | 22.6 | 1.7% | 593,812,373 | 45.7 | 7.8% | 593,1029,373 | 47.8 | 4.5% | 13 |
| 92 | IL6 | 74 | 154,374 | 52.5 | 12.6% | 594,813,374 | 56.4 | 7.1% | 594,1030,374 | 88.3 | 15.5% | 12 |
| 93 | IL6 | 160 | 155,375 | 49.8 | 7.8% | 595,814,375 | 50.6 | 6.1% | 595,1031,375 | 68.3 | 9.4% | 14 |
| 94 | IL6 | 370 | 156,376 | 44.7 | 8.2% | 596,815,376 | 52.5 | 4.2% | 596,1032,376 | 74.3 | 9.3% | 13 |
| 95 | IL6 | 451 | 157,377 | 39.3 | 5.0% | 597,816,377 | 35.6 | 4.1% | 597,1033,377 | 66.6 | 7.1% | 13 |
| 96 | IL6 | 481 | 158,378 | 68.3 | 8.1% | 598,817,378 | 78.7 | 15.6% | 598,1034,378 | 63.2 | 6.2% | 11 |
| 97 | IL6 | 710 | 159,379 | 29.2 | 4.2% | 599,818,379 | 32.0 | 4.1% | 599,1035,379 | 77.3 | 11.4% | 12 |
| 98 | IL6 | 822 | 160,380 | 73.7 | 11.0% | 600,819,380 | 72.2 | 11.6% | 600,1036,380 | 85.2 | 13.3% | 12 |
| 99 | IL6 | 836 | 161,381 | 98.8 | 21.8% | 601,820,381 | 95.0 | 13.2% | 601,1037,381 | 90.5 | 15.6% | 13 |
| 100 | IL6 | 960 | 162,382 | 31.1 | 4.4% | 602,821,382 | 20.5 | 6.1% | 602,1038,382 | 25.6 | 2.4% | 12 |
| 101 | MAP2K1 | 1237 | 163,383 | 21.0 | 3.3% | 603,822,383 | 27.9 | 3.8% | 603,1039,383 | 50.0 | 8.8% | 11 |
| 102 | MAP2K1 | 1342 | 164,384 | 3.9 | 0.5% | 604,823,384 | 8.7 | 1.5% | 604, 1040, 384 | 11.4 | 1.3% | 13 |
| 103 | MAP2K1 | 1501 | 165,385 | 12.9 | 1.9% | 605,824,385 | 19.4 | 2.9% | 605,1041,385 | 19.7 | 5.3% | 12 |
| 104 | MAP2K1 | 1542 | 166,386 | 7.2 | 1.3% | 606,825,386 | 11.7 | 2.1% | 606,1042,386 | 18.7 | 3.2% | 11 |
| 105 | MAP2K1 | 1544 | 167,387 | 13.1 | 2.1% | 607,826,387 | 11.1 | 1.1% | 607,1043,387 | 16.5 | 3.0% | 10 |
| 106 | MAP2K1 | 1728 | 168,388 | 11.9 | 1.7% | 608,827,388 | 11.9 | 1.0% | 608,1044,388 | 27.9 | 4.3% | 13 |
| 107 | MAP2K1 | 1777 | 169,389 | 18.3 | 2.8% | 609,828,389 | 37.2 | 4.3% | 609, 1045, 389 | 64.5 | 8.5% | 13 |
| 108 | MAP2K1 | 1892 | 170,390 | 34.5 | 4.7% | 610,829,390 | 37.6 | 6.8% | 610, 1046, 390 | 42.4 | 7.3% | 12 |
| 109 | MAP2K1 | 1954 | 171,391 | 4.6 | 0.5% | 611,830,391 | 4.2 | 0.5% | 611, 1047, 391 | 6.5 | 1.1% | 13 |
| 110 | MAP2K1 | 2062 | 172,392 | 10.2 | 0.8% | 612,831,392 | 10.4 | 2.9% | 612, 1048, 392 | 12.2 | 2.0% | 12 |
| 111 | MAPK1 | 3683 | 173,393 | 7.0 | 0.9% | 613,614,393 | 24.4 | 17.3% | 613, 1049, 393 | 25.2 | 2.6% | 12 |
| 112 | MAPK1 | 3695 | 174,394 | 32.9 | 4.6% | 614,832,394 | 30.9 | 4.0% | 614, 1050, 394 | 33.8 | 3.1% | 13 |
| 113 | MAPK1 | 3797 | 175,395 | 7.4 | 1.1% | 615,833,395 | 6.4 | 1.3% | 615, 1051, 395 | 40.4 | 5.8% | 11 |
| 114 | MAPK1 | 3905 | 176,396 | 8.0 | 1.0% | 616,834,396 | 8.1 | 0.5% | 616, 1052, 396 | 14.8 | 1.4% | 12 |
| 115 | MAPK1 | 3916 | 177,397 | 11.0 | 1.7% | 617,835,397 | 16.0 | 3.3% | 617, 1053, 397 | 45.5 | 8.1% | 10 |
| 116 | MAPK1 | 3943 | 178,398 | 6.8 | 0.8% | 618,836,398 | 6.6 | 0.7% | 618, 1054, 398 | 11.0 | 2.3% | 10 |
| 117 | MAPK1 | 4121 | 179,399 | 7.6 | 1.1% | 619,837,399 | 12.7 | 1.6% | 619, 1055, 399 | 25.1 | 3.1% | 12 |
| 118 | MAPK1 | 4256 | 180,400 | 27.6 | 2.5% | 620,838,400 | 36.8 | 4.0% | 620, 1056, 400 | 57.7 | 7.0% | 13 |
| 119 | MAPK1 | 4294 | 181,401 | 31.0 | 3.0% | 621, 839, 401 | 22.3 | 3.6% | 621, 1057, 401 | 50.9 | 4.6% | 12 |
| 120 | MAPK1 | 4375 | 182,402 | 10.9 | 1.1% | 622,840,402 | 12.4 | 1.4% | 622, 1058, 402 | 16.9 | 2.7% | 11 |
| 121 | MAPK14 | 2715 | 183,403 | 11.4 | 2.8% | 623,841,403 | 16.5 | 4.1% | 623, 1059, 403 | 16.6 | 2.4% | 12 |
| 122 | MAPK14 | 2737 | 184,404 | 7.5 | 0.8% | 624,842,404 | 10.3 | 1.1% | 624, 1060, 404 | 13.1 | 1.2% | 11 |
| 123 | MAPK14 | 2750 | 185,405 | 8.7 | 1.0% | 625,843,405 | 12.2 | 1.8% | 625, 1061, 405 | 15.8 | 1.9% | 13 |
| 124 | MAPK14 | 2817 | 186,406 | 6.4 | 0.8% | 626,844,406 | 14.6 | 1.7% | 626, 1062, 406 | 19.4 | 2.0% | 11 |
| 125 | MAPK14 | 3091 | 187,407 | 9.9 | 0.6% | 627,845,407 | 10.3 | 1.3% | 627, 1063, 407 | 24.7 | 1.5% | 11 |
| 126 | MAPK14 | 3312 | 188,408 | 20.4 | 1.8% | 628,846,408 | 30.5 | 2.9% | 628, 1064, 408 | 38.5 | 3.4% | 13 |
| 127 | MAPK14 | 3346 | 189,409 | 20.9 | 1.6% | 629,847,409 | 23.0 | 2.6% | 629, 1065, 409 | 58.3 | 6.7% | 11 |
| 128 | MAPK14 | 3531 | 190,410 | 42.4 | 3.2% | 630, 848, 410 | 55.1 | 5.0% | 630, 1066, 410 | 61.9 | 3.6% | 12 |
| 129 | MAPK14 | 3621 | 191,411 | 28.6 | 1.9% | 631, 849, 411 | 42.4 | 13.5% | 631, 1067, 411 | 71.9 | 5.2% | 11 |
| 130 | MAPK14 | 3680 | 192,412 | 15.6 | 1.3% | 632,850,412 | 15.5 | 1.9% | 632, 1068, 412 | 19.8 | 2.1% | 12 |
| 131 | PDGFA | 1322 | 193,413 | 23.7 | 3.6% | 633,851,413 | 31.6 | 4.3% | 633, 1069, 413 | 38.4 | 3.3% | 12 |
| 132 | PDGFA | 1332 | 194,414 | 35.5 | 5.4% | 634,852,414 | 48.4 | 3.0% | 634, 1070, 414 | 65.4 | 10.5% | 14 |
| 133 | PDGFA | 1395 | 195,415 | 25.9 | 3.3% | 635,853,415 | 40.2 | 6.0% | 635, 1071, 415 | 55.2 | 9.8% | 14 |
| 134 | PDGFA | 1669 | 196,416 | 40.4 | 5.1% | 636, 854, 416 | 29.5 | 4.3% | 636, 1072, 416 | 33.9 | 5.9% | 12 |
| 135 | PDGFA | 1676 | 197,417 | 27.1 | 2.5% | 637, 855, 417 | 36.8 | 4.5% | 637, 1073, 417 | 47.4 | 3.4% | 13 |
| 136 | PDGFA | 1748 | 198,418 | 27.4 | 4.7% | 638, 856, 418 | 34.5 | 5.0% | 638, 1074, 418 | 47.5 | 4.7% | 11 |
| 137 | PDGFA | 2020 | 199,419 | 31.6 | 6.6% | 639,857,419 | 37.5 | 4.3% | 639, 1075, 419 | 51.9 | 5.0% | 13 |
| 138 | PDGFA | 2021 | 200,420 | 16.7 | 1.0% | 640,858,420 | 24.2 | 3.1% | 640, 1076, 420 | 62.6 | 6.9% | 14 |
| 139 | PDGFA | 2030 | 201,421 | 38.7 | 6.2% | 641, 859, 421 | 47.0 | 10.5% | 641, 1077, 421 | 80.5 | 7.6% | 13 |
| 140 | PDGFA | 2300 | 202,422 | 55.3 | 7.7% | 642, 860, 422 | 41.2 | 4.7% | 642, 1078, 422 | 71.7 | 9.1% | 15 |
| 141 | PDGFRA | 4837 | 203,423 | 16.9 | 3.1% | 643,861,423 | 21.1 | 5.1% | 643, 1079, 423 | 23.1 | 4.8% | 12 |
| 142 | PDGFRA | 4900 | 204,424 | 23.8 | 3.8% | 644,862,424 | 40.9 | 8.4% | 644, 1080, 424 | 62.5 | 12.5% | 16 |
| 143 | PDGFRA | 5007 | 205,425 | 52.6 | 9.4% | 645, 863, 425 | 49.6 | 7.7% | 645, 1081, 425 | 47.0 | 9.5% | 12 |
| 144 | PDGFRA | 5043 | 206,426 | 30.1 | 7.9% | 646,864,426 | 30.0 | 5.4% | 646, 1082, 426 | 57.3 | 7.8% | 11 |
| 145 | PDGFRA | 5082 | 207,427 | 8.3 | 1.1% | 647,865,427 | 11.9 | 1.8% | 647, 1083, 427 | 18.2 | 4.0% | 13 |
| 146 | PDGFRA | 5352 | 208,428 | 6.3 | 1.4% | 648,866,428 | 8.2 | 1.6% | 648, 1084, 428 | 7.9 | 1.1% | 12 |
| 147 | PDGFRA | 5367 | 209,429 | 19.1 | 5.6% | 649, 867, 429 | 10.9 | 1.6% | 649, 1085, 429 | 25.1 | 2.9% | 14 |
| 148 | PDGFRA | 5496 | 210,430 | 18.9 | 5.4% | 650,868,430 | 17.0 | 2.9% | 650, 1086, 430 | 17.8 | 4.0% | 12 |
| 149 | PDGFRA | 5706 | 211,431 | 24.5 | 4.0% | 651, 869, 431 | 47.8 | 4.3% | 651, 1087, 431 | 50.6 | 5.5% | 13 |
| 150 | PDGFRA | 5779 | 212,432 | 13.0 | 1.4% | 652,870,432 | 14.0 | 2.1% | 652, 1088, 432 | 17.2 | 4.3% | 14 |
| 151 | PIK3CA | 213 | 213,433 | 4.3 | 1.0% | 653,871,433 | 3.7 | 0.6% | 653, 1089, 433 | 5.7 | 0.9% | 12 |
| 152 | PIK3CA | 389 | 214,434 | 5.3 | 1.0% | 654,872,434 | 7.0 | 1.5% | 654, 1090, 434 | 5.6 | 1.5% | 10 |
| 153 | PIK3CA | 517 | 215,435 | 9.6 | 1.1% | 655,873,435 | 11.5 | 2.1% | 655, 1091, 435 | 13.5 | 1.6% | 11 |
| 154 | PIK3CA | 630 | 216,436 | 6.1 | 1.2% | 656,874,436 | 8.9 | 2.6% | 656, 1092, 436 | 9.3 | 1.8% | 12 |
| 155 | PIK3CA | 680 | 217,437 | 3.8 | 0.3% | 657,875,437 | 5.9 | 0.6% | 657, 1093, 437 | 6.9 | 1.0% | 11 |
| 156 | PIK3CA | 732 | 218,438 | 5.7 | 1.7% | 658, 876, 438 | 15.3 | 1.5% | 658, 1094, 438 | 17.4 | 4.0% | 11 |
| 157 | PIK3CA | 736 | 219,439 | 5.9 | 0.9% | 659,877,439 | 7.8 | 1.1% | 659, 1095, 439 | 6.5 | 1.4% | 12 |
| 158 | PIK3CA | 923 | 220,440 | 5.0 | 0.7% | 660,878,440 | 8.5 | 1.5% | 660, 1096, 440 | 7.4 | 0.6% | 12 |
| 159 | PIK3CA | 1087 | 221,441 | 8.1 | 2.3% | 661,879,441 | 8.5 | 1.6% | 661, 1097, 441 | 17.5 | 4.9% | 12 |
| 160 | PIK3CA | 1094 | 222,442 | 13.0 | 3.8% | 662,880,442 | 13.0 | 2.5% | 662, 1098, 442 | 30.1 | 6.4% | 11 |
| 161 | PKN3 | 2408 | 223,443 | 9.4 | 2.1% | 663,881,443 | 15.2 | 3.7% | 663,665,443 | 32.1 | 6.6% | 12 |
| 162 | PKN3 | 2420 | 224,444 | 14.5 | 1.7% | 664,882,444 | 30.4 | 7.5% | 664, 1099, 444 | 40.1 | 6.7% | 12 |
| 163 | PKN3 | 2421 | 225,445 | 15.2 | 2.0% | 665,883,445 | 20.6 | 2.7% | 665,1100,445 | 50.8 | 7.8% | 12 |
| 164 | PKN3 | 2425 | 226,446 | 28.4 | 3.8% | 666,884,446 | 27.0 | 6.9% | 666,1101,446 | 36.2 | 4.8% | 15 |
| 165 | PKN3 | 2682 | 227,447 | 30.0 | 4.6% | 667,885,447 | 27.1 | 2.8% | 667,1102,447 | 37.1 | 6.2% | 11 |
| 166 | PKN3 | 2683 | 228,448 | 22.4 | 2.8% | 668,886,448 | 34.8 | 2.2% | 668,1103,448 | 51.9 | 7.4% | 12 |
| 167 | PKN3 | 2931 | 229,449 | 35.1 | 4.4% | 669,887,449 | 57.3 | 7.8% | 669,1104,449 | 88.6 | 7.1% | 13 |
| 168 | PKN3 | 3063 | 230,450 | 21.8 | 3.1% | 670,888,450 | 28.6 | 8.5% | 670,1105,450 | 40.5 | 6.2% | 12 |
| 169 | PKN3 | 3314 | 231,451 | 9.7 | 1.8% | 671, 889, 451 | 12.0 | 1.4% | 671, 1106, 451 | 17.3 | 1.3% | 10 |
| 170 | PKN3 | 3315 | 232,452 | 10.1 | 1.3% | 672,890,452 | 15.3 | 2.8% | 672,1107,452 | 37.4 | 3.6% | 11 |
| 171 | RAF1 | 1509 | 233,453 | 46.2 | 9.4% | 673,891,453 | 51.3 | 10.7% | 673,1108,453 | 61.3 | 4.4% | 12 |
| 172 | RAF1 | 1512 | 234,454 | 40.1 | 9.7% | 674,892,454 | 34.5 | 5.6% | 674,1109,454 | 62.4 | 8.6% | 13 |
| 173 | RAF1 | 1628 | 235,455 | 48.3 | 7.9% | 675, 893, 455 | 47.4 | 7.1% | 675,1110,455 | 41.1 | 5.1% | 12 |
| 174 | RAF1 | 1645 | 236,456 | 38.9 | 2.3% | 676,894,456 | 62.1 | 9.0% | 676,1111,456 | 85.0 | 9.3% | 13 |
| 175 | RAF1 | 1780 | 237,457 | 22.6 | 4.9% | 677,895,457 | 24.8 | 5.3% | 677,1112,457 | 37.6 | 10.4% | 12 |
| 176 | RAF1 | 1799 | 238,458 | 23.2 | 3.1% | 678,896,458 | 43.6 | 7.6% | 678,1113,458 | 50.7 | 6.2% | 12 |
| 177 | RAF1 | 1807 | 239,459 | 28.0 | 5.4% | 679,897,459 | 34.8 | 5.8% | 679,1114,459 | 37.0 | 5.3% | 15 |
| 178 | RAF1 | 1863 | 240,460 | 28.2 | 3.1% | 680,898,460 | 38.1 | 4.5% | 680,1115,460 | 35.7 | 4.2% | 14 |
| 179 | RAF1 | 2157 | 241,461 | 68.8 | 6.5% | 681,899,461 | 64.1 | 8.0% | 681,1116,461 | 86.7 | 12.6% | 14 |
| 180 | RAF1 | 2252 | 242,462 | 11.4 | 1.7% | 682,900,462 | 25.8 | 5.4% | 682, 1117,462 | 71.2 | 10.7% | 13 |
| 181 | SRD5A1 | 1150 | 243,463 | 3.7 | 0.5% | 683,901,463 | 4.4 | 0.7% | 683,1118,463 | 3.8 | 0.4% | 12 |
| 182 | SRD5A1 | 1153 | 244,464 | 3.2 | 0.4% | 684,902,464 | 5.2 | 0.5% | 684,1119,464 | 7.0 | 0.9% | 12 |
| 183 | SRD5A1 | 1845 | 245,465 | 3.9 | 0.5% | 685,903,465 | 4.5 | 0.6% | 685,1120,465 | 7.4 | 0.8% | 13 |
| 184 | SRD5A1 | 1917 | 246,466 | 9.4 | 0.8% | 686,904,466 | 10.2 | 1.3% | 686,1121,466 | 22.0 | 2.8% | 12 |
| 185 | SRD5A1 | 1920 | 247,467 | 4.6 | 0.3% | 687,905,467 | 4.9 | 1.0% | 687,1122,467 | 6.4 | 0.5% | 11 |
| 186 | SRD5A1 | 1964 | 248,468 | 6.2 | 0.7% | 688,906,468 | 10.4 | 0.7% | 688,1123,468 | 21.0 | 4.6% | 10 |
| 187 | SRD5A1 | 1981 | 249,469 | 6.5 | 1.0% | 689,907,469 | 7.1 | 0.7% | 689,1124,469 | 8.8 | 1.5% | 12 |
| 188 | SRD5A1 | 2084 | 250,470 | 16.9 | 1.1% | 690,908,470 | 15.7 | 1.5% | 690,1125,470 | 13.3 | 1.5% | 12 |
| 189 | SRD5A1 | 2085 | 251,471 | 17.3 | 1.6% | 691, 909, 471 | 19.4 | 1.7% | 691, 1126, 471 | 20.8 | 2.6% | 12 |
| 190 | SRD5A1 | 2103 | 252,472 | 7.5 | 1.3% | 692,910,472 | 10.9 | 1.2% | 692,1127,472 | 12.3 | 1.7% | 12 |
| 191 | TNF | 32 | 253,473 | 71.4 | 13.2% | 693,911,473 | 93.7 | 14.9% | 693,1128,473 | 122.6 | 21.1% | 12 |
| 192 | TNF | 649 | 254,474 | 100.0 | 16.3% | 694,912,474 | 127.7 | 12.6% | 694,1129,474 | 147.9 | 21.7% | 12 |
| 193 | TNF | 802 | 255,475 | 67.2 | 10.7% | 695,913,475 | 64.0 | 6.6% | 695,1130,475 | 116.4 | 21.0% | 12 |
| 194 | TNF | 875 | 256,476 | 101.7 | 19.9% | 696,914,476 | 99.3 | 15.5% | 696,1131,476 | 108.8 | 14.2% | 12 |
| 195 | TNF | 983 | 257,477 | 94.5 | 7.0% | 697,915,477 | 83.1 | 7.3% | 697, 1132,477 | 140.6 | 20.4% | 11 |
| 196 | TNF | 987 | 258,478 | 82.0 | 10.9% | 698,916,478 | 139.4 | 8.2% | 698,1133,478 | 143.8 | 9.2% | 10 |
| 197 | TNF | 992 | 259,479 | 126.7 | 15.8% | 699,700,479 | 121.7 | 10.8% | 699,1134,479 | 115.9 | 16.4% | 11 |
| 198 | TNF | 1003 | 260,480 | 123.4 | 16.7% | 700,917,480 | 114.4 | 47.8% | 700, 1135,480 | 98.5 | 17.2% | 14 |
| 199 | TNF | 1630 | 261,481 | 58.0 | 5.7% | 701,918,481 | 56.1 | 9.4% | 701, 1136, 481 | 71.0 | 17.2% | 11 |
| 200 | TNF | 1631 | 262,482 | 54.2 | 13.4% | 702,919,482 | 63.9 | 10.1% | 702,1137,482 | 73.8 | 14.8% | 11 |
| 201 | TNFSF13B | 188 | 263,483 | 20.4 | 3.2% | 703,920,483 | 46.2 | 11.9% | 703, 1138,483 | 58.4 | 12.7% | 13 |
| 202 | TNFSF13B | 313 | 264,484 | 15.9 | 5.1% | 704,921,484 | 18.9 | 7.4% | 704,1139,484 | 48.0 | 8.1% | 12 |
| 203 | TNFSF13B | 337 | 265,485 | 22.3 | 4.6% | 705,922,485 | 37.1 | 11.0% | 705,1140,485 | 63.6 | 10.4% | 12 |
| 204 | TNFSF13B | 590 | 266,486 | 35.8 | 8.7% | 706,923,486 | 49.4 | 11.0% | 706, 1141,486 | 50.7 | 10.3% | 10 |
| 205 | TNFSF13B | 652 | 267,487 | 21.3 | 7.2% | 707,924,487 | 57.6 | 16.7% | 707,1142,487 | 78.8 | 5.6% | 14 |
| 206 | TNFSF13B | 661 | 268,488 | 28.8 | 3.0% | 708,925,488 | 38.3 | 8.4% | 708,1143,488 | 56.5 | 16.3% | 12 |
| 207 | TNFSF13B | 684 | 269,489 | 46.3 | 7.2% | 709,926,489 | 43.8 | 9.7% | 709,1144,489 | 54.5 | 4.6% | 12 |
| 208 | TNFSF13B | 905 | 270,490 | 18.5 | 5.0% | 710,927,490 | 27.9 | 3.1% | 710,1145,490 | 51.7 | 10.9% | 12 |
| 209 | TNFSF13B | 961 | 271,491 | 21.4 | 4.0% | 711,928,491 | 37.5 | 10.1% | 711, 1146, 491 | 77.6 | 11.2% | 14 |
| 210 | TNFSF13B | 1150 | 272,492 | 24.1 | 7.0% | 712,929,492 | 23.4 | 5.7% | 712,1147,492 | 35.9 | 8.0% | 13 |
| 211 | VEGFA | 1426 | 273,493 | 14.5 | 2.2% | 713,930,493 | 18.1 | 3.2% | 713,1148,493 | 21.0 | 3.8% | 13 |
| 212 | VEGFA | 1428 | 274,494 | 18.5 | 2.6% | 714,931,494 | 32.1 | 5.8% | 714,1149,494 | 46.7 | 9.4% | 12 |
| 213 | VEGFA | 1603 | 275,495 | 14.6 | 2.1% | 715,932,495 | 36.6 | 17.5% | 715, 1150,495 | 65.6 | 6.9% | 13 |
| 214 | VEGFA | 1685 | 276,496 | 17.1 | 1.3% | 716,933,496 | 20.2 | 5.5% | 716, 1151, 496 | 23.4 | 3.8% | 13 |
| 215 | VEGFA | 1792 | 277,497 | 17.0 | 1.8% | 717,934,497 | 21.2 | 3.2% | 717,1152,497 | 39.5 | 6.3% | 12 |
| 216 | VEGFA | 2100 | 278,498 | 116.9 | 11.5% | 718,935,498 | 103.6 | 7.5% | 718,1153,498 | 101.5 | 12.9% | 12 |
| 217 | VEGFA | 2102 | 279,499 | 116.3 | 9.1% | 719,936,499 | 110.2 | 9.3% | 719,1154,499 | 105.0 | 8.0% | 12 |
| 218 | VEGFA | 2196 | 280,500 | 24.2 | 2.7% | 720,937,500 | 26.6 | 3.1% | 720,1155,500 | 43.5 | 3.5% | 12 |
| 219 | VEGFA | 2261 | 281,501 | 15.6 | 2.2% | 721,938,501 | 44.2 | 6.2% | 721,1156,501 | 109.0 | 9.8% | 12 |
| 220 | VEGFA | 2292 | 282,502 | 48.4 | 4.3% | 722,939,502 | 45.1 | 7.2% | 722,1157,502 | 80.7 | 6.7% | 15 |

| | | | | | | | | | | | | |
|---|---|---|---|---|---|---|---|---|---|---|---|---|
| * All samples were normalized to the respective dsRNA QNeg (Qiagen) negative control samples run in the same experiment. That is, QNeg values were set as 100% active (*i.e*., no knockdown), with 95% confidence intervals (CI) ranging from 6.3-22.5%. As a positive control, an siRNA specific for rLuc was used, which samples showed on average expression levels that varied from 1.2% to 16.8% (*i.e.*, about 83% to about 99% knockdown activity and a 95% CI ranging from 0.3% to 13.7%). **†** "Pos" refers to the position on the target gene mRNA message that aligns with the 5'-end of the dsRNA sense strand. The mRNA numbering is based on the GenBank accession numbers as described herein. ‡ The SEQ ID NOS. are provided in the following order: (1) Dicer: sense strand, antisense strand; (2) Nicked: 5'-sense strand fragment, 3'-sense strand fragment, and antisense strand; and (3) Gapped: 5'-sense strand fragment, 3'-sense strand fragment, and antisense strand. The Dicer dsRNA has two strands, while ndsRNA and gdsRNA have three strands each. The nicked or gapped sense strand fragments have three locked nucleic acids each. ^ "Length 5'-S" refers to the length of the 5'-sense strand fragment of the nicked or gapped mdRNA, which indicates the position of the nick (e.g., 10 means the nick is between position 10 and 11, so the 5'sense strand fragment is 10 nucleotides long and the 3'-sense strand fragment is 15 nucelotides long) or one nucleotide gap (e.g., 10 means the missing nucleotide is number 11, so the 5'sense strand fragment is 10 nucleotides long and the 3'-sense strand fragment is 14 nucelotides long). | | | | | | | | | | | | |

### EXAMPLE 2

### KNOCKDOWN OF β-GALACTOSIDASE ACTIVITY BY GAPPED DSRNA DICER SUBSTRATE

The activity of a Dicer substrate dsRNA containing a gap in the double-stranded structure in silencing LacZ mRNA as compared to the normal Dicer substrate dsRNA *(i.e.,* not having a gap) was examined.

### Nucleotide Sequences of dsRNA and mdRNA Targeting LacZ mRNA

The nucleic acid sequence of the one or more sense strands, and the antisense strand of the dsRNA and gapped dsRNA (also referred to herein as a meroduplex or mdRNA) are shown below and were synthesized using standard techniques. The RISC activator LacZ dsRNA comprises a 21 nucleotide sense strand and a 21 nucleotide antisense strand, which can anneal to form a double-stranded region of 19 base pairs with a two deoxythymidine overhang on each strand (referred to as 21/21 dsRNA).
LacZ dsRNA (21/21) ― RISC Activator
   Sense 5'-CUACACAAAUCAGCGAUUUdTdT-3' (SEQ ID NO:1)
   Antisense 3'-dTdTGAUGUGUUUAGUCGCUAAA-5' (SEQ ID NO:2)

The Dicer substrate LacZ dsRNA comprises a 25 nucleotide sense strand and a 27 nucleotide antisense strand, which can anneal to form a double-stranded region of 25 base pairs with one blunt end and a cytidine and uridine overhang on the other end (referred to as 25/27 dsRNA).
LacZ dsRNA (25/27) ― Dicer Substrate
   Sense 5'-CUACACAAAUCAGCGAUUUCCAUdGdT-3' (SEQ ID NO:3)
   Antisense 3'-CU GAUGUGUUUAGUCGCUAAAGGUA C A - 5' (SEQ ID NO:4)
The LacZ mdRNA comprises two sense strands of 13 nucleotides (5'-portion) and 11 nucleotides (3'-portion) and a 27 nucleotide antisense strand, which three strands can anneal to form two double-stranded regions of 13 and 11 base pairs separated by a single nucleotide gap (referred to as a 13, 11/27 mdRNA). The 5'-end of the 11 nucleotide sense strand fragment may be optionally phosphorylated. The "*" indicates a gap ― in this case, a single nucleotide gap (e.g., a cytidine is missing).
LacZ mdRNA (13, 11/27) ― Dicer Substrate
   Sense 5'-CUACACAAAUCAG*GAUUUCCAUdGdT-3' (SEQ ID NOS:5, 6)
   Antisense 3'-CUGAUGUGUUUAGUCGCUAAAGGUA C A - 5' (SEQ ID NO:4)
Each of the LacZ dsRNA or mdRNA was used to transfect 9lacZ/R cells.

### Transfection

Six well collagen-coated plates were seeded with 5 x 10⁵ 9lacZ/R cells/well in a 2 ml volume per well, and incubated overnight at 37°C / 5% CO₂ in DMEM/high glucose media. Preparation for transfection: 250 µl of OPTIMEM media without serum was mixed with 5 µl of 20 pmol/µl dsRNA and 5µl of HIPERFECT transfection solution (Qiagen) was mixed with another 250 µl OPTIMEM media. After both mixtures were allowed to equilibrate for 5 minutes, the RNA and transfection solutions were combined and left at room temperature for 20 minutes to form transfection complexes. The final concentration of HIPERFECT was 50 µM, and the dsRNAs were tested at 0.05nM, 0.1nM, 0.2nM, 0.5nM, 1nM, 2nM, 5nM, and 10nM, while the mdRNA was tested at 0.2nM, 0.5nM, 1nM, 2nM, 5nM, 10nM, 20nM, and 50nM. Complete media was removed, the cells were washed with incomplete OPTIMEM, and then 500 µl transfection mixture was applied to the cells, which were incubated with gentle shaking at 37°C for 4 hours. After transfecting, the transfection media was removed, cells were washed once with complete DMEM/high glucose media, fresh media added, and the cells were then incubated for 48 hours at 37°C, 5% CO₂.

### ß-Galactosidase Assay

Transfected cells were washed with PBS, and then detached with 0.5 ml trypsin/EDTA. The detached cells were suspended in 1 ml complete DMEM/high glucose and transferred to a clean tube. The cells were harvested by centrifugation at 250 x g for 5 minutes, and then resuspended in 50 µl 1x lysis buffer at 4°C. The lysed cells were subjected to two freeze-thaw cycles on dry ice and a 37°C water bath. The lysed samples were centrifuged for 5 minutes at 4°C and the supernatant was recovered. For each sample, 1.5 µl and 10 µl of lysate was transferred to a clean tube and sterile water added to a final volume of 30 µl followed by the addition of 70 µl *o*-nitrophenyl-β-*D*-galactopyranose (ONPG) and 200 µl 1x cleavage buffer with ß-mercaptoethanol. The samples were mixed briefly, incubated for 30 minutes at 37°C, and then 500 µl stop buffer was added (final volume 800 µl). ß-Galactosidase activity for each sample was measured in disposable cuvettes at 420 nm. Protein concentration was determined by the BCA (bicinchoninic acid) method. For the purpose of the instant example, the level of measured LacZ activity was correlated with the quantity of LacZ transcript within 9L/LacZ cells. Thus, a reduction in ß-galactosidase activity after dsRNA transfection, absent a negative impact on cell viability, was attributed to a reduction in the quantity of LacZ transcripts resulting from targeted degradation mediated by the LacZ dsRNA.

### Results

Knockdown activity in transfected and untransfected cells was normalized to a Qneg control dsRNA and presented as a normalized value of the Qneg control (*i.e*., Qneg represented 100% or "normal" gene expression levels). Both the lacZ RISC activator and Dicer substrate dsRNAs molecule showed good knockdown of ß-galactosidase activity at concentration as low as 0.1 nM (Figure 2), while the Dicer substrate antisense strand alone (single stranded 27mer) had no silencing effect. Surprisingly, a gapped mdRNA showed good knockdown although somewhat lower than that of intact RISC activator and Dicer substrate dsRNAs (Figure 2). The presence of the gapmer cytidine (*i.e.,* the missing nucleotide) at various concentrations (0.1 µM to 50 µM) had no effect on the activity of the mdRNA (data not shown). None of the dsRNA or mdRNA solutions showed any detectable toxicity in the transfected 9L/LacZ cells. The IC₅₀ of the lacZ mdRNA was calculated to be 3.74 nM, which is about 10 fold lower than what had been previously measured for lacZ dsRNA 21/21 (data not shown). These results show that a meroduplex (gapped dsRNA) is capable of inducing gene silencing.

### EXAMPLE 3

### KNOCKDOWN OF INFLUENZA GENE EXPRESSION BY NICKED DSRNA

The activity of a nicked dsRNA (21/21) in silencing influenza gene expression as compared to a normal dsRNA *(i.e.,* not having a nick) was examined.

### Nucleotide Sequences of dsRNA and mdRNA Targeting Influenza mRNA

The dsRNA and nicked dsRNA (another form of meroduplex, referred to herein as ndsRNA) are shown below and were synthesized using standard techniques. The RISC activator influenza G1498 dsRNA comprises a 21 nucleotide sense strand and a 21 nucleotide antisense strand, which can anneal to form a double-stranded region of 19 base pairs with a two deoxythymidine overhang on each strand.
G1498-wt dsRNA (21/21)
   Sense 5'-GGAUCUUAUUUCUUCGGAGdTdT-3' (SEQ ID NO:7)
   Antisense 3'-dTdTCCUAGAAUAAAGAAGCCUC-5' (SEQ ID NO:8)

The RISC activator influenza G1498 dsRNA was nicked on the sense strand after nucleotide 11 to produce a ndsRNA having two sense strands of 11 nucleotides (5'-portion, italic) and 10 nucleotides (3'-portion) and a 21 nucleotide antisense strand, which three strands can anneal to form two double-stranded regions of 11 (shown in italics) and 10 base pairs separated by a one nucleotide gap (which may be referred to as G1498 11, 10/21 ndsRNA-wt). The 5'-end of the 10 nucleotide sense strand fragment may be optionally phosphorylated, as depicted by a "p" preceding the nucleotide (*e.g*., pC).
G1498 ndsRNA-wt (11, 10/21)
   Sense 5'-*GGAUCUUAUUUCUU*CGGAGdTdT-3' (SEQ ID NO:9, 10)
   Antisense 3'-dTdTCCUAGAAUAAAGAAGCCUC-5' (SEQ ID NO:8)
G1498 ndsRNA-wt (11, 10/21)
   Sense 5'- *GGAUCUUAUUU*qCUUCGGAGdTdT-3' (SEQ ID NOS:9, 10)
   Antisense 3'-dTdTCCUAGAAUAAAGAAGCCUC-5' (SEQ ID NO:8)
In addition, each of these G1498 dsRNAs were made with each U substituted with a 5-methyluridine (ribothymidine) and are referred to as G 1498 dsRNA-rT. Each of the G 1498 dsRNA or ndsRNA (meroduplex), with or without the 5-methyluridine substitution, was used to transfect HeLa S3 cells having an influenza target sequence associated with a luciferase gene. Also, the G 1498 antisense strand alone or the antisense strand annealed to the 11 nucleotide sense strand portion alone or the 10 nucleotide sense strand portion alone were examined for activity.

### Transfection and Dual Luciferase Assay

The reporter plasmid psiCHECK™-2 (Promega, Madison, WI), which constitutively expresses both firefly *luc2* (*Photinus pyralis*) and *Renilla* (*Renilla reniformis,* also known as sea pansy) luciferases, was used to clone in a portion of the influenza NP gene downstream of the *Renilla* translational stop codon that results in a *Renilla*-influenza NP fusion mRNA. The firefly luciferase in the psiCHECK™-2 vector is used to normalize Renilla luciferase expression and serves as a control for transfection efficiency.

Multi-well plates were seeded with HeLa S3 cells/well in 100 µl Ham's F12 medium and 10% fetal bovine serum, and incubated overnight at 37°C / 5% CO₂. The HeLa S3 cells were transfected with the psiCHECK™-influenza plasmid (75 ng) and G1498 dsRNA or ndsRNA (final concentration of 10 nM or 100 nM) formulated in Lipofectamine™ 2000 and OPTIMEM reduced serum medium. The transfection mixture was incubated with the HeLa S3 cells with gentle shaking at 37°C for about 18 to 20 hours.

After transfecting, firefly luciferase reporter activity was measured first by adding Dual-Glo™ Luciferase Reagent (Promega, Madison, WI) for 10 minutes with shaking, and then quantitating the luminescent signal using a VICTOR³™ 1420 Multilabel Counter (PerkinElmer, Waltham, MA). After measuring the firefly luminescence, Stop & Glo^{®} Reagent (Promega, Madison, WI) was added for 10 minutes with shaking to simultaneously quench the firefly reaction and initiate the *Renilla* luciferase reaction, and then the *Renilla* luciferase luminescent signal was quantitated VICTOR³™ 1420 Multilabel Counter (PerkinElmer, Waltham, MA).

### Results

Knockdown activity in transfected and untransfected cells was normalized to a Qneg control dsRNA and presented as a normalized value of the Qneg control (*i.e.,* Qneg represented 100% or "normal" gene expression levels). Thus, a smaller value indicates a greater knockdown effect. The G1498 dsRNA-wt and dsRNA-rT showed similar good knockdown at a 100 nM concentration (Figure 3). Surprisingly, the G1498 ndsRNA-rT, whether phosphorylated or not, showed good knockdown although somewhat lower than the G1498 dsRNA-wt (Figure 3). Similar results were obtained with dsRNA or ndsRNA at 10 nM (data not shown). None of the G1498 dsRNA or ndsRNA solutions showed any detectable toxicity in HeLa S3 cells at either 10 nM or 100 nM. Even the presence of only half a nicked sense strand (an 11 nucleotide or 10 nucleotide strand alone) with a G1498 antisense strand showed some detectable activity. These results show that a nicked-type meroduplex dsRNA molecule is unexpectedly capable of promoting gene silencing.

### EXAMPLE 4

### KNOCKDOWN ACTIVITY OF NICKED MDRNA

In this example, the activity of a dicer substrate LacZ dsRNA of Example 1 having a sense strand with a nick at various positions was examined. In addition, a dideoxy nucleotide (e.g., ddG) was incorporated at the 5'-end of the 3'-most strand of a sense sequence having a nick or a single nucleotide gap to determine whether the *in vivo* ligation of the nicked sense strand is "rescuing" activity. The ddG is not a substrate for ligation. Also examined was the influenza dicer substrate dsRNA of Example 7 having a sense strand with a nick at one of positions 8 to 14. The "p" designation indicates that the 5'-end of the 3'-most strand of the nicked sense influenza sequence was phosphorylated. The "L" designation indicates that the G at position 2 of the 5'-most strand of the nicked sense influenza sequence was substituted for a locked nucleic acid G. The Qneg is a negative control dsRNA.

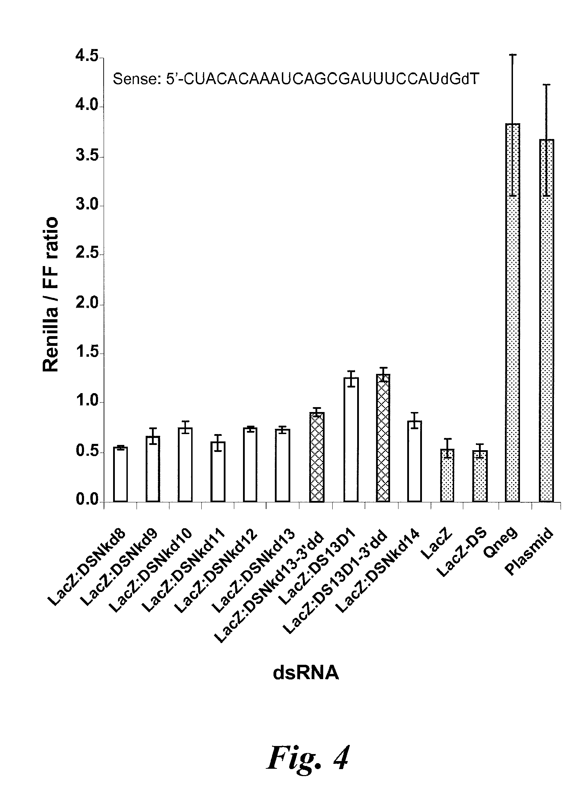

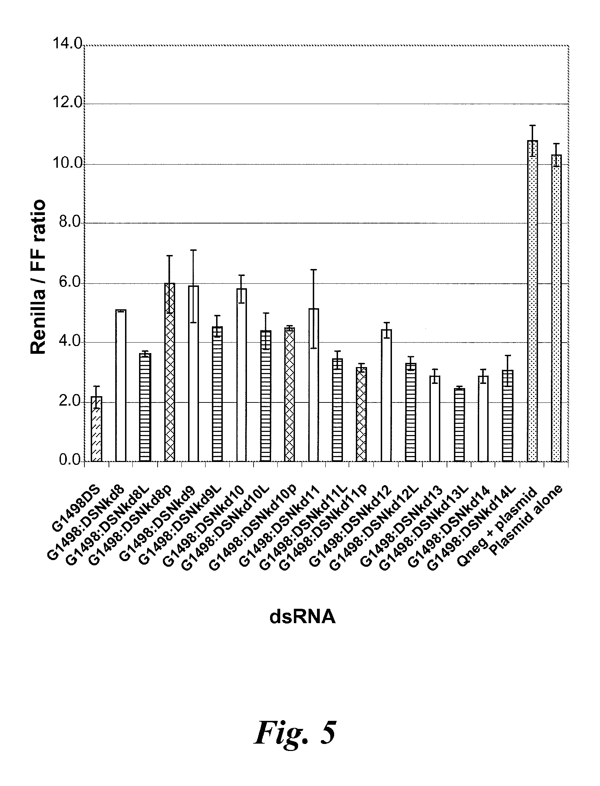

The dual fluorescence assay of Example 3 was used to measure knockdown activity with 5 nM of the LacZ sequences and 0.5 nM of the influenza sequences. The lacZ dicer substrate (25/27, LacZ-DS) and lacZ RISC activator (21/21, LacZ) are equally active, and the LacZ-DS can be nicked in any position between 8 and 14 without affecting activity (Figure 3). In addition, the inclusion of a ddG on the 5'-end of the 3'-most LacZ sense sequence having a nick (LacZ:DSNkd13-3'dd) or a one nucleotide gap (LacZ:DSNkd13D1-3'dd) was essentially as active as the unsubstituted sequence (Figure 4). The influenza dicer substrate (G1498DS) nicked at any one of positions 8 to 14 was also highly active (Figure 5). Phosphorylation of the 5'-end of the 3'-most strand of the nicked sense influenza sequence had essentially no effect on activity, but addition of a locked nucleic acid appears to improve activity.

### EXAMPLE 5

### MEAN INHIBITORY CONCENTRATION OF MDRNA

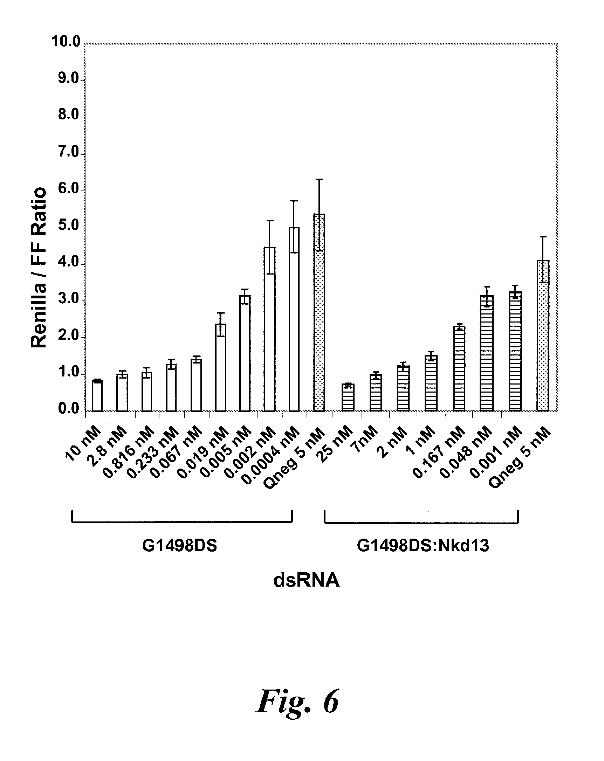

In this example, a dose response assay was performed to measure the mean inhibitory concentration (IC₅₀) of the influenza dicer substrate dsRNA of Example 8 having a sense strand with a nick at position 12, 13, or 14, including or not a locked nucleic acid. The dual luciferase assay of Example 2 was used. The influenza dicer substrate dsRNA (G1498DS) was tested at 0.0004 nM, 0.002 nM, 0.005 nM, 0.019 nM, 0.067 nM, 0.233 nM, 0.816 nM, 2.8 nM, and 10nM, while the mdRNA with a nick at position 13 (G1498DS:Nkd13) was tested at 0.001 nM, 0.048 nM, 0.167 nM, 1 nM, 2 nM, 7 nM, and 25 nM (*see* Figure 6). Also tested were RISC activator molecules (21/21) with or without a nick at various positions (including G1498DS:Nkd11, G1498DS:Nkd12, and G1498DS:Nkd14), each of the nicked versions with a locked nucleic acid as described above (data not shown). The Qneg is a negative control dsRNA.

The IC₅₀ of the RISC activator G1498 was calculated to be about 22 pM, while the dicer substrate G1498DS IC₅₀ was calculated to be about 6 pM. The IC₅₀ of RISC and Dicer mdRNAs range from about 200 pM to about 15 nM. The inclusion of a single locked nucleic acid reduced the IC₅₀ of Dicer mdRNAs by up 4 fold (data not shown). These results show that a meroduplex dsRNA having a nick or gap in any position is capable of inducing gene silencing.

### EXAMPLE 6

### KNOCKDOWN ACTIVITY OF GAPPED MDRNA

The activity of an influenza dicer substrate dsRNA having a sense strand with a gap of differing sizes and positions was examined. The influenza dicer substrate dsRNA of Example 8 was generated with a sense strand having a gap of 0 to 6 nucleotides at position 8, a gap of 4 nucleotides at position 9, a gap of 3 nucleotides at position 10, a gap of 2 nucleotides at position 11, and a gap of 1 nucleotide at position 12 *(see* Table 2). The Qneg is a negative control dsRNA. Each of the mdRNAs was tested at a concentration of 5 nM (data not shown) and 10 nM. The mdRNAs have the following antisense strand 5'-CAUUGUCUCCGAAGAAAUAAGAUCCUU (SEQ ID NO:11), and nicked or gapped sense strands as shown in Table 2.

**Table 2.**

| mdRNA | 5' Sense* (SEQ ID NO.) | 3' Sense (SEQ ID NO.) | Gap Pos | Gap Size | % KD^{†} |
|---|---|---|---|---|---|
| G1498:DSNkd8 | G**G**AUCUUA (12) | UUUCUUCGGAGACAAdTdG (13) | 8 | 0 | 67.8 |
| G1498:DSNkd8D1 | G**G**AUCUUA (12) | UUCUUCGGAGACAAdTdG (14) | 8 | 1 | 60.9 |
| G1498:DSNkd8D2 | G**G**AUCUUA (12) | UCUUCGGAGACAAdTdG (15) | 8 | 2 | 48.2 |
| G1498:DSNkd8D3 | G**G**AUCUUA (12) | CUUCGGAGACAAdTdG (16) | 8 | 3 | 44.1 |
| G1498:DSNkd8D4 | G**G**AUCUUA (12) | UUCGGAGACAAdTdG (17) | 8 | 4 | 30.8 |
| G1498:DSNkd8D5 | G**G**AUCUUA (12) | UCGGAGACAAdTdG (18) | 8 | 5 | 10.8 |
| G1498:DSNkd8D6 | G**G**AUCUUA (12) | CGGAGACAAdTdG (19) | 8 | 6 | 17.9 |
| G1498:DSNkd9D4 | G**G**AUCUUAU (20) | UCGGAGACAAdTdG (18) | 9 | 4 | 38.9 |
| G1498:DSNkd10D3 | G**G**AUCUUAUU (21) | UCGGAGACAAdTdG (18) | 10 | 3 | 38.4 |
| G1498:DSNkd11D2 | G**G**AUCUUAUUU (22) | UCGGAGACAAdTdG (18) | 11 | 2 | 46.2 |
| G1498:DSNkd12D1 | G**G**AUCUUAUUUC (23) | UCGGAGACAAdTdG (18) | 12 | 1 | 49.6 |
| Plasmid | - | - | - | - | 5.3 |

| | | | | | |
|---|---|---|---|---|---|
| * **G** indicates a locked nucleic acid G in the 5' sense strand. ^{†} % KD means percent knockdown activity. | | | | | |

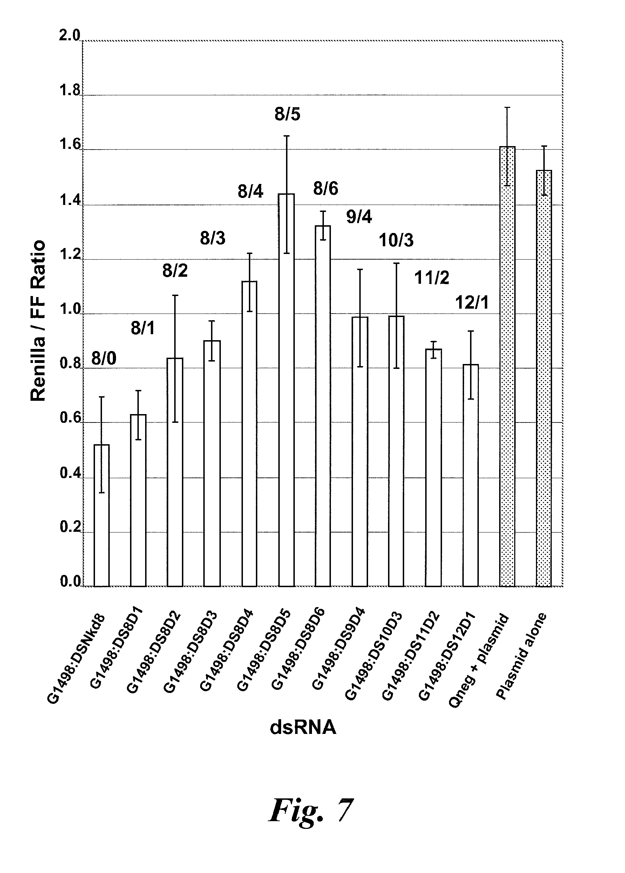

The dual fluorescence assay of Example 2 was used to measure knockdown activity. Similar results were obtained at both the 5 nM and 10 nM concentrations. These data show that an mdRNA having a gap of up to 6 nucleotides still has activity, although having four or fewer missing nucleotides shows the best activity (*see, also,* Figure 7). Thus, mdRNA having various sizes gaps that are in various different positions have knockdown activity.

To examine the general applicability of a sequence having a sense strand with a gap of differing sizes and positions, a different dsRNA sequence was tested. The lacZ RISC dsRNA of Example 1 was generated with a sense strand having a gap of 0 to 6 nucleotides at position 8, a gap of 5 nucleotides at position 9, a gap of 4 nucleotides at position 10, a gap of 3 nucleotides at position 11, a gap of 2 nucleotides at position 12, a gap of 1 nucleotide at position 12, and a nick (gap of 0) at position 14 *(see* Table 3). The Qneg is a negative control dsRNA. Each of the mdRNAs was tested at a concentration of 5 nM (data not shown) and 25 nM. The lacZ mdRNAs have the following antisense strand 5'-AAAUCGCUGAUUUGUGUAGdTdTUAAA (SEQ ID NO:2) and nicked or gapped sense strands as shown in Table 3.

**Table 3.**

| mdRNA | 5' Sense* (SEQ ID NO.) | 3' Sense* (SEQ ID NO.) | Gap Pos | Gap Size |
|---|---|---|---|---|
| LacZ:Nkd8 | CU**A**CACAA (24) | AUCAGCG**A**UUUdTdT (25) | 8 | 0 |
| LacZ:Nkd8D1 | CU**A**CACAA (24) | UCAGCG**A**UUUdTdT (26) | 8 | 1 |
| LacZ:Nkd8D2 | CU**A**CACAA (24) | CAGCG**A**UUUdTdT (27) | 8 | 2 |
| LacZ:Nkd8D3 | CU**A**CACAA (24) | AGCG**A**UUUdTdT (2 8) | 8 | 3 |
| LacZ:Nkd8D4 | CU**A**CACAA (24) | GCG**A**UUUdTdT (29) | 8 | 4 |
| LacZ:Nkd8D5 | CU**A**CACAA (24) | CG**A**UUUdTdT (30) | 8 | 5 |
| LacZ:Nkd8D6 | CU**A**CACAA (24) | G**A**UUUdTdT (31) | 8 | 6 |
| LacZ:Nkd9D5 | CU**A**CACAAA (32) | G**A**UUUdTdT (31) | 9 | 5 |
| LacZ:Nkd10D4 | CU**A**CACAAAU (33) | G**A**UUUdTdT (31) | 10 | 4 |
| LacZ:Nkd11D3 | CU**A**CACAAAUC (34) | G**A**UUUdTdT (31) | 11 | 3 |
| LacZ:Nkd12D2 | CU**A**CACAAAUCA (35) | G**A**UUUdTdT (31) | 12 | 2 |
| LacZ:Nkd13D1 | CU**A**CACAAAUCAG (36) | G**A**UUUdTdT (31) | 13 | 1 |
| LacZ:Nkd14 | CU**A**CACAAAUCAGC (37) | G**A**UUUdTdT (31) | 14 | 0 |

| | | | | |
|---|---|---|---|---|
| * **A** indicates a locked nucleic acid A in each sense strand. | | | | |

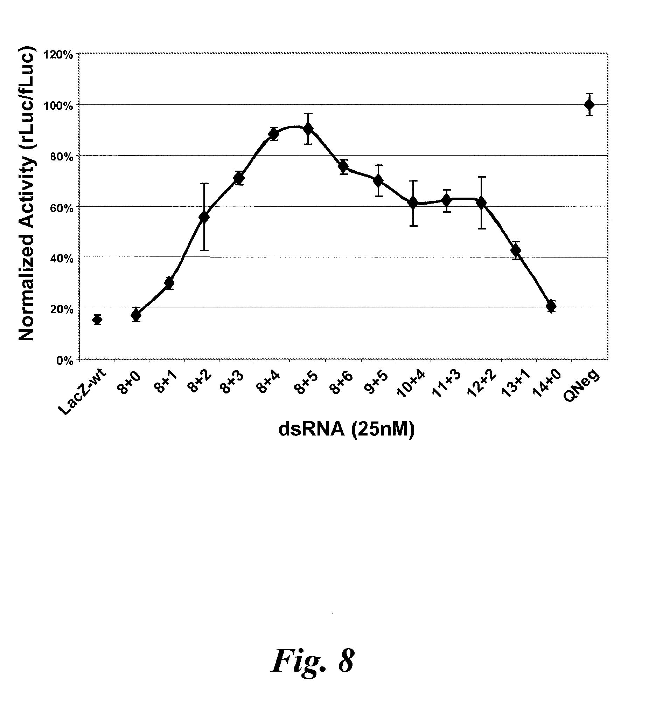

The dual fluorescence assay of Example 3 was used to measure knockdown activity. Figure 8 shows that an mdRNA having a gap of up to 6 nucleotides has substantial activity and the position of the gap may affect the potency of knockdown. Thus, mdRNA having various sizes gaps that are in various different positions and in different mdRNA sequences have knockdown activity.

### EXAMPLE 7

### KNOCKDOWN ACTIVITY OF SUBSTITUTED MDRNA

The activity of an influenza dsRNA RISC sequences having a nicked sense strand and the sense strands having locked nucleic acid substitutions were examined. The influenza RISC sequence G1498 of Example 3 was generated with a sense strand having a nick at positions 8 to 14 counting from the 5'-end. Each sense strand was substituted with one or two locked nucleic acids as shown in Table 4. The Qneg and Plasmid are negative controls. Each of the mdRNAs was tested at a concentration of 5 nM. The antisense strand used was 5'-CUCCGAAGAAAUAAGAUCCdTdT (SEQ ID NO:8).

**Table 4.**

| mdRNA | 5' Sense* (SEQ ID NO.) | 3' Sense* (SEQ ID NO.) | Nick Pos | % KD |
|---|---|---|---|---|
| G1498-wt | GGAUCUUAUUUCUUCGGAGdTdT (7) | - | - | 85.8 |
| G1498-L | G**G**AUCUUAUUUCUUC**G**GAGdTdT (61) | - | - | 86.8 |
| G1498:Nkd8-1 | G**G**AUCUUA (12) | UUUCUUC**G**GAGdTdT (47) | 8 | 36.0 |
| G1498:Nkd8-2 | G**G**AUCUU**A** (40) | **U**UUCUUC**G**GAGdTdT (54) | 8 | 66.2 |
| G1498:Nkd9-1 | G**G**AUCUUAU (20) | UUCUUC**G**GAGdTdT (48) | 9 | 60.9 |
| G1498:Nkd9-2 | G**G**AUCUUA**U** (41) | **U**UCUUC**G**GAGdTdT (55) | 9 | 64.4 |
| G1498:Nkd10-1 | G**G**AUCUUAUU (21) | UCUUC**G**GAGdTdT (49) | 10 | 58.2 |
| G1498:Nkd10-2 | G**G**AUCUUAU**U** (42) | **U**CUUC**G**GAGdTdT (56) | 10 | 68.5 |
| G1498:Nkd11-1 | G**G**AUCUUAUUU (22) | CUUC**G**GAGdTdT (50) | 11 | 75.9 |
| G1498:Nkd11-2 | G**G**AUCUUAUU**U** (43) | **C**UUC**G**GAGdTdT (57) | 11 | 67.1 |
| G1498:Nkd12-1 | G**G**AUCUUAUUUC (23) | UUC**G**GAGdTdT (51) | 12 | 59.9 |
| G1498:Nkd12-2 | G**G**AUCUUAUUU**C** (44) | **U**UC**G**GAGdTdT (58) | 12 | 72.8 |
| G1498:Nkd13-1 | G**G**AUCUUAUUUCU (38) | UC**G**GAGdTdT (52) | 13 | 37.1 |
| G1498:Nkd13-2 | G**G**AUCUUAUUUC**U** (45) | **U**C**G**GAGdTdT (59) | 13 | 74.3 |
| G1498:Nkd14-1 | G**G**AUCUUAUUUCUU (39) | C**G**GAGdTdT (53) | 14 | 29.0 |
| G1498:Nkd14-2 | G**G**AUCUUAUUUCU**U** (46) | **CG**GAGdTdT (60) | 14 | 60.2 |
| Qneg | - | - | - | 0 |
| Plasmid | - | - | - | 3.6 |

| | | | | |
|---|---|---|---|---|
| * Nucleotides that are bold and underlined are locked nucleic acids. | | | | |

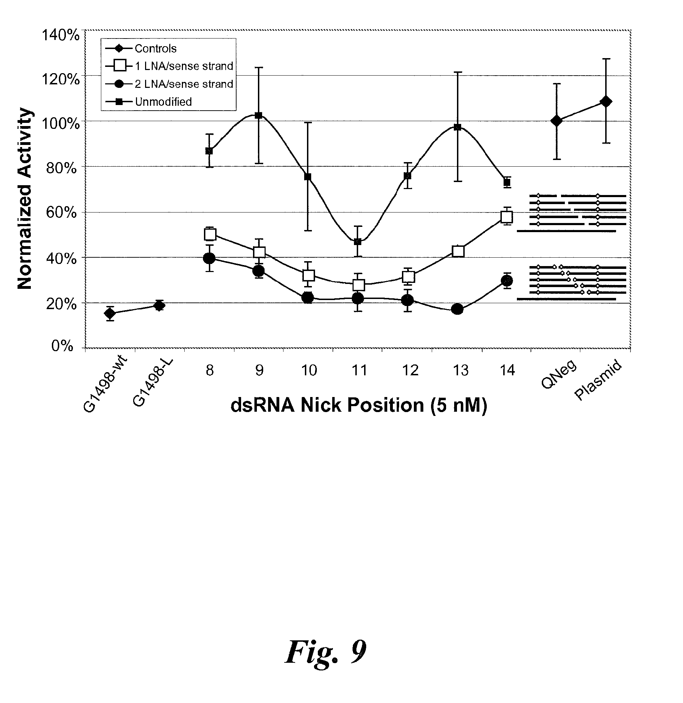

The dual fluorescence assay of Example 3 was used to measure knockdown activity. These data show that increasing the number of locked nucleic acid substitutions tends to increase activity of an mdRNA having a nick at any of a number of positions. The single locked nucleic acid per sense strand appears to be most active when the nick is at position 11 (*see* Figure 9). But, multiple locked nucleic acids on each sense strand make mdRNA having a nick at any position as active as the most optimal nick position with a single substitution (*i.e*., position 11) (Figure 9). Thus, mdRNA having duplex stabilizing modifications make mdRNA essentially equally active regardless of the nick position.

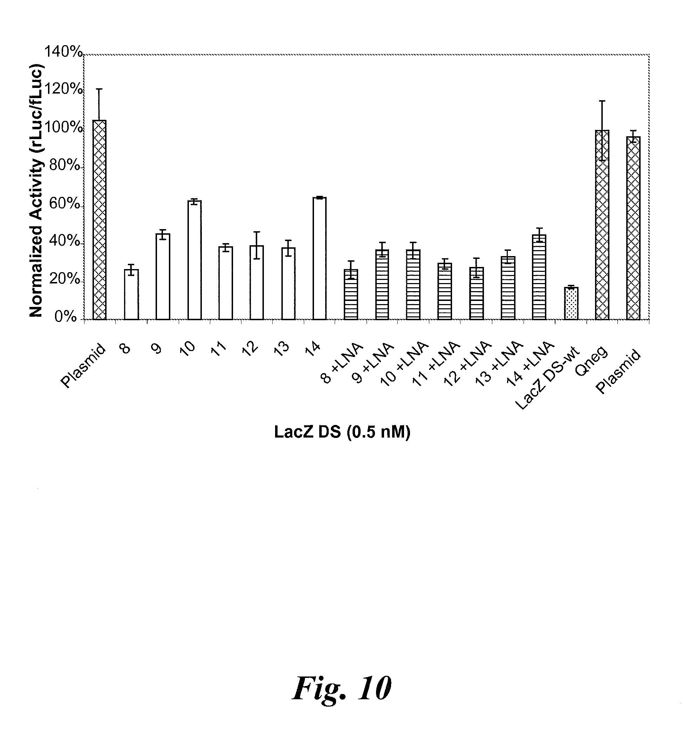

Similar results were observed when locked nucleic acid substitutions were made in the LacZ dicer substrate mdRNA of Example 2 (SEQ ID NOS:3 and 4). The lacZ dicer was nicked at positions 8 to 14, and a duplicate set of nicked LacZ dicer molecules were made with the exception that the A at position 3 (from the 5'-end) of the 5' sense strand was substituted for a locked nucleic acid A (LNA-A). As is evident from Figure 10, most of the nicked lacZ dicer molecules containing LNA-A were as potent in knockdown activity as the unsubstituted lacZ dicer.

### EXAMPLE 7

### MDRNA KNOCKDOWN OF INFLUENZA VIRUS TITER

The activity of a dicer substrate nicked dsRNA in reducing influenza virus titer as compared to a wild-type dsRNA (*i.e.*, not having a nick) was examined. The influenza dicer substrate sequence (25/27) is as follows:
Sense 5'-GGAUCUUAUUUCUUCGGAGACAAdTdG (SEQ ID NO:62)
Antisense 5'-CAUUGUCUCCGAAGAAAUAAGAUCCUU (SEQ ID NO:11)
The mdRNA sequences have a nicked sense strand after position 12, 13, and 14, respectively, as counted from the 5'-end, and the G at position 2 is substituted with locked nucleic acid G.

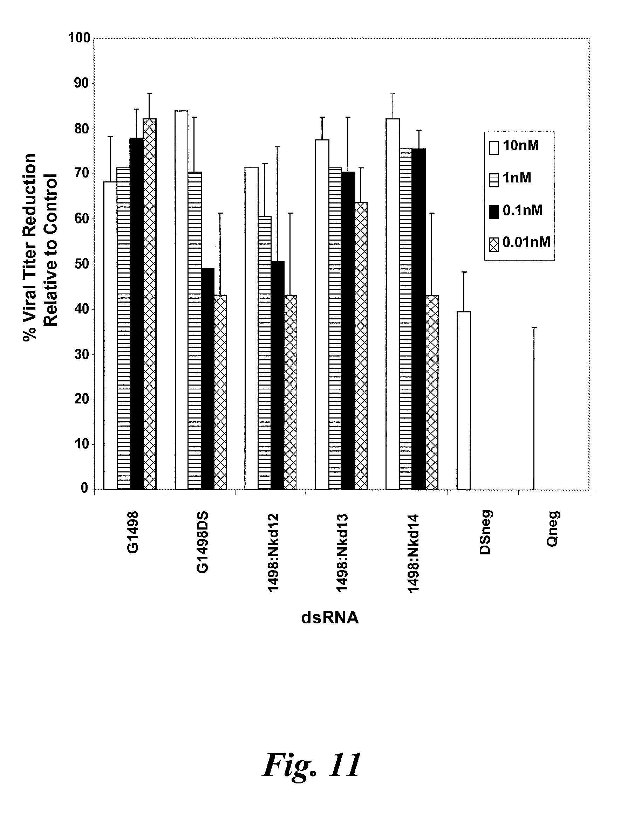

For the viral infectivity assay, Vero cells were seeded at 6.5 x 10⁴ cells/well the day before transfection in 500 µl 10% FBS/DMEM media per well. Samples of 100, 10, 1, 0.1, and 0.01 nM stock of each dsRNA were complexed with 1.0 µl (1 mg/ml stock) of Lipofectamine™ 2000 (Invitrogen, Carlsbad, CA) and incubated for 20 minutes at room temperature in 150 µl OPTIMEM (total volume) (Gibco, Carlsbad, CA). Vero cells were washed with OPTIMEM, and 150 µl of the transfection complex in OPTIMEM was then added to each well containing 150 µl of OPTIMEM media. Triplicate wells were tested for each condition. An additional control well with no transfection condition was prepared. Three hours post transfection, the media was removed. Each well was washed once with 200 µl PBS containing 0.3% BSA and 10 mM HEPES/PS. Cells in each well were infected with WSN strain of influenza virus at an MOI 0.01 in 200 µl of infection media containing 0.3% BSA/10 mM HEPES/PS and 4 µg/ml trypsin. The plate was incubated for 1 hour at 37°C. Unadsorbed virus was washed off with the 200 µl of infection media and discarded, then 400 µl DMEM containing 0.3% BSA/10 mM HEPES/PS and 4 µg/ml trypsin was added to each well. The plate was incubated at 37°C, 5% CO₂ for 48 hours, then 50 µl supernatant from each well was tested in duplicate by TCID₅₀ assays (50% Tissue-Culture Infective Dose, WHO protocol) in MDCK cells and titers were estimated using the Spearman and Karber formula. The results show that these mdRNAs show about a 50% to 60% viral titer knockdown, even at a concentration as low as 10 pM (Figure 11).

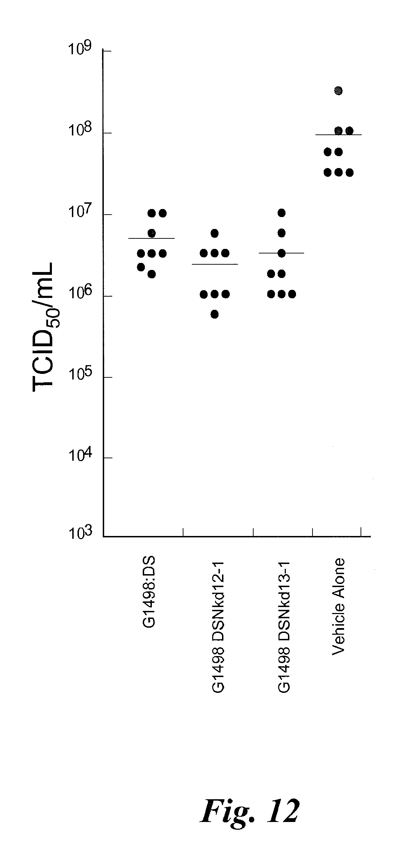

An *in vivo* influenza mouse model was also used to examine the activity of a dicer substrate nicked dsRNA in reducing influenza virus titer as compared to a wild-type dsRNA *(i.e.,* not having a nick). Female BALB/c mice (age 8-10 weeks with 5-10 mice per group) were dosed intranasally with 120 nmol/kg/day dsRNA (formulated in C12-norArg(NH₃+Cl⁻)-C12/DSPE-PEG2000/DSPC/cholesterol at a ratio of 30:1:20:49) for three consecutive days before intranasal challenge with influenza strain PR8 (20 PFU/mouse). Two days after infection, whole lungs are harvested from each mouse and placed in a solution of PBS/0.3% BSA with antibiotics, homogenize, and measure the viral titer (TCID₅₀). Doses were well tolerated by the mice, indicated by less than 2% body weight reduction in any of the dose groups. The mdRNAs tested exhibit similar, if not slightly greater, virus reduction *in vivo* as compared to unmodified and unnicked G1498 dicer substrate *(see* Figure 12). Hence, mdRNA are active *in vivo.*

### EXAMPLE 8

### EFFECT OF MDRNA ON CYTOKINE INDUCTION

The effect of the mdRNA structure on cytokine induction *in vivo* was examined. Female BALB/c mice (age 7-9 weeks) were dosed intranasally with about 50 µM dsRNA (formulated in C12-norArg(NH₃+Cl-)-C12/DSPE-PEG2000/DSPC/cholesterol at a ratio of 30:1:20:49) or with 605 nmol/kg/day naked dsRNA for three consecutive days. About four hours after the final dose is administered, the mice were sacrificed to collect bronchoalveolar fluid (BALF), and collected blood is processed to serum for evaluation of the cytokine response. Bronchial lavage was performed with 0.5 mL ice-cold 0.3% BSA in saline two times for a total of 1 mL. BALF was spun and supernatants collected and frozen until cytokine analysis. Blood was collected from the vena cava immediately following euthanasia, placed into serum separator tubes, and allowed to clot at room temperature for at least 20 minutes. The samples were processed to serum, aliquoted into Millipore ULTRAFREE 0.22 µm filter tubes, spun at 12,000 rpm, frozen on dry ice, and then stored at -70°C until analysis. Cytokine analysis of BALF and plasma were performed using the Procarta™ mouse 10-Plex Cytokine Assay Kit (Panomics, Fremont, CA) on a Bio-Plex™ array reader. Toxicity parameters were also measured, including body weights, prior to the first dose on day 0 and again on day 3 (just prior to euthanasia). Spleens were harvested and weighed (normalized to final body weight). The results are provided in Table 5.

**Table 5. In vivo Cytokine Induction by Naked mdRNA**

| Cytokine | | G1498 | G1498:Nkd 11-1 | G1498:DS | G1498:DSNkd 12-1 | G1498:DSNkd 13-1 | G1498:DSNkd 14-1 |
|---|---|---|---|---|---|---|---|
| IL-6 | Conc (pg/mL) | 90.68 | 10.07 | 77.35 | 17.17 | 18.21 | 38.59 |
| | Fold decrease | - | 9 | - | 5 | 4 | 2 |
| IL-12 (p40) | Cone (pg/mL) | 661.48 | 20.32 | 1403.61 | 25.07 | 37.70 | 57.02 |
| | Fold decrease | - | 33 | - | 56 | 37 | 25 |
| TNFα | Cone (pg/mL) | 264.49 | 25.59 | 112.95 | 20.52 | 29.00 | 64.93 |
| | Fold decrease | - | 10 | - | 6 | 4 | 2 |

The mdRNA (RISC or dicer sized) induced cytokines to lesser extent than the intact (*i.e.*, not nicked) parent molecules. The decrease in cytokine induction was greatest when looking at IL-12(p40), the cytokine with consistently the highest levels of induction of the 10 cytokine multiplex assay. For the mdRNA, the decrease in IL-12 (p40) ranges from 25- to 56-fold, while the reduction in either IL-6 or TNFα induction was more modest (the decrease in these two cytokines ranges from 2- to 10-fold). Thus, the mdRNA structure appears to provide an advantage in vivo in that cytokine induction is minimized compared to unmodified dsRNA.

Similar results were obtained with the formulated mdRNA, although the reduction in induction was not as prominent. In addition, the presence or absence of a locked nucleic acid has no effect on cytokine induction. These results are shown in Table 6.

**Table 6. In vivo Cytokine Induction by Formulated mdRNA**

| Cytokine | | G1498:DS | G1498:Nkd 12-1 | G1498:Nkd 13-1 | G1498:DSNkd 14-1 | G1498:DSNkd 13 |
|---|---|---|---|---|---|---|
| IL-6 | Cone (pg/mL) | 29.04 | 52.95 | 10.28 | 7.79 | 44.29 |
| | Fold decrease | - | -1.8 | 3 | 4 | -1.5 |
| IL-12 (p40) | Conc (pg/mL) | 298.93 | 604.24 | 136.45 | 126.71 | 551.49 |
| | Fold decrease | - | 0 | 2 | 2 | 1 |
| TNFα | Conc (pg/mL) | 13.49 | 21.35 | 3.15 | 3.15 | 18.69 |
| | Fold decrease | - | -1.6 | 4 | 4 | 1.4 |

The teachings of all of references cited herein including patents, patent applications, journal articles, wedpages, tables, and priority documents are incorporated herein in their entirety by reference. Although the foregoing disclosure has been described in detail by way of example for purposes of clarity of understanding, it will be apparent to the artisan that certain changes and modifications may be practiced within the scope of the appended claims which are presented by way of illustration not limitation. In this context, various publications and other references have been cited within the foregoing disclosure for economy of description. It is noted, however, that the various publications discussed herein are incorporated solely for their disclosure prior to the filing date of the present application, and the inventors reserve the right to antedate such disclosure by virtue of prior invention.

### CLAUSES

1. A meroduplex ribonucleic acid (mdRNA) molecule, comprising a first strand of 15 to 40 nucleotides in length that is complementary to human vascular endothelial growth factor (VEGF) mRNA as set forth in SEQ ID NO:1158, 1159, 1160, 1161, 1162, 1163, or 1164 and is fully complementary with up to three mismatches to at least one other human VEGF family mRNA selected from SEQ ID NO:1165, 1166, 1167, or 1168, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands.
2. The mdRNA molecule of clause 1 wherein the first strand is 15 to 25 nucleotides in length or 26 to 40 nucleotides in length.
3. The mdRNA molecule of clause 1 wherein the gap is a nick, or the gap comprises at least one unpaired nucleotide in the first strand positioned between the double-stranded regions formed by the second and third strands when annealed to the first strand.
4. The mdRNA molecule of clause 1 wherein at least one uridine of the mdRNA molecule is a 5-methyluridine, 2-thioribothymidine, or 2'-O-methyl-5-methyluridine.
5. The mdRNA molecule of clause 1 wherein the mdRNA molecule comprises at least one locked nucleic acid (LNA) molecule, deoxy nucleotide, G clamp, 2'-sugar modification, modified internucleoside linkage, or any combination thereof.
6. The mdRNA molecule of clause 1 wherein the mdRNA contains an overhang of one to four nucleotides on at least one 3'-end that is not part of the gap or has a blunt end at one or both ends of the mdRNA.
7. The mdRNA molecule of clause 1 wherein the first strand is complementary to SEQ ID NOS:1158-1165, or SEQ ID NOS:1158-1162, 1164, and 1165, or SEQ ID NOS:1158-1164 and 1166, or SEQ ID NOS:1158-1164 and 1167, or SEQ ID NOS:1158-1164 and 1168, or SEQ ID NOS:1158-1164, 1166, and 1167, or SEQ ID NOS:1165 and 1166, or SEQ ID NOS:1165 and 1167, or SEQ ID NOS:1166 and 1167.
8. An mdRNA molecule, comprising a first strand of 15 to 40 nucleotides in length that is complementary to a human VEGF mRNA as set forth in SEQ ID NO:1158, 1159, 1160, 1161, 1162, 1163, or 1164 and is fully complementary with up to three mismatches to at least one other human VEGF family mRNA selected from SEQ ID NO:1165, 1166, 1167, or 1168, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein at least one pyrimidine of the mdRNA comprises a pyrimidine nucleoside according to Formula I or II: 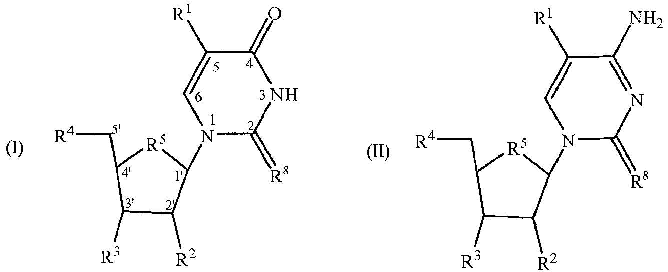 wherein:
   R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted ―O-allyl, ―O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C≡, or heterocyclo group,
   R³ and R⁴ are each independently a hydroxyl, a protected hydroxyl, a phosphate, or an internucleoside linking group, and
   R⁵ and R⁸ are each independently O or S.
9. The mdRNA molecule of clause 8 wherein the first strand is 15 to 25 nucleotides in length or 26 to 40 nucleotides in length.
10. The mdRNA molecule of clause 8 wherein the gap is a nick, or the gap comprises at least one unpaired nucleotide in the first strand positioned between the double-stranded regions formed by the second and third strands when annealed to the first strand.
11. The mdRNA molecule of clause 8 wherein at least one nucleoside is according to Formula (I) and in which R¹ is methyl and R² is ―OH or ―O-methyl.
12. The mdRNA molecule of clause 8 wherein at least one R² is selected from the group consisting of 2ʹ-O-(C₁-C₅) alkyl, 2ʹ-O-methyl, 2ʹ-OCH₂OCH₂CH₃, 2ʹ-OCH₂CH₂OCH₃, 2ʹ-O-allyl, and fluoro.
13. The mdRNA molecule of clause 8 wherein at least one uridine of the mdRNA molecule is a 5-methyluridine, 2-thioribothymidine, or 2'-O-methyl-5-methyluridine.
14. The mdRNA molecule of clause 8 wherein the mdRNA molecule comprises at least one LNA molecule, deoxy nucleotide, G clamp, 2'-sugar modification, modified internucleoside linkage, or any combination thereof.
15. The mdRNA molecule of clause 8 wherein contains an overhang of one to four nucleotides on at least one 3'-end that is not a part of the gap or the dsRNA molecule has a blunt end on one or both ends of the mdRNA molecule.
16. The mdRNA molecule of clause 8 wherein the first strand is complementary to SEQ ID NOS:1158-1165, or SEQ ID NOS:1158-1162, 1164, and 1165, or SEQ ID NOS:1158-1164 and 1166, or SEQ ID NOS:1158-1164 and 1167, or SEQ ID NOS:1158-1164 and 1168, or SEQ ID NOS:1158-1164, 1166, and 1167, or SEQ ID NOS:1165 and 1166, or SEQ ID NOS:1165 and 1167, or SEQ ID NOS:1166 and 1167.
17. An mdRNA molecule, comprising a first strand that is complementary to a human VEGF mRNA as set forth in SEQ ID NO:1158, 1159, 1160, 1161, 1162, 1163, or 1164 and is fully complementary with up to three mismatches to at least one other human VEGF family mRNA selected from SEQ ID NO:1165, 1166, 1167, or 1168, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein the combined double-stranded regions total about 15 base pairs to about 40 base pairs.
18. The mdRNA molecule of clause 17 wherein the first strand is 15 to 25 nucleotides in length or 26 to 40 nucleotides in length.
19. The mdRNA molecule of clause 17 wherein the gap is a nick, or the gap comprises at least one unpaired nucleotide in the first strand positioned between the double-stranded regions formed by the second and third strands when annealed to the first strand.
20. The mdRNA molecule of clause 17 wherein at least one uridine of the mdRNA molecule is a 5-methyluridine, 2-thioribothymidine, or 2'-O-methyl-5-methyluridine.
21. The mdRNA molecule of clause 1 wherein the first strand is 19 to 23 nucleotides in length and is complementary to a human VEGF family nucleic acid sequence as set forth in any one of SEQ ID NOS:1169-1398.
22. The mdRNA molecule of clause 1 wherein the first strand is 25 to 29 nucleotides in length and is complementary to a human VEGF family nucleic acid sequence as set forth in any one of SEQ ID NOS:1169-1398.
23. A method for reducing the expression of one or more human VEGF family gene, comprising administering an mdRNA molecule according to any one of claims 1-22 to a cell expressing one or more VEGF family gene, wherein the mdRNA molecule is capable of specifically binding to one or more VEGF family mRNA and thereby reducing expression of one or more VEGF family gene in the cell.
24. The method according to clause 23 wherein the cell is human.
25. Use of an mdRNA as defined in any one of the preceding clauses for the manufacture of a medicament for use in the therapy of a hyperproliferative or inflammatory disease.

## Claims

1. A double-stranded ribonucleic acid (dsRNA) molecule that down regulates the expression of a human vascular endothelial growth factor (VEGF) mRNA, the dsRNA molecule comprising a first strand of 26 to 40 nucleotides in length, that is complementary to the human VEGF mRNA as set forth in SEQ ID NO: 1158, 1159, 1160, 1161, 1162, 1163, or 1164 and is fully complementary with up to three mismatches to at least one other human VEGF family mRNA selected from SEQ ID NO: 1165, 1166, 1167, or 1168, and a second strand that is complementary to the first strand, and wherein upon annealing of the first strand and the second strand the dsRNA has a 3' overhang and a blunt end.

2. The dsRNA molecule according to Claim 1, wherein the first strand is from 27 to 35 nucleotides in length.

3. The dsRNA molecule according to Claim 1, wherein the 3'-overhang has from one to four nucleotides and is on the first strand.

4. The dsRNA molecule according to Claim 1, wherein at least one pyrimidine of the dsRNA molecule is a pyrimidine nucleoside according to Formula I or II: 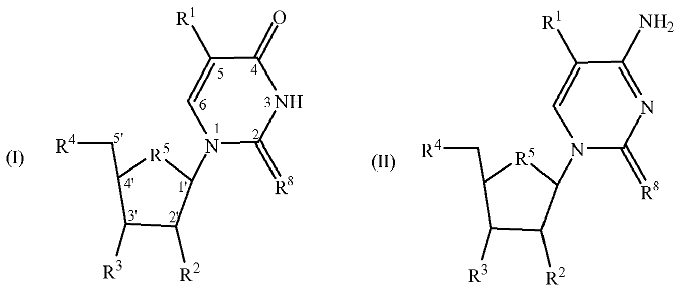 wherein:
R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C≡, or heterocyclo group,
R³ and R⁴ are each independently a hydroxyl, a protected hydroxyl, a phosphate, or an internucleoside linking group, and
R⁵ and R⁸ are each independently O or S.

5. The dsRNA molecule of any of claims 1 to 4, wherein the molecule comprises at least one 5-methyluridine, 2-thioribothymidine, or 2'-O-methyl-5-methyluridine.

6. The dsRNA molecule of any of claims 1 to 4, wherein the molecule comprises at least one locked nucleic acid (LNA) molecule, deoxy nucleotide, G clamp, 2'-sugar modification, modified internucleoside linkage, or any combination thereof.

7. The dsRNA molecule of claim 4, wherein at least one nucleoside is according to Formula I and in which R¹ is methyl and R² is-OH or ―O-methyl;

8. The dsRNA molecule of claim 4, wherein at least one R² is selected from the group consisting of 2'-O-(C₁-C₅) alkyl, 2'-O-methyl, 2'-OCH₂OCH₂CH₃, 2'-OCH₂CH₂OCH₃, 2'-O-allyl, and 2'-fluoro.

9. The dsRNA molecule of claim 1, wherein the dsRNA molecule has a 5'-terminal end comprising a hydroxyl or a phosphate.

10. A dsRNA molecule according to any preceding claim for reducing the expression of one or more human VEGF family genes in a cell expressing one or more human VEGF family genes.

11. A dsRNA molecule according to any preceding claim for use according to claim 10, wherein the cell is a human cell.

12. A dsRNA molecule according to any preceding claim for use in treating a hyperproliferative or inflammatory disease.

13. Use of a dsRNA molecule as defined in any preceding claim for the manufacture of a medicament for reducing the expression of one or more human VEGF family genes in a cell expressing one or more human VEGF family genes.

14. Use according to Claim 13, wherein the cell is a human cell.

15. Use of a dsRNA molecule as defined in any preceding claim for the manufacture of a medicament for use in the therapy of a hyperproliferative or inflammatory disease.
